# EUROPEAN PATENT APPLICATION

(11) **EP 4 219 484 A1**
(43) Date of publication of application: **02.08.2023**
(21) Application number: 21872791.5
(22) Date of filing: 09.09.2021
(51) Int. Cl.: C07D 405/10, C07D 405/14, C07D 409/14, C07D 409/10, C07D 209/82, C07D 403/04, C07D 403/14, H01L 51/00

(54) **ORGANIC LIGHT-EMITTING DEVICE, COMPOSITION FOR ORGANIC LAYER OF ORGANIC LIGHT-EMITTING DEVICE, AND METHOD FOR MANUFACTURING ORGANIC LIGHT-EMITTING DEVICE**

(30) Priority: 28.09.2020 KR 20200125761
(71) Applicant: LT Materials Co., Ltd., Yongin-si, Gyeonggi-do 17118 (KR)
(72) Inventor: LEE, Hyun-Joo, Yongin-si, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/012275
(87) International publication number: WO 2022/065762

(57) **Abstract**

The present specification relates to an organic light emitting device comprising a first electrode, a second electrode, and an organic material layer comprising one or more layers provided between the first electrode and the second electrode, wherein the one or more layers of the organic material layer comprise a heterocyclic compound represented by Chemical Formula 1 and a heterocyclic compound represented by Chemical Formula 2, a composition for the organic material layer of the organic light emitting device, and a method for manufacturing the organic light emitting device.

## Description

### [Technical Field]

This application claims priority to and the benefits of Korean Patent Application No. 10-2020-0125761, filed with the Korean Intellectual Property Office on September 28, 2020, the entire contents of which are incorporated herein by reference.

The present specification relates to an organic light emitting device, a composition for an organic material layer of the organic light emitting device, and a method for manufacturing the organic light emitting device.

### [Background Art]

An organic electroluminescent device is a kind of self-emission type display device, and has advantages of a wide viewing angle, excellent contrast, and fast response speed.

The organic light emitting device has a structure in which an organic thin film is disposed between two electrodes. When a voltage is applied to the organic light emitting device having such a structure, light is emitted while they are disappeared after electrons and holes injected from the two electrodes combine in the organic thin film to form a pair. The organic thin film may be composed of a single layer or multiple layers as needed.

A material of the organic thin film may have a light emitting function as needed. For example, as the material of the organic thin film, a compound capable of forming a light emitting layer for itself may be used, or a compound capable of serving as a host or dopant of a host-dopant based light emitting layer may also be used. Besides that, a compound capable of performing the roles of hole injection, hole transport, electron blocking, hole blocking, electron transport, electron injection, and the like may also be used as the material of the organic thin film.

In order to improve the performance, lifespan, or efficiency of the organic light emitting device, the development of the material of the organic thin film is continuously required.

### Prior Art Documents

### Patent Documents

US Patent No. 4,356,429

### [Disclosure]

### [Technical Problem]

The present disclosure is directed to providing an organic light emitting device comprising a compound having a chemical structure which can satisfy conditions required for materials usable in the organic light emitting device, such as an appropriate energy level, electrochemical stability, thermal stability, etc., and can play various roles required in the organic light emitting device depending on substituents, a composition for an organic material layer of the organic light emitting device, and a method for manufacturing the organic light emitting device.

### [Technical Solution]

An embodiment of the present application provides an organic light emitting device comprising a first electrode, a second electrode, and an organic material layer comprising one or more layers provided between the first electrode and the second electrode, wherein the one or more layers of the organic material layer comprise a heterocyclic compound represented by Chemical Formula 1 below and a heterocyclic compound represented by Chemical Formula 2 below.

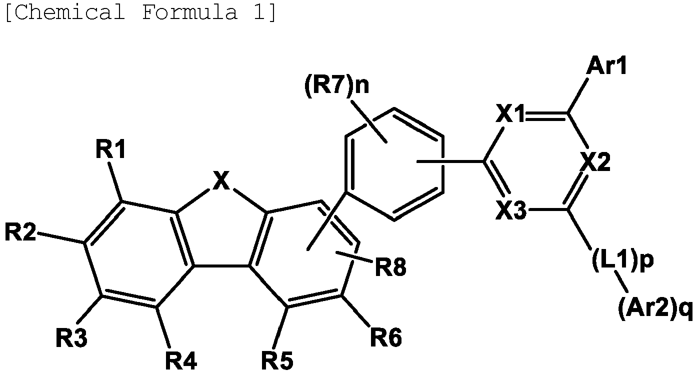

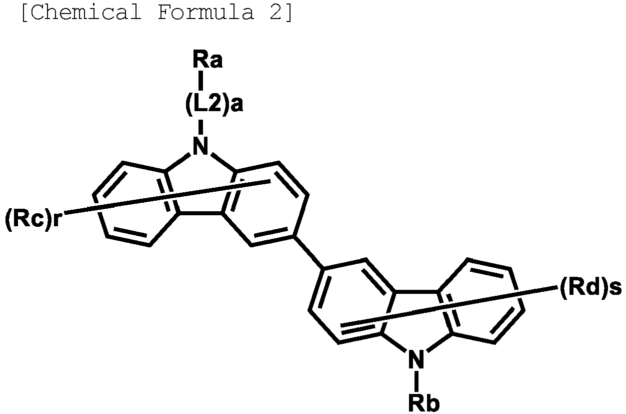

In Chemical Formulas 1 and 2 above,
X is O; or S,
R1 to R8, and Rc and Rd are the same as or different from each other, and are each independently selected from the group comprising hydrogen; deuterium; a halogen; a cyano group; a substituted or unsubstituted C1-C60 alkyl group; a substituted or unsubstituted C2-C60 alkenyl group; a substituted or unsubstituted C2-C60 alkynyl group; a substituted or unsubstituted C1-C60 alkoxy group; a substituted or unsubstituted C3-C60 cycloalkyl group; a substituted or unsubstituted C2-C60 heterocycloalkyl group; a substituted or unsubstituted C6-C60 aryl group; a substituted or unsubstituted C2-C60 heteroaryl group; -SiRR'R"; -P(=O)RR'; and -NRR', or two or more groups adjacent to each other are bonded to each other to form a substituted or unsubstituted aromatic hydrocarbon ring or a substituted or unsubstituted heterocycle ring,
X1 to X3 are N; or CRe, and at least one of X1 to X3 is N,
L1 and L2 are the same as or different from each other and are each independently a direct bond; a substituted or unsubstituted C6-C60 arylene group; or a substituted or unsubstituted C2-C60 heteroarylene group,
Ar1 and Ar2 are the same as or different from each other and are each independently a substituted or unsubstituted C1-C60 alkyl group; a substituted or unsubstituted C6-C60 aryl group; or a substituted or unsubstituted C2-C60 heteroaryl group,
Ra and Rb are the same as or different from each other and are each independently -CN; -SiRR'R"; a substituted or unsubstituted C6-C60 aryl group; or a substituted or unsubstituted C2-C60 heteroaryl group,
R, R' , R" , and Re are the same as or different from each other and are each independently hydrogen; deuterium; a substituted or unsubstituted C1-C60 alkyl group; or a substituted or unsubstituted C6-C60 aryl group,
n, p, and a are an integer of 0 to 4,
r and s are an integer of 0 to 7,
q is an integer of 1 to 5, and
when n, p, a, s, q, and r are 2 or more, the substituents in parentheses are the same as or different from each other.

Further, another embodiment of the present application provides a composition for an organic material layer of the organic light emitting device, comprising the heterocyclic compound represented by Chemical Formula 1 above and the heterocyclic compound represented by Chemical Formula 2 above.

Finally, an embodiment of the present application provides a method for manufacturing the organic light emitting device, comprising the steps of: preparing a substrate; forming a first electrode on the substrate; forming an organic material layer comprising one or more layers on the first electrode; and forming a second electrode on the organic material layers, wherein the step of forming the organic material layers comprises a step of forming an organic material layer comprising one or more layers using a composition for the organic material layer according to the present application.

### [Advantageous Effects]

The heterocyclic compound according to an embodiment of the present application may be used as an organic material layer material of an organic light emitting device. The heterocyclic compound may be used as a material for a hole injection layer, a hole transport layer, a light emitting layer, an electron transport layer, an electron injection layer, a charge generation layer, etc. in the organic light emitting device. In particular, the heterocyclic compound represented by Chemical Formula 1 above and the heterocyclic compound represented by Chemical Formula 2 above may be simultaneously used as a material of the light emitting layer of the organic light emitting device. Further, when the heterocyclic compound represented by Chemical Formula 1 above and the heterocyclic compound represented by Chemical Formula 2 above are simultaneously used in the organic light emitting device, driving voltage of the device may be lowered, light efficiency may be improved, and thermal stability of the compounds may cause lifespan characteristics of the device to be improved.

In particular, the heterocyclic compound represented by Chemical Formula 1 above has a linking group of a phenylene group in the core structure of dibenzofuran or dibenzothiophene, and has a substituent of azines. Accordingly, it may have characteristics of enabling the driving voltage to be reduced and enabling the efficiency and lifespan to be maximized by enhancing characteristics of the n-type and comprising a heterocyclic compound together corresponding to biscarbazoles having a specific substituent represented by Chemical Formula 2 above.

Specifically, in the heterocyclic compound of Chemical Formula 1 above, HOMO is localized in dibenzofuran and dibenzothiophene to effectively stabilize holes, and LUMO is localized in an azine-based substituent to effectively stabilize electrons. Therefore, it is especially effective when used as a host of the light emitting layer.

### [Description of Drawings]

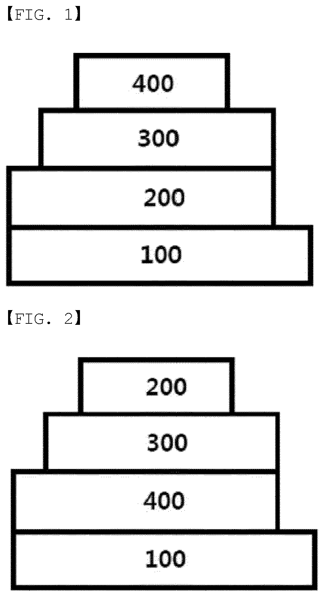

FIGS. 1 to 3 are diagrams each schematically illustrating a lamination structure of an organic light emitting device according to an embodiment of the present application.

### [Mode for Disclosure]

Hereinafter, the present application will be described in detail.

The term "substitution" means that a hydrogen atom bonded to a carbon atom of a compound is replaced with another substituent, and the position to be substituted is not limited as long as it is a position at which the hydrogen atom is substituted, that is, a position where the substituent is substitutable, and when two or more are substituted, two or more substituents may be the same as or different from each other.

In the present specification, "substituted or unsubstituted" means that it is substituted or unsubstituted with one or more substituents selected from the group comprising deuterium; a cyano group; a halogen group; an C1-C60 alkyl group; a C2-C60 alkenyl group; a C2-C60 alkynyl group; a C3-C60 cycloalkyl group; a C2-C60 heterocycloalkyl group; a C6-C60 aryl group; a C2-C60 heteroaryl group; a silyl group; a phosphine oxide group; and an amine group, or is substituted or unsubstituted with a substituent to which two or more substituents selected from the above-exemplified substituents are connected.

In the present specification, "when a substituent is not indicated in a Chemical Formula or compound structure" means that a hydrogen atom is bonded to a carbon atom. However, since deuterium (²H) is an isotope of hydrogen, some hydrogen atoms may be deuterium.

In an embodiment of the present application, "when a substituent is not indicated in a Chemical Formula or compound structure" may mean that all positions where a substituent can come are hydrogen or deuterium. That is, deuterium may be an isotope of hydrogen, and some hydrogen atoms may be deuterium that is an isotope, and the content of deuterium may be 0 to 100% at this time.

In an embodiment of the present application, in the case of "when a substituent is not indicated in a Chemical Formula or compound structure", if deuterium is not explicitly excluded, such as the content of deuterium of 0%, the content of hydrogen of 100%, etc., hydrogen and deuterium may be mixed and used in the compound. That is, when expressing "substituent X is hydrogen", deuterium is not excluded, such as the content of hydrogen of 100%, the content of deuterium of 0%, etc., it may mean a state in which hydrogen and deuterium are mixed.

In an embodiment of the present application, deuterium is an element having a deuteron comprising one proton and one neutron as one of the isotopes of hydrogen as a nucleus, it may be expressed as hydrogen-2, and an element symbol may also be written as D or 2H.

In an embodiment of the present application, although isotopes meaning atoms which have the same atomic number (Z), but have different mass numbers (A) have the same number of protons, they may also be interpreted as elements with different numbers of neutrons.

In an embodiment of the present application, when the total number of substituents that a basic compound may have is defined as T1, and the number of the specific substituents among them is defined as T2, the meaning of the content T% of specific substituents may be defined as T2/T1×100 = T%.

That is, as an example, the 20% content of deuterium in the phenyl group represented by 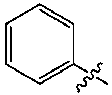 may be expressed as 20% when the total number of substituents that the phenyl group may have is 5 (T1 in the formula) and the number of deuteriums among them is 1 (T2 in the formula). That is, it may be represented by the structural formula below that the content of deuterium in the phenyl group is 20%.

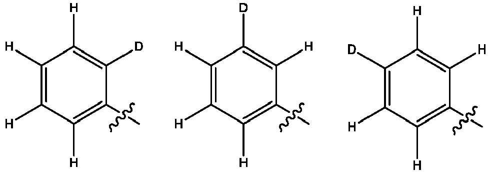

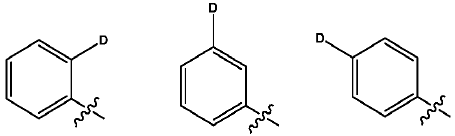

Further, in an embodiment of the present application, "a phenyl group having a deuterium content of 0%" may mean a phenyl group that does not contain a deuterium atom, that is, has 5 hydrogen atoms.

In the present specification, the halogen may be fluorine, chlorine, bromine, or iodine.

In the present specification, the alkyl group comprises a linear or branched chain having 1 to 60 carbon atoms, and may be further substituted by other substituents. The number of carbon atoms of the alkyl group may be 1 to 60, specifically 1 to 40, and more specifically 1 to 20. Specific examples of the alkyl group may comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, an 1-ethyl-butyl group, a pentyl group, an n-pentyl group, an isopentyl group, a neopentyl group, a tert-pentyl group, a hexyl group, an n-hexyl group, an 1-methylpentyl group, a 2-methylpentyl group, a 4-methyl-2-pentyl group, a 3,3-dimethylbutyl group, a 2-ethylbutyl group, a heptyl group, an n-heptyl group, an 1-methylhexyl group, a cyclopentylmethyl group, a cyclohexylmethyl group, an octyl group, an n-octyl group, a tert-octyl group, an 1-methylheptyl group, a 2-ethylhexyl group, a 2-propylpentyl group, an n-nonyl group, a 2,2-dimethylheptyl group, an 1-ethyl-propyl group, an 1,1-dimethylpropyl group, an isohexyl group, a 2-methylpentyl group, a 4-methylhexyl group, a 5-methylhexyl group, etc., but are not limited thereto.

In the present specification, the alkenyl group may comprise a linear or branched chain having 2 to 60 carbon atoms, and may be further substituted by other substituents. The number of carbon atoms of the alkenyl group may be 2 to 60, specifically 2 to 40, and more specifically 2 to 20. Specific examples of the alkenyl group may comprise a vinyl group, an 1-propenyl group, an isopropenyl group, an 1-butenyl group, a 2-butenyl group, a 3-butenyl group, an 1-pentenyl group, a 2-pentenyl group, a 3-pentenyl group, a 3-methyl-1-butenyl group, an 1,3-butadienyl group, an allyl group, an 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stylbenyl group, a styrenyl group, etc., but are not limited thereto.

In the present specification, the alkynyl group may comprise a linear or branched chain having 2 to 60 carbon atoms, and may be further substituted by other substituents. The number of carbon atoms of the alkynyl group may be 2 to 60, specifically 2 to 40, and more specifically 2 to 20.

In the present specification, the alkoxy group may be a linear, branched, or cyclic chain. The number of carbon atoms of the alkoxy group is not particularly limited, but is preferably 1 to 20. Specific examples of the alkoxy group may comprise methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, etc., but are not limited thereto.

In the present specification, the cycloalkyl group may comprise a monocyclic or polycyclic ring having 3 to 60 carbon atoms, and may be further substituted by other substituents. Here, the polycyclic ring refers to a group in which a cycloalkyl group is directly connected or condensed with other ring group. Here, although the other ring group may be a cycloalkyl group, it may also be a different type of ring group, for example, a heterocycloalkyl group, an aryl group, a heteroaryl group, or the like. The number of carbon atoms of the cycloalkyl group may be 3 to 60, specifically 3 to 40, and more specifically 5 to 20. Specific examples of the cycloalkyl group may comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, etc., but are not limited thereto.

In the present specification, the heterocycloalkyl group may comprise O, S, Se, N, or Si as a heteroatom, may comprise a monocyclic or polycyclic ring having 2 to 60 carbon atoms, and may be further substituted by other substituents. Here, the polycyclic ring refers to a group in which a heterocycloalkyl group is directly connected or condensed with other ring group. Here, although the other ring group may be a heterocycloalkyl group, it may also be a different type of ring group, for example, a cycloalkyl group, an aryl group, a heteroaryl group, or the like. The number of carbon atoms of the heterocycloalkyl group may be 2 to 60, specifically 2 to 40, and more specifically 3 to 20.

In the present specification, the aryl group may comprise a monocyclic or polycyclic ring having 6 to 60 carbon atoms, and may be further substituted by other substituents. Here, the polycyclic ring means a group in which an aryl group is directly connected or condensed with other ring group. Here, although the other ring group may be an aryl group, it may also be a different type of ring group, for example, a cycloalkyl group, a heterocycloalkyl group, a heteroaryl group, or the like. The aryl group comprises a spiro group. The number of carbon atoms of the aryl group may be 6 to 60, specifically 6 to 40, and more specifically 6 to 25. Specific examples of the aryl group may comprise a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, condensed ring groups thereof, etc., but are not limited thereto.

In the present specification, the fluorenyl group may be substituted, and adjacent substituents may be bonded to each other to form a ring.

When the fluorenyl group is substituted, it may become 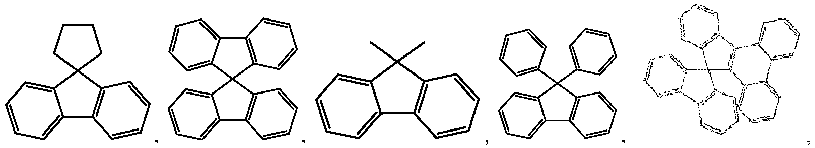 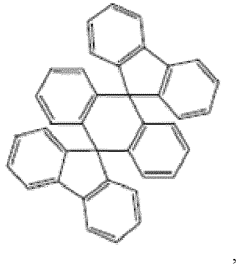 etc., but is not limited thereto.

In the present specification, the heteroaryl group may comprise S, O, Se, N, or Si as a heteroatom, may comprise a monocyclic or polycyclic ring having 2 to 60 carbon atoms, and may be further substituted by other substituents. Here, the polycyclic ring refers to a group in which a heteroaryl group is directly connected or condensed with other ring group. Here, although the other ring group may be a heteroaryl group, it may also be a different type of ring group, for example, a cycloalkyl group, a heterocycloalkyl group, an aryl group, or the like. The number of carbon atoms of the heteroaryl group may be 2 to 60, specifically 2 to 40, and more specifically 3 to 25. Specific examples of the heteroaryl group may comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a deoxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilol group, a spirobi (dibenzosilol) group, dihydrophenazinyl group, a phenoxazinyl group, a phenantridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiazinyl group, a phthalazinyl group, a naphthylidinyl group, a phenanthrolinyl group, a benzo[c][1,2,5]thiadiazolyl group, a 5,10-dihydrodibenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, etc., but are not limited thereto.

In the present specification, the amine group may be selected from the group comprising a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group may comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, etc., but are not limited thereto.

In the present specification, the arylene group means that the aryl group has two bonding positions, that is, a divalent group. The description of the aryl group described above may be applied except that each of these is a divalent group. Further, the heteroarylene group means that the heteroaryl group has two bonding positions, that is, a divalent group. The description of the heteroaryl group described above may be applied except that each of these is a divalent group.

In the present specification, the phosphine oxide group is represented by -P(=O)R101R102, and R101 and R102 may be the same as or different from each other and may be each independently a substituent comprising at least one of hydrogen; deuterium; a halogen group; an alkyl group; an alkenyl group; an alkoxy group; a cycloalkyl group; an aryl group; and a heterocyclic group. Specifically, it may be substituted with an aryl group, and the above-described examples may be applied to the aryl group. For example, the phosphine oxide group comprises a diphenylphosphine oxide group, a dinaphthylphosphine oxide group, etc., but is not limited thereto.

In the present specification, the silyl group may be a substituent which comprises Si, and to which the Si atom is directly connected as a radical, and is represented by - SiR104R105R106, and R104 to R106 may be the same as or different from each other and may be each independently a substituent comprising at least one of hydrogen; deuterium; a halogen group; an alkyl group; an alkenyl group; an alkoxy group; a cycloalkyl group; an aryl group; and a heterocyclic group. Specific examples of the silyl group may comprise a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, etc., but are not limited thereto.

In the present specification, the "adjacent" group may mean a substituent substituted on an atom directly connected to the atom in which the corresponding substituent is substituted, a substituent positioned to be sterically closest to the corresponding substituent, or another substituent substituted on the atom in which the corresponding substituent is substituted. For example, two substituents substituted at an ortho position in a benzene ring and two substituents substituted at the same carbon in an aliphatic ring may be interpreted as groups "adjacent" to each other.

The structures exemplified by the above-described cycloalkyl group, cycloheteroalkyl group, aryl group and heteroaryl group may be applied except that the aliphatic or aromatic hydrocarbon ring or heterocycle ring that adjacent groups may form is not a monovalent group.

An embodiment of the present application provides an organic light emitting device comprising a first electrode, a second electrode, and an organic material layer comprising one or more layers provided between the first electrode and the second electrode, wherein the one or more layers of the organic material layer comprise a heterocyclic compound represented by Chemical Formula 1 above and a heterocyclic compound represented by Chemical Formula 2 above.

In an embodiment of the present application, Chemical Formula 1 above may be represented by Chemical Formula 3 or 4 below.

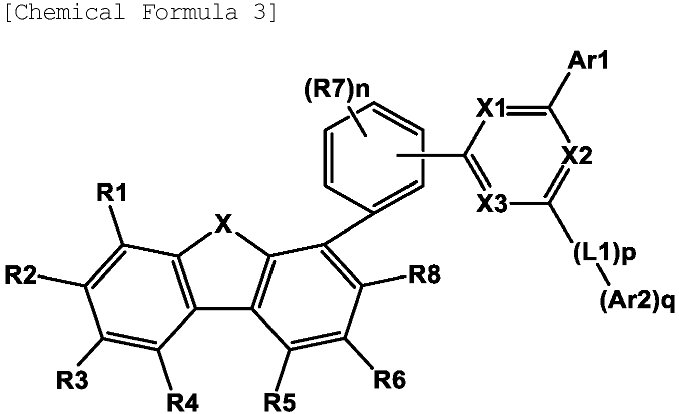

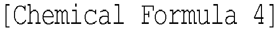

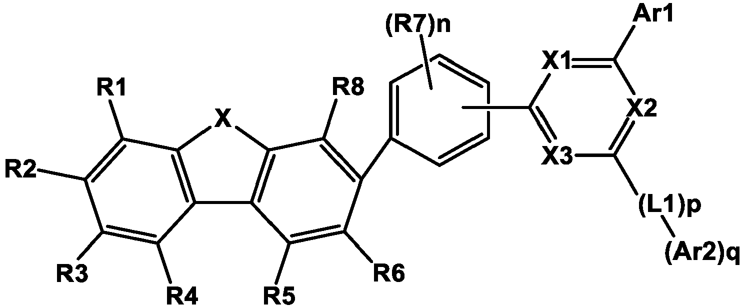

In Chemical Formulas 3 and 4 above,
the definition of each substituent is the same as defined in Chemical Formula 1 above.

In an embodiment of the present application, 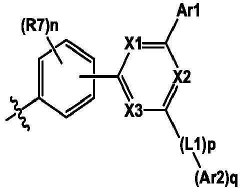 of Chemical Formula 1 above may be represented by any one of Chemical Formulas 1-1 to 1-3 below.

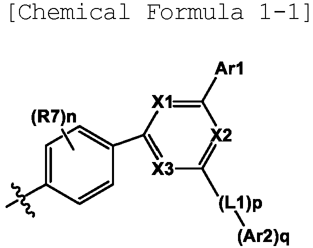

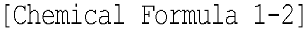

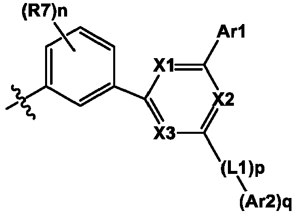

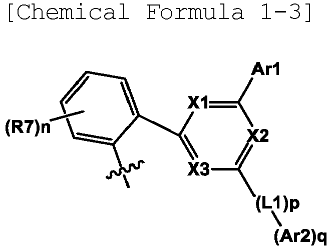

In Chemical Formulas 1-1 to 1-3 above,
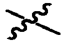 means a position connected to Chemical Formula 1, and
the definition of each substituent is the same as defined in Chemical Formula 1 above.

In an embodiment of the present application, X may be O; or S.

In an embodiment of the present application, X may be O.

In an embodiment of the present application, X may be S.

In an embodiment of the present application, X1 to X3 may be N; or CRe, and at least one of X1 to X3 may be N.

In an embodiment of the present application, X1 to X3 may be N.

In an embodiment of the present application, X1 and X2 may be N, and X3 may be CRe.

In an embodiment of the present application, X1 and X3 may be N, and X2 may be CRe.

In an embodiment of the present application, R1 to R8, and Rc and Rd may be the same as or different from each other, and may be each independently selected from the group comprising hydrogen; deuterium; a halogen; a cyano group; a substituted or unsubstituted C1-C60 alkyl group; a substituted or unsubstituted C2-C60 alkenyl group; a substituted or unsubstituted C2-C60 alkynyl group; a substituted or unsubstituted C1-C60 alkoxy group; a substituted or unsubstituted C3-C60 cycloalkyl group; a substituted or unsubstituted C2-C60 heterocycloalkyl group; a substituted or unsubstituted C6-C60 aryl group; a substituted or unsubstituted C2-C60 heteroaryl group; -SiRR'R"; -P(=O)RR'; and -NRR', or two or more groups adjacent to each other may be bonded to each other to form a substituted or unsubstituted aromatic hydrocarbon ring or a substituted or unsubstituted heterocycle ring.

In another embodiment, R1 to R8, and Rc and Rd may be the same as or different from each other and may be each independently selected from the group comprising hydrogen; deuterium; a substituted or unsubstituted C1-C60 alkyl group; a substituted or unsubstituted C6-C60 aryl group; a substituted or unsubstituted C2-C60 heteroaryl group; -SiRR'R"; -P(=O)RR'; and -NRR'.

In another embodiment, R1 to R8, and Rc and Rd may be the same as or different from each other and may be each independently hydrogen; deuterium; a substituted or unsubstituted C1-C60 alkyl group; a substituted or unsubstituted C6-C60 aryl group; or a substituted or unsubstituted C2-C60 heteroaryl group.

In another embodiment, R1 to R8, and Rc and Rd may be the same as or different from each other and may be each independently hydrogen; deuterium; a substituted or unsubstituted C1-C40 alkyl group; a substituted or unsubstituted C6-C40 aryl group; or a substituted or unsubstituted C2-C40 heteroaryl group.

In another embodiment, R1 to R8, and Rc and Rd may be the same as or different from each other and may be each independently hydrogen; deuterium; or a substituted or unsubstituted C6-C40 aryl group.

In another embodiment, R1 to R8, and Rc and Rd may be the same as or different from each other and may be each independently hydrogen; deuterium; or a C6-C40 aryl group.

In another embodiment, R1 to R8, and Rc and Rd may be the same as or different from each other and may be each independently hydrogen; deuterium; or a C6-C20 aryl group.

In another embodiment, R1 to R8, and Rc and Rd may be the same as or different from each other and may be each independently hydrogen; deuterium; a monocyclic C6-C10 aryl group; or a polycyclic C10-C20 aryl group.

In another embodiment, R1 to R8, and Rc and Rd may be the same as or different from each other and may be each independently hydrogen; deuterium; a substituted or unsubstituted phenyl group; or a substituted or unsubstituted biphenyl group.

In another embodiment, R1 to R8, and Rc and Rd may be the same as or different from each other and may be each independently hydrogen; deuterium; a phenyl group; or a biphenyl group.

In an embodiment of the present application, Ar1 may be a substituted or unsubstituted C1-C60 alkyl group; a substituted or unsubstituted C6-C60 aryl group; or a substituted or unsubstituted C2-C60 heteroaryl group.

In another embodiment, Ar1 may be a substituted or unsubstituted C6-C60 aryl group.

In another embodiment, Ar1 may be a substituted or unsubstituted C6-C40 aryl group.

In another embodiment, Ar1 may be a C6-C40 aryl group.

In another embodiment, Ar1 may be a monocyclic C6-C10 aryl group; or a polycyclic C10-C40 aryl group.

In another embodiment, Ar1 may be a monocyclic C6-C10 aryl group; or a polycyclic C10-C20 aryl group.

In another embodiment, Ar1 may be a substituted or unsubstituted phenyl group; or a substituted or unsubstituted biphenyl group.

In another embodiment, Ar1 may be a phenyl group; or a biphenyl group.

In another embodiment, Ar1 may be represented by any one of Chemical Formulas 1-1-1 to 1-1-3 below.

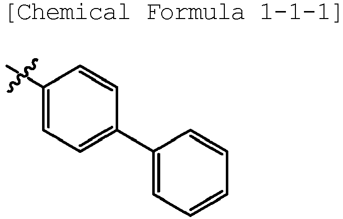

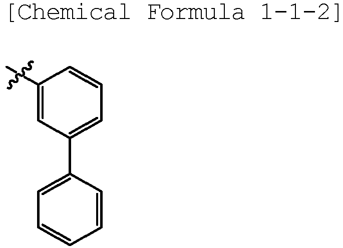

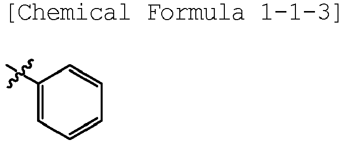

In Chemical Formulas 1-1-1 to 1-1-3 above,
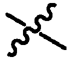 means a position connected to Chemical Formula 1.

In an embodiment of the present application, L1 may be a direct bond; a substituted or unsubstituted C6-C60 arylene group; or a substituted or unsubstituted C2-C60 heteroarylene group.

In another embodiment, L1 may be a direct bond; a substituted or unsubstituted C6-C40 arylene group; or a substituted or unsubstituted C2-C40 heteroarylene group.

In another embodiment, L1 may be a direct bond; or a substituted or unsubstituted C6-C40 arylene group.

In another embodiment, L1 may be a direct bond; or a C6-C40 arylene group.

In another embodiment, L1 may be a direct bond; or a C6-C20 arylene group.

In another embodiment, L1 may be a direct bond; a substituted or unsubstituted phenylene group; or a substituted or unsubstituted biphenylene group.

In another embodiment, L1 may be a direct bond; a phenylene group; or a biphenylene group.

In an embodiment of the present application, Ar2 may be a substituted or unsubstituted C1-C60 alkyl group; a substituted or unsubstituted C6-C60 aryl group; or a substituted or unsubstituted C2-C60 heteroaryl group.

In another embodiment, Ar2 may be a substituted or unsubstituted C1-C40 alkyl group; a substituted or unsubstituted C6-C40 aryl group; or a substituted or unsubstituted C2-C40 heteroaryl group.

In another embodiment, Ar2 may be a substituted or unsubstituted C6-C40 aryl group; or a substituted or unsubstituted C2-C40 heteroaryl group.

In another embodiment, Ar2 may be a C6-C40 aryl group; or a C2-C40 heteroaryl group.

In another embodiment, Ar2 may be a substituted or unsubstituted phenyl group; a substituted or unsubstituted biphenyl group; a substituted or unsubstituted terphenyl group; a substituted or unsubstituted triphenylenyl group; a substituted or unsubstituted dibenzofuran group; or a substituted or unsubstituted dibenzothiophene group.

In another embodiment, Ar2 may be a phenyl group; a biphenyl group; a terphenyl group; a triphenylenyl group; a dibenzofuran group; or a dibenzothiophene group.

In another embodiment, Ar2 may be represented by Chemical Formula 1-2-1 or 1-2-2 below.

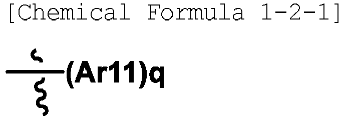

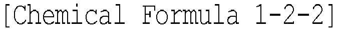

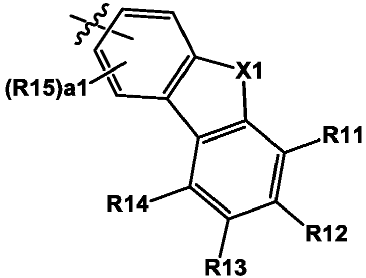

In Chemical Formulas 1-2-1 and 1-2-2 above,
q is the same as the definition in Chemical Formula 1 above,
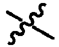 means a position connected to Chemical Formula 1,
X1 is O; or S,
R11 to R15 are the same as or different from each other, and are each independently selected from the group comprising hydrogen; deuterium; a halogen; a cyano group; a substituted or unsubstituted C1-C60 alkyl group; a substituted or unsubstituted C2-C60 alkenyl group; a substituted or unsubstituted C2-C60 alkynyl group; a substituted or unsubstituted C1-C60 alkoxy group; a substituted or unsubstituted C3-C60 cycloalkyl group; a substituted or unsubstituted C2-C60 heterocycloalkyl group; a substituted or unsubstituted C6-C60 aryl group; a substituted or unsubstituted C2-C60 heteroaryl group; -SiRR'R"; -P(=O)RR'; and -NRR', or two or more groups adjacent to each other are bonded to each other to form a substituted or unsubstituted aromatic hydrocarbon ring or a substituted or unsubstituted heterocycle ring,
a1 is an integer of 0 to 3, and when it is 2 or more, the substituents in parentheses are the same as or different from each other,
the definitions of R, R', and R" are the same as those in Chemical Formula 1 above, and
Ar11 is a substituted or unsubstituted C6-C60 aryl group.

In an embodiment of the present application, R11 to R15 may be the same as or different from each other, and may be each independently selected from the group comprising hydrogen; deuterium; a halogen; a cyano group; a substituted or unsubstituted C1-C60 alkyl group; a substituted or unsubstituted C2-C60 alkenyl group; a substituted or unsubstituted C2-C60 alkynyl group; a substituted or unsubstituted C1-C60 alkoxy group; a substituted or unsubstituted C3-C60 cycloalkyl group; a substituted or unsubstituted C2-C60 heterocycloalkyl group; a substituted or unsubstituted C6-C60 aryl group; a substituted or unsubstituted C2-C60 heteroaryl group; -SiRR'R"; -P(=O)RR'; and -NRR', or two or more groups adjacent to each other may be bonded to each other to form a substituted or unsubstituted aromatic hydrocarbon ring or a substituted or unsubstituted heterocycle ring.

In another embodiment, R11 to R15 may be the same as or different from each other, and may be each independently selected from the group comprising hydrogen; deuterium; a substituted or unsubstituted C1-C60 alkyl group; a substituted or unsubstituted C6-C60 aryl group; and a substituted or unsubstituted C2-C60 heteroaryl group, or two or more groups adjacent to each other may be bonded to each other to form a substituted or unsubstituted aromatic hydrocarbon ring or a substituted or unsubstituted heterocycle ring.

In another embodiment, R11 to R15 may be the same as or different from each other, and may be each independently selected from the group comprising hydrogen; deuterium; a substituted or unsubstituted C1-C60 alkyl group; a substituted or unsubstituted C6-C60 aryl group; and a substituted or unsubstituted C2-C60 heteroaryl group.

In another embodiment, R11 to R15 may be the same as or different from each other, and may be each independently selected from the group comprising hydrogen; deuterium; a C1-C60 alkyl group; a C6-C60 aryl group; and a C2-C60 heteroaryl group.

In another embodiment, R11 to R15 may be the same as or different from each other, and may be each independently selected from the group comprising hydrogen; deuterium; a C1-C40 alkyl group; a C6-C40 aryl group; and a C2-C40 heteroaryl group.

In another embodiment, R11 to R15 may be the same as or different from each other, and may be each independently selected from the group comprising hydrogen; deuterium; a C6-C40 aryl group; and a C2-C40 heteroaryl group.

In another embodiment, R11 to R15 may be the same as or different from each other, and may be each independently hydrogen; or deuterium.

In an embodiment of the present application, Ar11 is a substituted or unsubstituted C6-C60 aryl group.

In another embodiment, Ar11 is a substituted or unsubstituted C6-C40 aryl group.

In another embodiment, Ar11 is a C6-C40 aryl group.

In another embodiment, Ar11 is a C6-C20 aryl group.

In another embodiment, Ar11 is a C6-C10 monocyclic aryl group; or a C10-C20 polycyclic aryl group.

In another embodiment, Ar11 may be a C6-C10 substituted or unsubstituted phenyl group; a substituted or unsubstituted biphenyl group; a substituted or unsubstituted terphenyl group; or a substituted or unsubstituted triphenylenyl group.

In another embodiment, Ar11 may be a C6-C10 phenyl group; a biphenyl group; a terphenyl group; or a triphenylenyl group.

In an embodiment of the present application, Chemical Formula 1-2-2 above may be represented by any one of Chemical Formulas 1-A to 1-D below.

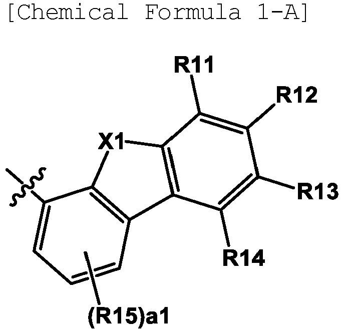

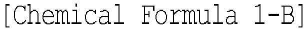

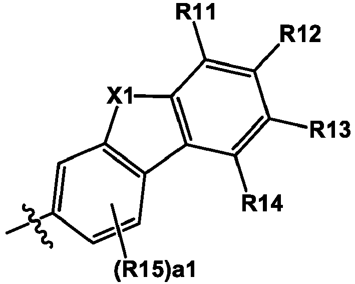

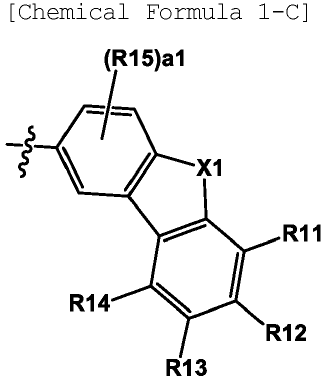

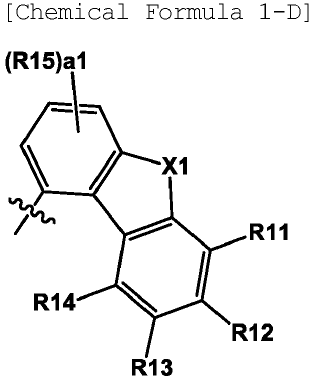

In Chemical Formulas 1-A to 1-D above,
the definition of each substituent is the same as the definition in Chemical Formula 1-2-2 above.

In an embodiment of the present application, L2 may be a direct bond; a substituted or unsubstituted C6-C60 arylene group; or a substituted or unsubstituted C2-C60 heteroarylene group.

In another embodiment, L2 may be a direct bond; a substituted or unsubstituted C6-C40 arylene group; or a substituted or unsubstituted C2-C40 heteroarylene group.

In another embodiment, L2 may be a direct bond; a C6-C40 arylene group; or a C2-C40 heteroarylene group.

In another embodiment, L2 may be a direct bond; a substituted or unsubstituted phenylene group; a substituted or unsubstituted biphenylene group; or a substituted or unsubstituted divalent dibenzofuran group.

In another embodiment, L2 may be a direct bond; a phenylene group; a biphenylene group; or a divalent dibenzofuran group.

In an embodiment of the present application, Ra and Rb may be the same as or different from each other, and may be each independently -CN; SiRR'R"; a substituted or unsubstituted C6-C60 aryl group; or a substituted or unsubstituted C2-C60 heteroaryl group.

In another embodiment, Ra may be -CN; SiRR'R"; a substituted or unsubstituted C6-C60 aryl group; or a substituted or unsubstituted C2-C60 heteroaryl group.

In another embodiment, Ra may be -CN; SiRR'R"; a C6-C40 aryl group substituted or unsubstituted with a C1-C40 alkyl group or a C6-C40 aryl group; or a C2-C60 heteroaryl group substituted or unsubstituted with a C6-C40 aryl group.

In another embodiment, Ra may be -CN; SiRR'R"; a substituted or unsubstituted phenyl group; a substituted or unsubstituted biphenyl group; a substituted or unsubstituted terphenyl group; a substituted or unsubstituted dimethylfluorenyl group; a substituted or unsubstituted diphenylfluorenyl group; a substituted or unsubstituted spirobifluorenyl group; or a substituted or unsubstituted dibenzofuran group.

In another embodiment, Ra may be -CN; SiRR'R"; a phenyl group; a biphenyl group; a terphenyl group; a dimethylfluorenyl group; a diphenylfluorenyl group; a spirobifluorenyl group; or a dibenzofuran group substituted or unsubstituted with a phenyl group.

In another embodiment, Rb may be a substituted or unsubstituted C6-C60 aryl group; or a substituted or unsubstituted C2-C60 heteroaryl group.

In another embodiment, Rb may be a C6-C60 aryl group substituted or unsubstituted with a C1-C40 alkyl group, -CN, SiRR'R", or a C6-C40 aryl group.

In another embodiment, Rb may be a C6-C40 aryl group substituted or unsubstituted with a C1-C40 alkyl group, -CN, SiRR'R", or a C6-C40 aryl group.

In another embodiment, Rb may be a phenyl group substituted or unsubstituted with -CN or SiRR'R"; a biphenyl group substituted or unsubstituted with a phenyl group; a terphenyl group; and a dimethylfluorenyl group.

In an embodiment of the present application, -(L2)a-Ra and Rb of Chemical Formula 2 above may be different from each other.

In an embodiment of the present application, -(L2)a-Ra and Rb of Chemical Formula 2 above may be the same as each other.

In an embodiment of the present application, Re may be hydrogen; deuterium; a substituted or unsubstituted C1-C60 alkyl group; or a substituted or unsubstituted C6-C60 aryl group.

In another embodiment, Re may be hydrogen; or deuterium.

In an embodiment of the present application, R, R', and R" may be the same as or different from each other and may be each independently a substituted or unsubstituted C1-C60 alkyl group; a substituted or unsubstituted C6-C60 aryl group; or a substituted or unsubstituted C2-C60 heteroaryl group.

In another embodiment, R, R', and R" may be the same as or different from each other and may be each independently a substituted or unsubstituted C1-C60 alkyl group; or a substituted or unsubstituted C6-C60 aryl group.

In another embodiment, R, R', and R" may be the same as or different from each other and may be each independently a C1-C60 alkyl group; or a C6-C60 aryl group.

In another embodiment, R, R', and R" may be the same as or different from each other and may be each independently a methyl group; or a phenyl group.

In another embodiment, R, R', and R" may be a substituted or unsubstituted phenyl group.

In another embodiment, R, R', and R" may be a substituted or unsubstituted methyl group.

In another embodiment, R, R', and R" may be a phenyl group.

When the compound of Chemical Formula 1 above and the compound of Chemical Formula 2 above are simultaneously included in the organic material layer of the organic light emitting device, better efficiency and lifespan effects are exhibited. This result may expect an exciplex phenomenon to occur when both compounds are included at the same time.

The exciplex phenomenon is a phenomenon in which energy having a size of a HOMO level of a donor (p-host) and a LUMO level of an acceptor (n-host) is emitted through electron exchange between two molecules. When a donor (p-host) with good hole transport ability and an acceptor (n-host) with good electron transport ability are used as a host of the light emitting layer, since holes are injected into the p-host and electrons are injected into the n-host, the driving voltage may be lowered, thereby helping to improve the lifespan.

According to an embodiment of the present application, Chemical Formula 1 above may be represented by any one of compounds below, but is not limited thereto.

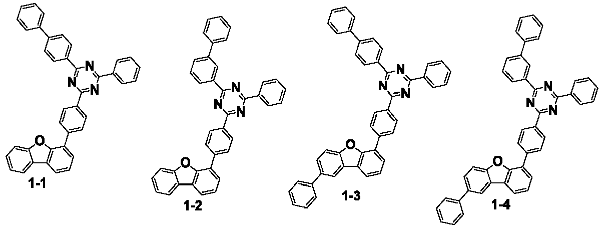

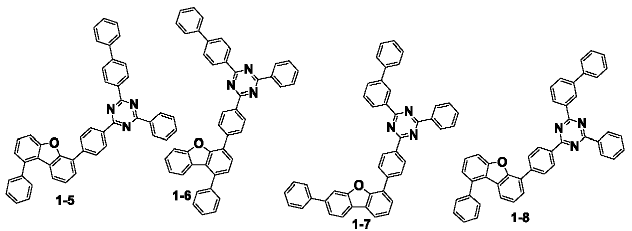

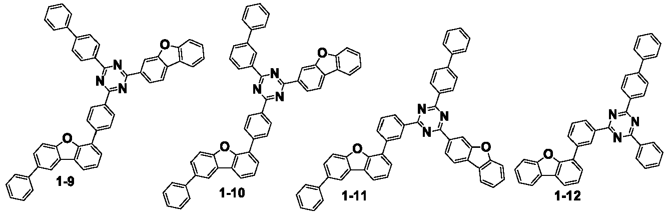

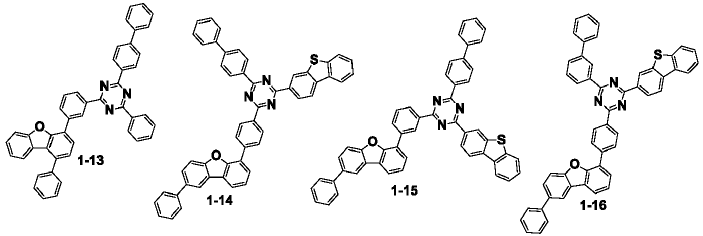

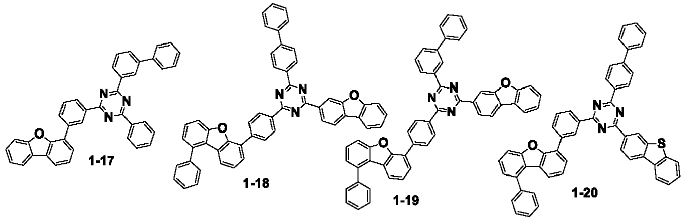

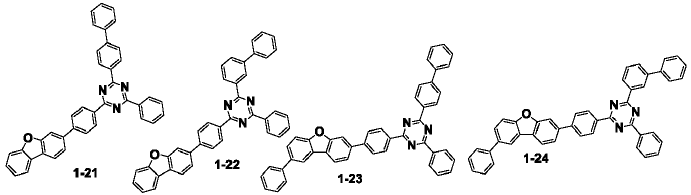

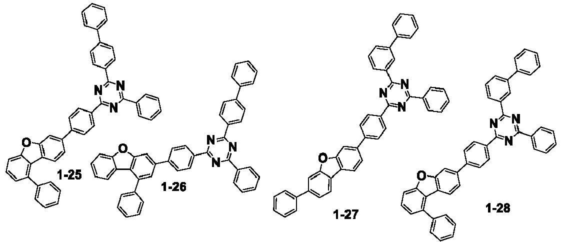

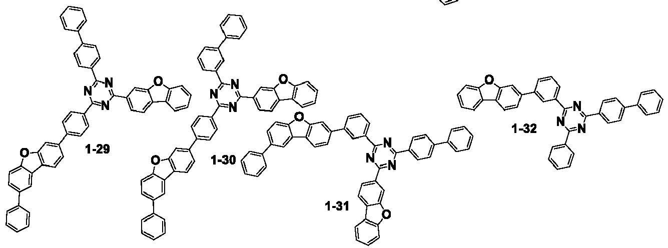

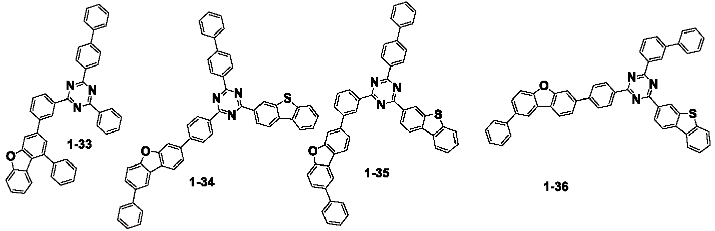

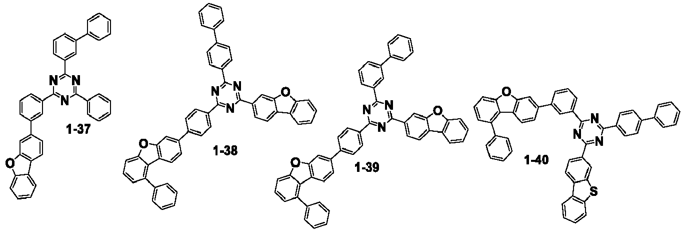

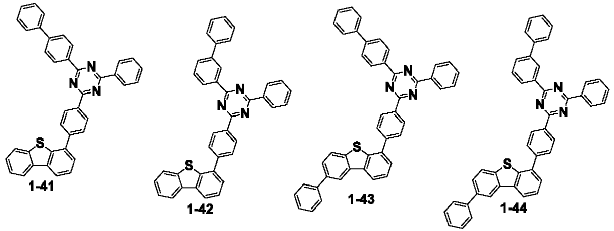

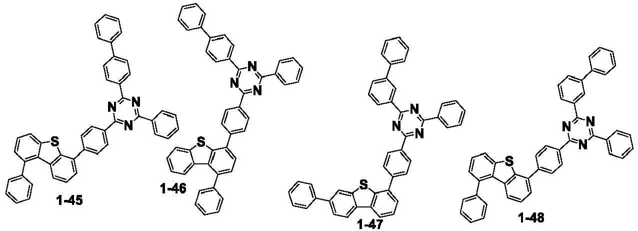

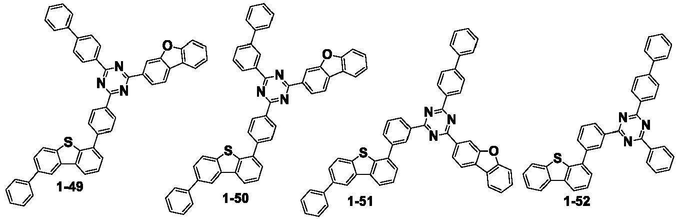

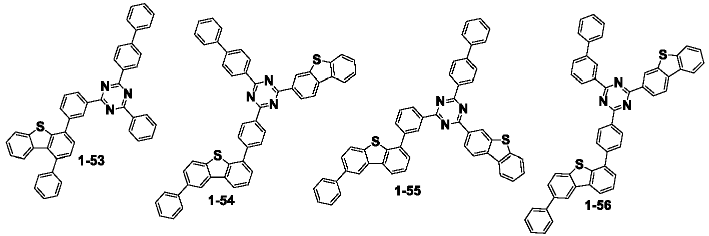

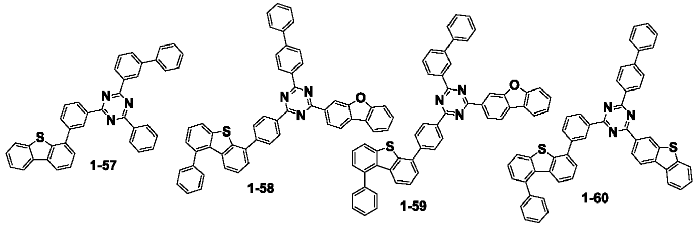

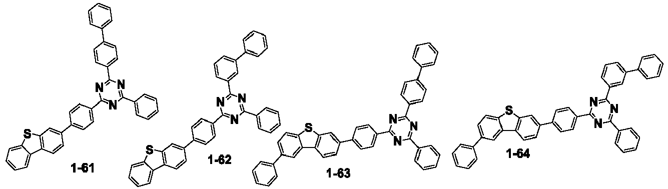

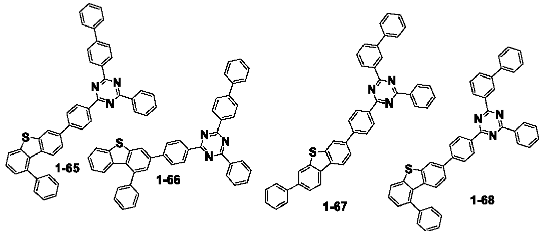

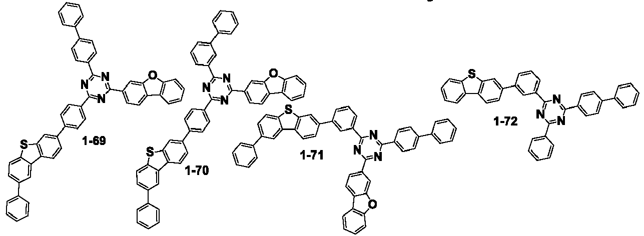

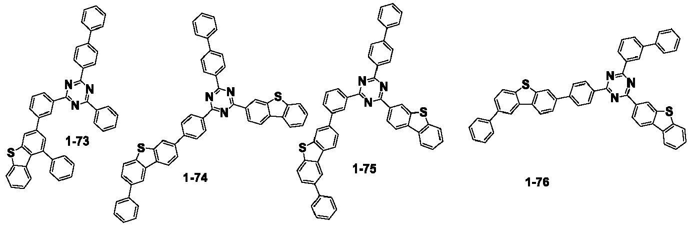

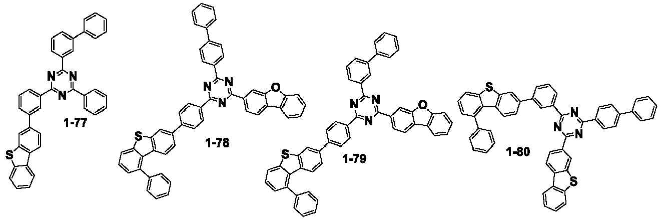

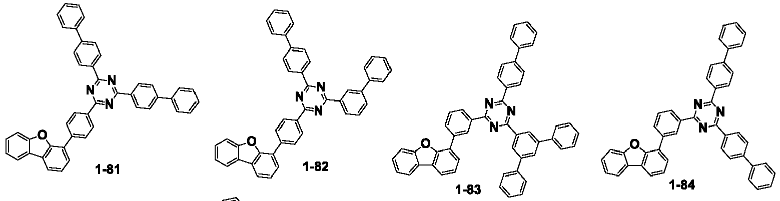

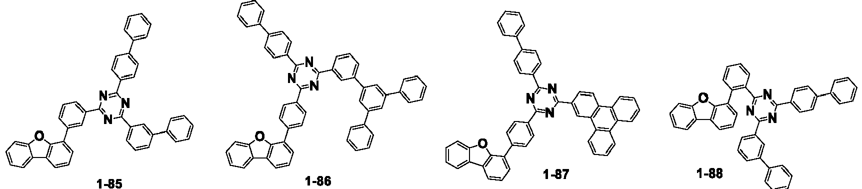

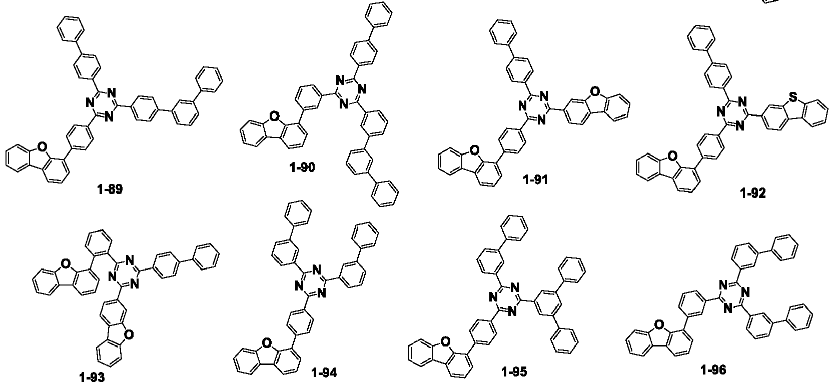

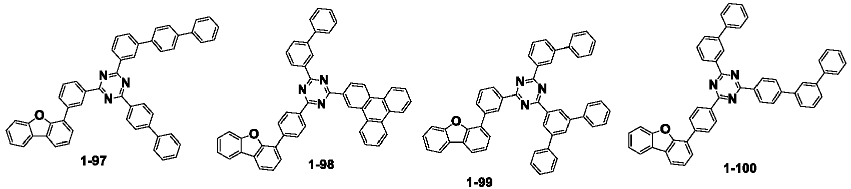

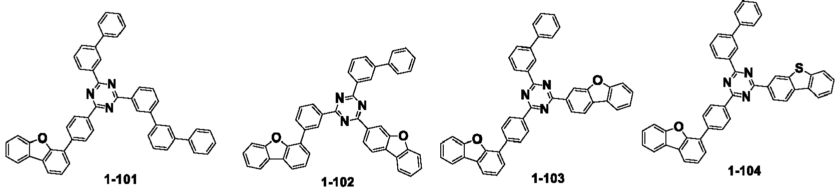

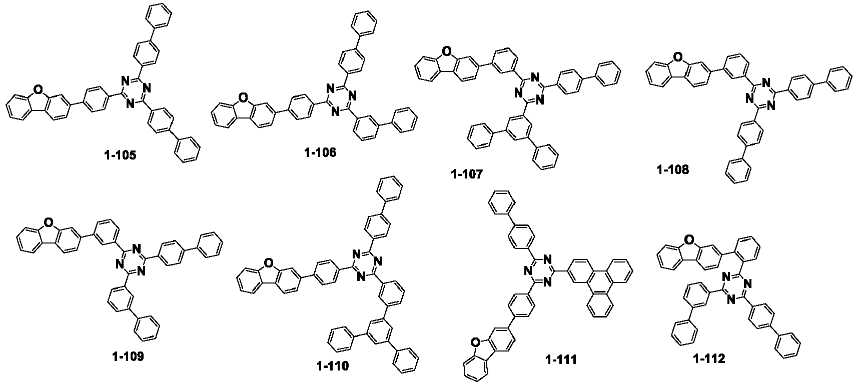

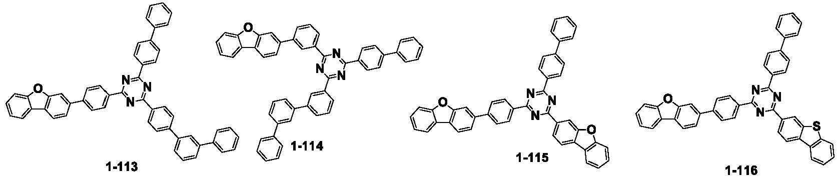

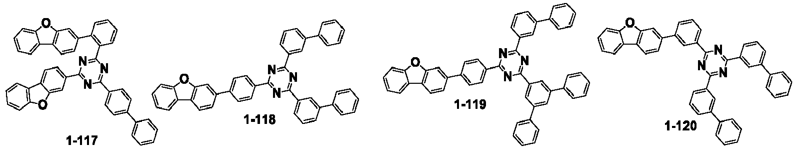

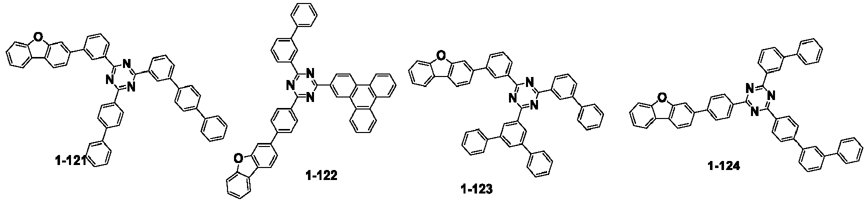

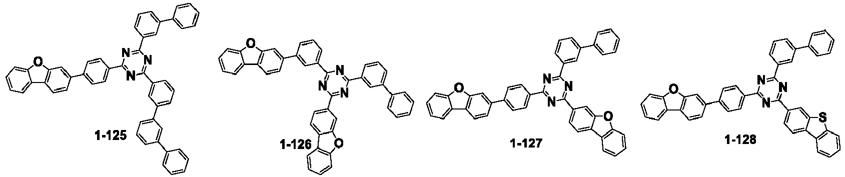

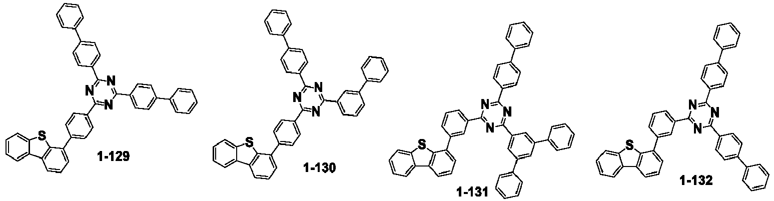

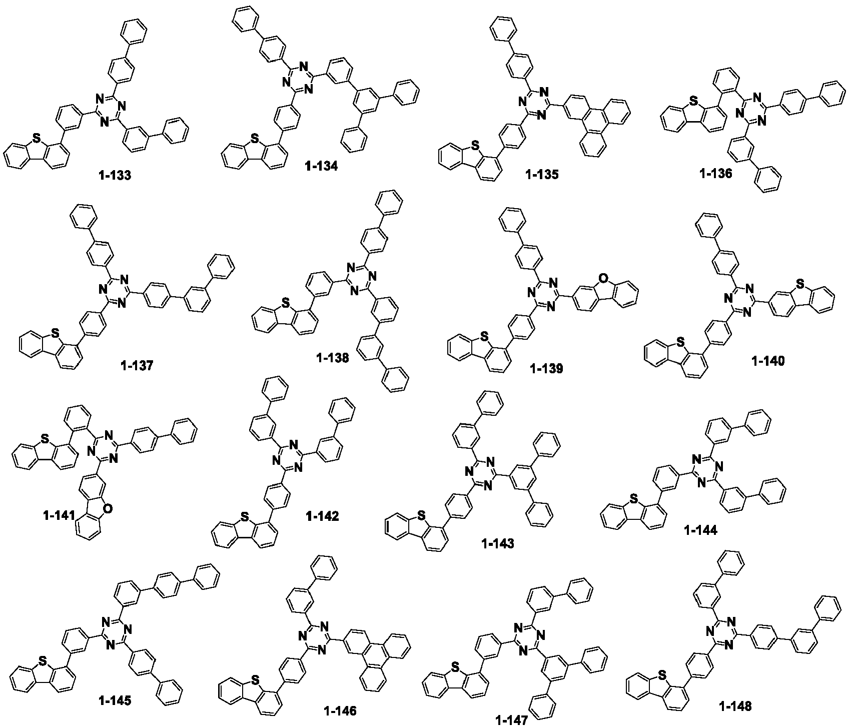

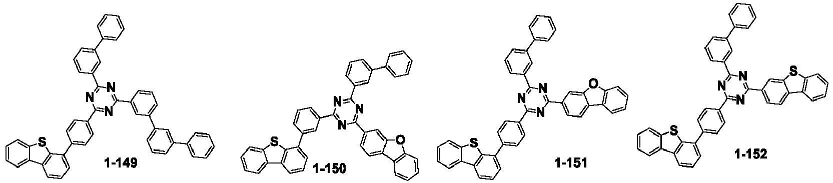

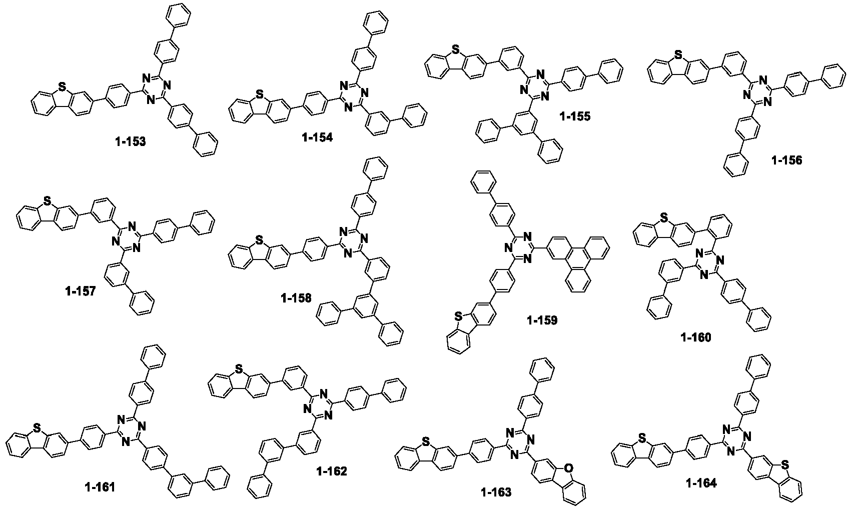

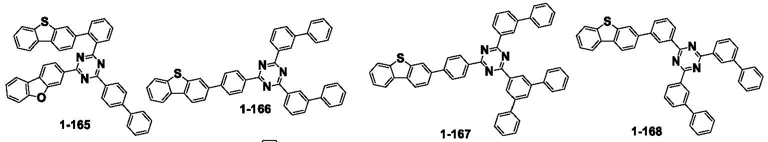

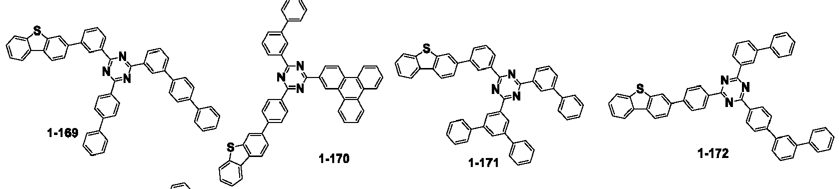

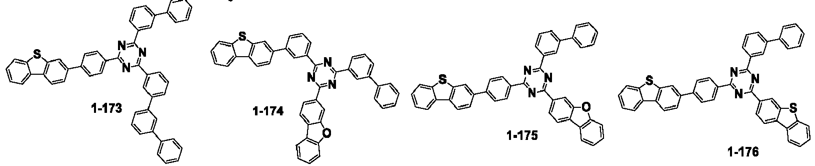

In an embodiment of the present application, Chemical Formula 2 above may be represented by any one of compounds below, but is not limited thereto.

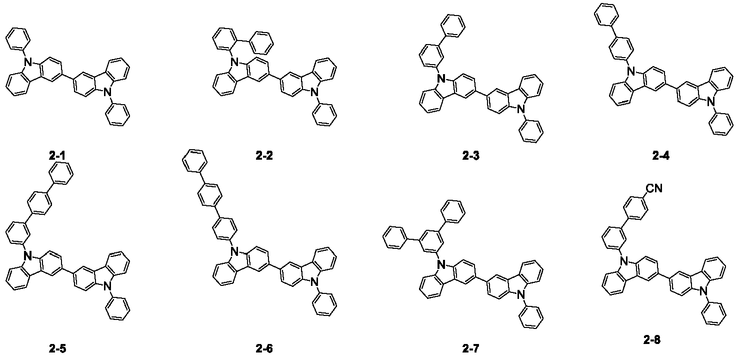

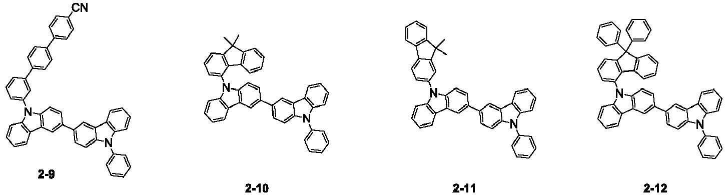

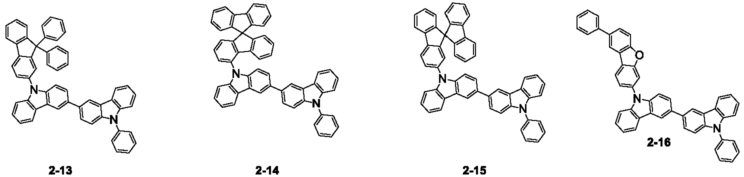

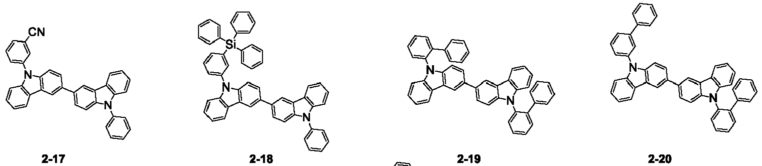

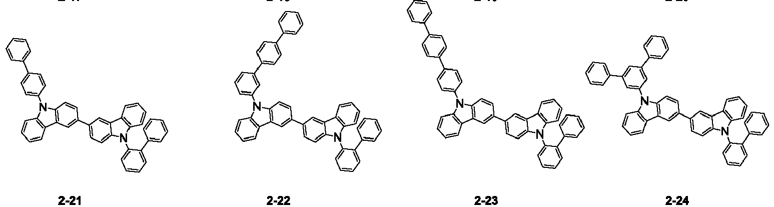

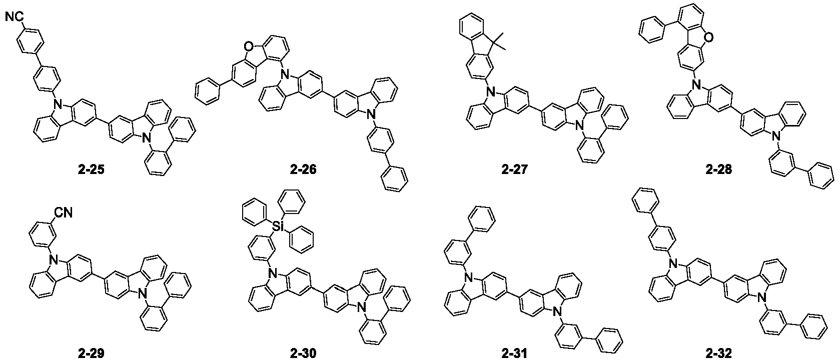

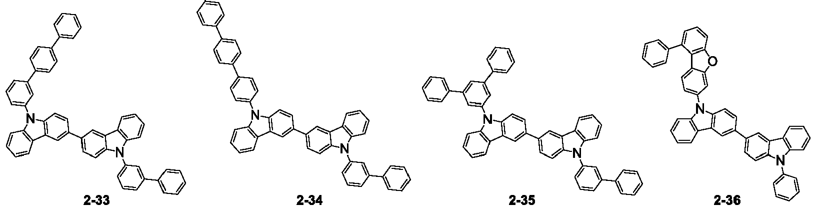

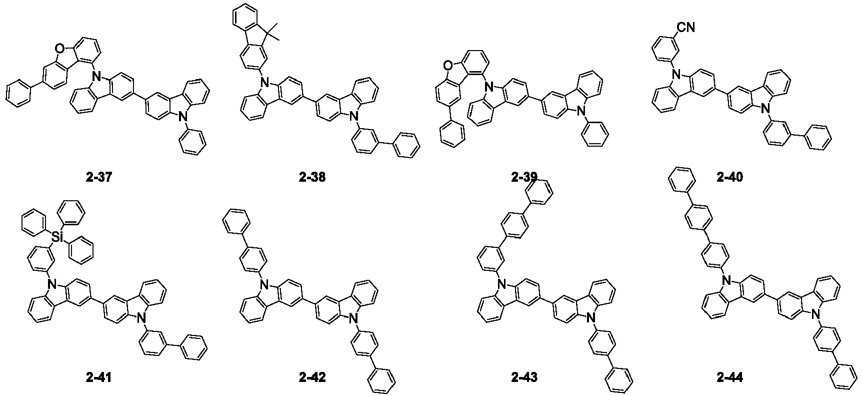

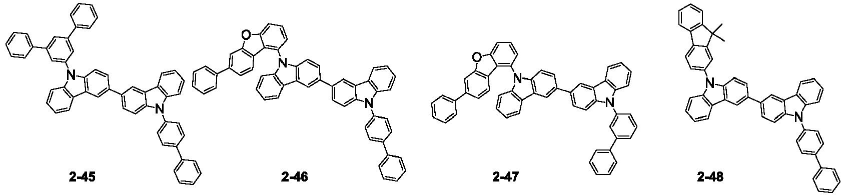

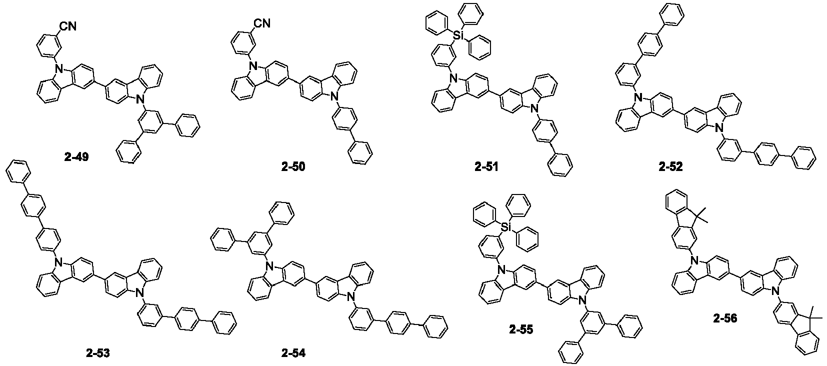

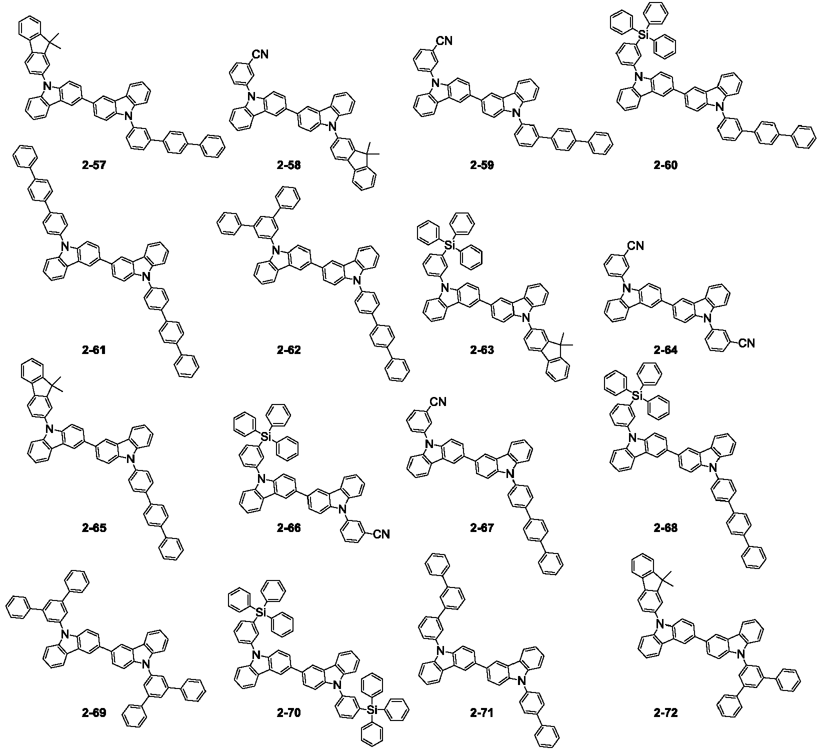

Further, compounds having intrinsic properties of the introduced substituents may be synthesized by introducing various substituents into the structures of Chemical Formulas 1 and 2 above. For example, materials satisfying the conditions required in each organic material layer may be synthesized by introducing into the core structure the substituents mainly used for a hole injection layer material, a hole transport layer material, a light emitting layer material, an electron transport layer material, and a charge generation layer material used during manufacturing of an organic light emitting device.

Further, various substituents are introduced into the structures of Chemical Formulas 1 and 2 above so that the energy band gap can be finely controlled, whereas the properties at the interface between organic materials may be improved, and the use of the material may be diversified.

Meanwhile, the heterocyclic compound has a high glass transition temperature (Tg) to have excellent thermal stability. Such an increase in thermal stability becomes an important factor providing driving stability to the device.

The heterocyclic compound according to an embodiment of the present application may be prepared by a multi-step chemical reaction. Some intermediate compounds are prepared first, and compounds of Chemical Formula 1 or 2 may be prepared from the intermediate compounds. More specifically, the heterocyclic compound according to an embodiment of the present application may be prepared based on Preparation Examples to be described later.

Further, another embodiment of the present application provides a composition for the organic material layer of the organic light emitting device, comprising the heterocyclic compound represented by Chemical Formula 1 above and the heterocyclic compound represented by Chemical Formula 2 above.

Specific details of the heterocyclic compound represented by Chemical Formula 1 above and the heterocyclic compound represented by Chemical Formula 2 above are the same as described above.

The weight ratio of the heterocyclic compound represented by Chemical Formula 1 above to the heterocyclic compound represented by Chemical Formula 2 above in the composition may be 1:10 to 10:1, 1:8 to 8:1, 1:5 to 5:1, and 1:2 to 2:1, but is not limited thereto.

The composition may be used when forming an organic material of an organic light emitting device, and in particular, it may be more preferably used when forming a host of a light emitting layer.

The composition is a form in which two or more compounds are simply mixed, and a material in a powder state may be mixed before forming an organic material layer of an organic light emitting device, or a compound in a liquid state at an appropriate temperature or higher may be mixed. The composition is in a solid state at a melting point or lower of each material, and may be maintained in a liquid state if the temperature is adjusted.

The composition may further comprise materials known in the art such as solvents, additives, etc.

The organic light emitting device according to an embodiment of the present application may be manufactured by conventional manufacturing method and material of the organic light emitting device except that one or more organic material layers are formed using the heterocyclic compound represented by Chemical Formula 1 and the heterocyclic compound represented by Chemical Formula 2 described above.

The heterocyclic compound represented by Chemical Formula 1 above and the heterocyclic compound represented by Chemical Formula 2 above may be formed into an organic material layer by a solution application method as well as a vacuum deposition method when manufacturing an organic light emitting device. Here, the solution application method refers to spin coating, dip coating, inkjet printing, screen printing, spraying, roll coating, etc., but is not limited thereto.

The organic material layer of the organic light emitting device of the present disclosure may be formed in a single-layer structure, but may be formed in a multilayer structure in which two or more organic material layers are stacked. For example, the organic light emitting device of the present disclosure may have a structure comprising a hole injection layer, a hole transport layer, a light emitting layer, an electron transport layer, an electron injection layer, etc. as an organic material layer. However, the structure of the organic light emitting device is not limited thereto and may comprise a smaller number of organic material layers.

Specifically, the organic light emitting device according to an embodiment of the present application comprises a first electrode, a second electrode, and an organic material layer comprising one or more layers provided between the first electrode and the second electrode, wherein the one or more layers of the organic material layer comprise a heterocyclic compound represented by Chemical Formula 1 above and a heterocyclic compound represented by Chemical Formula 2 above.

In an embodiment of the present application, the first electrode may be an anode, and the second electrode may be a cathode.

In another embodiment, the first electrode may be a cathode, and the second electrode may be an anode.

In an embodiment of the present application, the organic light emitting device may be a blue organic light emitting device, and the heterocyclic compound according to Chemical Formula 1 above and the heterocyclic compound according to Chemical Formula 2 above may be used as a material of the blue organic light emitting device.

In an embodiment of the present application, the organic light emitting device may be a green organic light emitting device, and the heterocyclic compound represented by Chemical Formula 1 above and the heterocyclic compound represented by Chemical Formula 2 above may be used as a material of the green organic light emitting device.

In an embodiment of the present application, the organic light emitting device may be a red organic light emitting device, and the heterocyclic compound represented by Chemical Formula 1 above and the heterocyclic compound represented by Chemical Formula 2 above may be used as a material of the red organic light emitting device.

The organic light emitting device of the present disclosure may further comprise one layer or two or more layers selected from the group comprising a light emitting layer, a hole injection layer, a hole transport layer, an electron injection layer, an electron transport layer, an electron blocking layer, and a hole blocking layer.

In an embodiment of the present application, there is provided an organic light emitting device in which the organic material layer comprises at least one layer of a hole blocking layer, an electron injection layer, and an electron transport layer, and the at least one layer of the hole blocking layer, the electron injection layer, and the electron transport layer comprises a heterocyclic compound represented by Chemical Formula 1 above and a heterocyclic compound represented by Chemical Formula 2 above.

In an embodiment of the present application, there is provided an organic light emitting device in which the organic material layer comprises a light emitting layer, and the light emitting layer comprises a heterocyclic compound represented by Chemical Formula 1 above and a heterocyclic compound represented by Chemical Formula 2 above.

In an embodiment of the present application, there is provided an organic light emitting device in which the organic material layer comprises a light emitting layer, the light emitting layer comprises a host material, and the host material comprises a heterocyclic compound represented by Chemical Formula 1 above and a heterocyclic compound represented by Chemical Formula 2 above.

FIGS. 1 to 3 exemplify a lamination order of electrodes and organic material layers of an organic light emitting device according to an embodiment of the present application. However, it is not intended that the scope of the present application be limited by these drawings, and the structure of an organic light emitting device known in the art may also be applied to the present application.

Referring to FIG. 1, an organic light emitting device in which an anode 200, an organic material layer 300, and a cathode 400 are sequentially laminated on a substrate 100 is illustrated. However, it is not limited to such a structure, and as shown in FIG. 2, an organic light emitting device in which a cathode, an organic material layer, and an anode are sequentially laminated on a substrate may also be implemented.

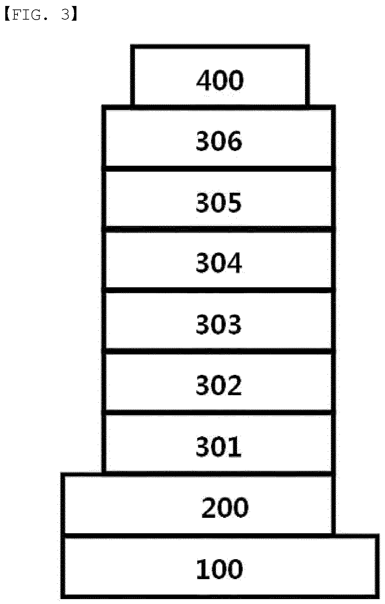

FIG. 3 exemplifies a case in which the organic material layer is multiple layers. The organic light emitting device according to FIG. 3 comprises a hole injection layer 301, a hole transport layer 302, a light emitting layer 303, a hole blocking layer 304, an electron transport layer 305, and an electron injection layer 306. However, the scope of the present application is not limited by such a lamination structure, the remaining layers except for the light emitting layer as needed may be omitted, and other necessary functional layers may be further added.

In an embodiment of the present application, there is provided a method for manufacturing an organic light emitting device, comprising the steps of: preparing a substrate; forming a first electrode on the substrate; forming an organic material layer comprising one or more layers on the first electrode; and forming a second electrode on the organic material layer, wherein the step of forming the organic material layer comprises a step of forming an organic material layer comprising one or more layers using the composition for the organic material layer according to an embodiment of the present application.

In an embodiment of the present application, there is provided a method for manufacturing an organic light emitting device, in which the step of forming the organic material layer is forming the organic material layer using a thermal vacuum deposition method by premixing the heterocyclic compound of Chemical Formula 1 above and the heterocyclic compound of Chemical Formula 2 above.

The premixing refers to mixing the premixed materials in the container after premixing materials and putting the premixed materials into one container before depositing the heterocyclic compound of Chemical Formula 1 above and the heterocyclic compound of Chemical Formula 2 above on the organic material layer.

The premixed materials may be referred to as the composition for the organic material layer according to an embodiment of the present application.

In the organic light emitting device according to an embodiment of the present application, although materials other than the heterocyclic compound of Chemical Formula 1 above and the heterocyclic compound of Chemical Formula 2 above are exemplified below, these are for illustration only, not for limiting the scope of the present application, and may be replaced with materials known in the art.

As an anode material, materials having a relatively high work function may be used, and transparent conductive oxides, metals, conductive polymers, or the like may be used. Specific examples of the anode material may comprise metals such as vanadium, chromium, copper, zinc, and gold, or alloys thereof; metal oxides such as zinc oxide, indium oxide, indium tin oxide (ITO), and indium zinc oxide (IZO); ZnO: Al or SnO₂: combinations of metals such as Sb and oxides; conductive polymers such as poly(3-methylthiophene), poly[3,4-(ethylene-1,2-dioxy)thiophene] (PEDOT), polypyrrole, and polyaniline; etc., but are not limited thereto.

As a cathode material, materials having a relatively low work function may be used, and metals, metal oxides, conductive polymers, or the like may be used. Specific examples of the cathode material may comprise metals such as magnesium, calcium, sodium, potassium, titanium, indium, yttrium, lithium, gadolinium, aluminum, silver, tin, and lead, or alloys thereof; a multilayered material such as LiF/Al or LiO₂/Al; etc., but are not limited thereto.

As a hole injection material, known hole injection materials may be used. For example, a phthalocyanine compound such as copper phthalocyanine or the like disclosed in US Pat. No. 4,356,429, or starburst-type amine derivatives disclosed in the literature [Advanced Material, 6, p.677 (1994)] such as tris (4-carbazolyl-9-ylphenyl) amine (TCTA), 4, 4', 4"-tri [phenyl (m-tolyl)amino]triphenylamine (m-MTDATA), 1,3,5-tris[4-(3-methylphenylphenylamino)phenyl]benzene (m-MTDAPB), a soluble conductive polymer Polyaniline/Dodecylbenzenesulfonic acid or Poly(3,4-ethylenedioxythiophene)/Poly(4-styrenesulfonate), Polyaniline/Camphor sulfonic acid, Polyaniline/Poly(4-styrene-sulfonate), or the like may be used.

As a hole transport material, pyrazoline derivatives, arylamine-based derivatives, stilbene derivatives, triphenyldiamine derivatives, etc. may be used, and low molecular weight or high molecular weight materials may also be used.

As an electron transport material, metal complexes of oxadiazole derivatives, anthraquinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, and 8-hydroxyquinoline and its derivatives may be used, and high molecular weight materials as well as low molecular weight materials may also be used.

As an electron injection material, for example, LiF is typically used in the art, but the present application is not limited thereto.

As a light emitting material, a red, green, or blue light emitting material may be used, and if necessary, two or more light emitting materials may be mixed and used. At this time, two or more light emitting materials may be deposited and used as individual sources, or may be premixed and deposited as a single source and used. Further, a fluorescent material may be used as a light emitting material, but a phosphorescent material may also be used. As the light emitting material, a material that emits light by combining holes and electrons respectively injected from the anode and the cathode may be used alone, but materials in which the host material and the dopant material together involve in light emission may also be used.

When mixing and using the host of the light emitting material, hosts of the same series may be mixed and used, or hosts of different series may be mixed and used. For example, any two or more types of n-type host materials and p-type host materials may be selected and used as a host material of the light emitting layer.

The organic light emitting device according to an embodiment of the present application may be a top emission type, a back emission type, or a double side emission type depending on materials used.

A heterocyclic compound according to an embodiment of the present application may act on a principle similar to that applied to an organic light emitting device even in an organic electronic device comprising an organic solar cell, an organic photoreceptor, an organic transistor, etc.

Hereinafter, the present specification will be described in more detail through Examples, but these are only for exemplifying the present application and not for limiting the scope of the present application.

### [Preparation Example 1] Preparation of Compound 1-81 (C-1)

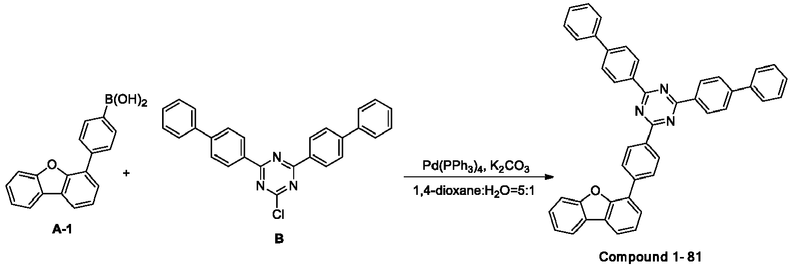

### Preparation of Compound 1-81

10 g (34.7 mmol) of (4-(dibenzo[b,d]furan-4-yl)phenyl)boronic acid, 17.5 g (41.6 mmol) of 2,4-di([1,1'-biphenyl]-4-yl)-6-chloro-1,3,5-triazine, 4 g (3.5 mmol) of tetrakis(triphenylphosphine)palladium (0), 14.4 g (104.1 mmol) of potassium carbonate, and a 1,4-dioxane/water mixture (100 ml/20 ml) were refluxed in an one neck round bottom flask at 120 °C for 3 hours. When the reaction was completed, the temperature was lowered to room temperature, and a reaction product was washed with 1,4-dioxane, distilled water, and MeOH to obtain Compound 1-81 (18 g, 82%).

In Preparation Example 1 above, Target Compound (C-1) was synthesized by performing the synthesis process in the same manner as in Preparation Example 1 above except that Compound A-1 of Table 1 below was used instead of (4-(dibenzo[b,d]furan-4-yl)phenyl)boronic acid, and Compound B of Table 1 below was used instead of 2,4-di([1,1'-biphenyl]-4-yl)-6-chloro-1,3,5-triazine.

**[Table 1]**

| Compound | A-1 | B | C-1 | Yield (%) |
|---|---|---|---|---|
| 1-82 | 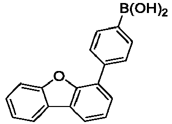 | 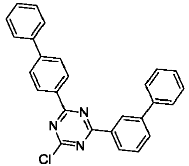 | 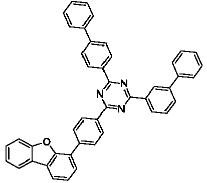 | 52 |
| 1-86 | 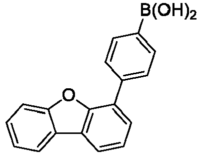 | 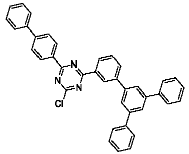 | 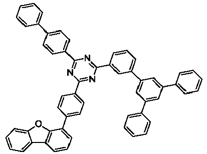 | 63 |
| 1-91 | 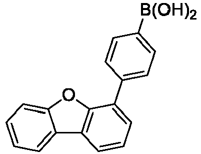 | 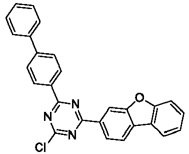 | 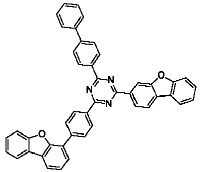 | 72 |
| 1-96 | 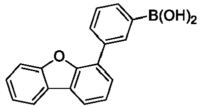 | 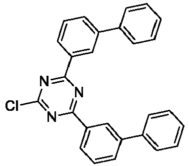 | 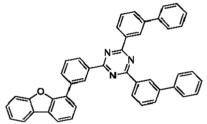 | 75 |
| 1-98 | 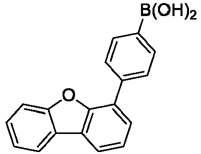 | 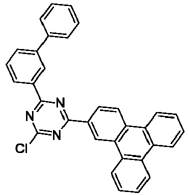 | 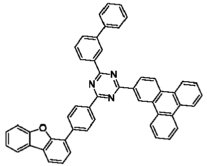 | 68 |
| 1-102 | 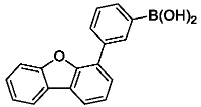 | 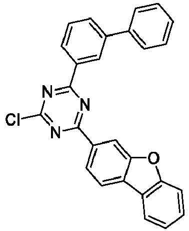 | 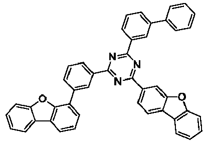 | 80 |
| 1-107 | 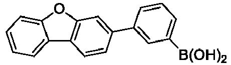 | 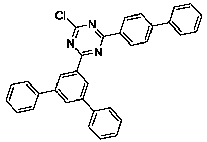 | 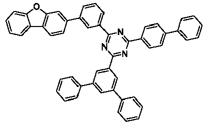 | 65 |
| 1-111 | 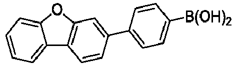 | 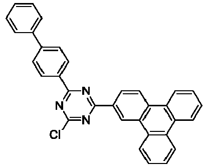 | 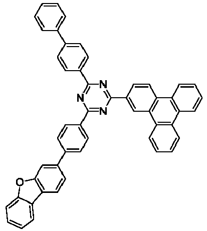 | 45 |
| 1-120 | 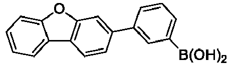 | 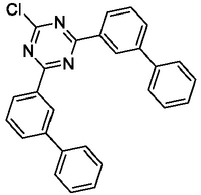 | 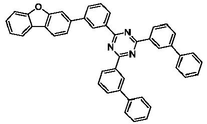 | 77 |
| 1-128 | 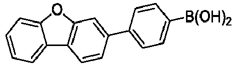 | 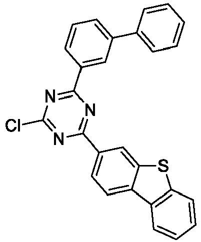 | 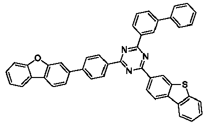 | 71 |

### [Preparation Example 2] Preparation of Compound 1-129 (C-2)

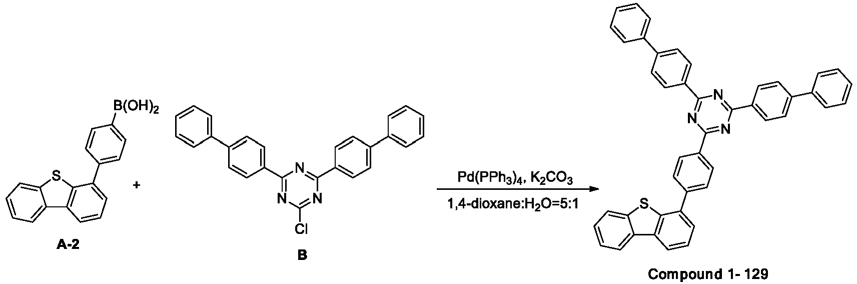

### Preparation of Compound 1-129

10 g (32.9 mmol) of (4-(dibenzo[b,d]thiophen-4-yl)phenyl)boronic acid, 16.5 g (39.5 mmol) of 2,4-di([1,1'-biphenyl]-4-yl)-6-chloro-1,3,5-triazine, 3.8 g (3.3 mmol) of tetrakis(triphenylphosphine)palladium(0), 13.6 g (98.6 mmol) of potassium carbonate, and a 1,4-dioxane/water mixture (100 ml/20 ml) were refluxed in an one neck round bottom flask at 120 °C for 3 hours. After performing filtration at 120 °C, a reaction product was washed with 1,4-dioxane, distilled water, and MeOH at 120 °C to obtain Compound 1-129 (15g, 70%).

In Preparation Example 2 above, Target Compound (C-2) was synthesized by performing the synthesis process in the same manner as in Preparation Example 2 above except that Compound A-2 of Table 2 below was used instead of (4-(dibenzo[b,d]furan-4-yl)phenyl)boronic acid, and Compound B of Table 2 below was used instead of 2,4-di([1,1'-biphenyl]-4-yl)-6-chloro-1,3,5-triazine.

**[Table 2]**

| Compound | A-2 | B | C-2 | Yiel d (%) |
|---|---|---|---|---|
| 1-130 | 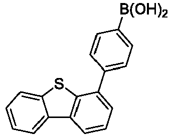 | 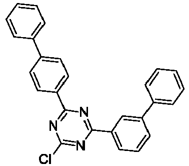 | 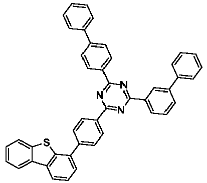 | 52 |
| 1-134 | 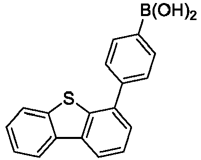 | 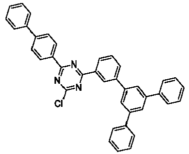 | 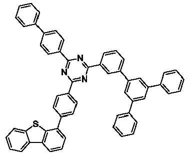 | 63 |
| 1-139 | 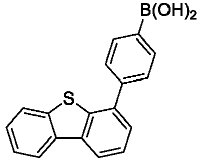 | 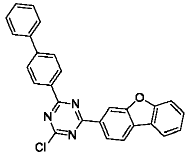 | 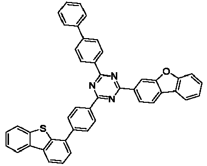 | 72 |
| 1-144 | 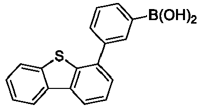 | 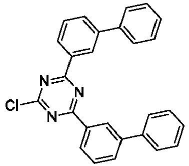 | 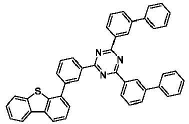 | 75 |
| 1-146 | 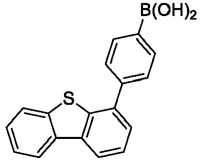 | 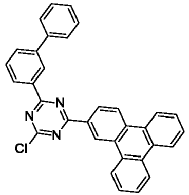 | 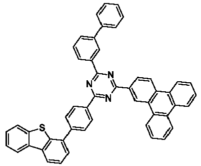 | 68 |
| 1-150 | 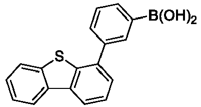 | 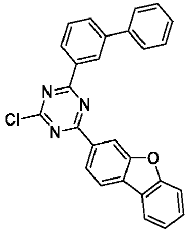 | 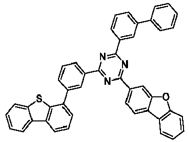 | 80 |
| 1-155 | 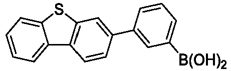 | 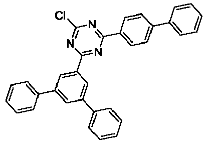 | 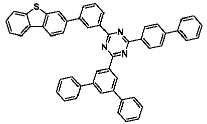 | 65 |
| 1-159 | 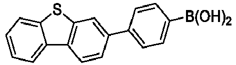 | 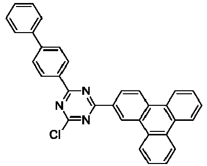 | 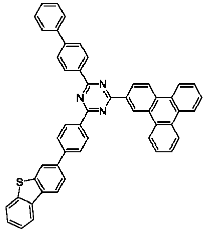 | 75 |
| 1-168 | 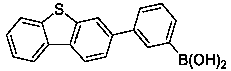 | 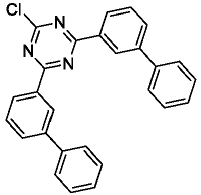 | 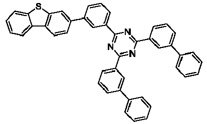 | 64 |
| 1-176 | 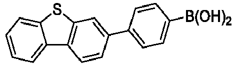 | 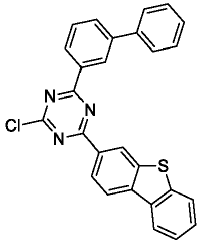 | 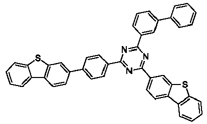 | 74 |

### [Preparation Example 3] Preparation of Compound 1-1 (C-3)

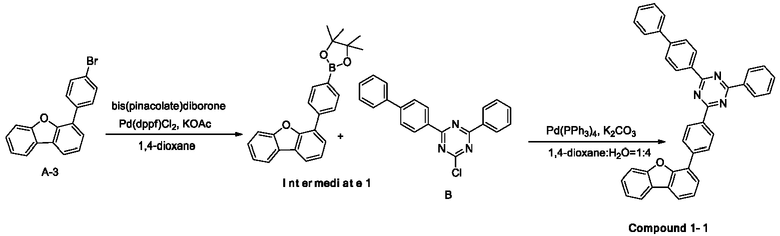

### Synthesis Method of Intermediate 1

After putting 20 g (61.88 mmol) of 4-(4-bromophenyl)dibenzo[b,d]furan(A), 31.4g (123.7 mmol) of bis(pinacolate)diboron, 4.52 g (6.18 mmol) of Pd(dppf)Cl₂, and 18.2 g (185.64 mmol) of KOA_{c} into a reaction flask and putting 1,4-dioxane thereinto, the materials were refluxed at 120 °C for 3 hours. When the reaction was completed, the temperature was lowered to room temperature, the reaction solution was extracted with MC and H₂O, and the organic layer was purified by column to obtain Intermediate 1 (18 g, 81%).

### Synthesis Method of Compound 1-1 (C-3)

After putting 18 g (48.6 mmol) of Intermediate 1, 20 g (58.3 mmol) of 2-([1,1'-biphenyl]-4-yl)-4-chloro-6-phenyl-1,3,5-triazine, 2.8 g (2.43 mmol) of Pd(PPh₃)₄, and 20.1 g (145.8 mmol) of K₂CO₃ into a reaction flask and putting a 1,4-dioxane/water mixture (180 ml/45 ml) thereinto, the materials were heated at 120 °C for 4 hours. After completing the reaction, the temperature was lowered to room temperature, and the resulting solid was washed with distilled water and MeOH to obtain Compound 1-1 (C-3) (20 g, 74%).

In Preparation Example 3 above, Compound (C-3) below was synthesized in the same manner except that Compounds A-3 and B of Table 3 below were used.

**[Table 3]**

| Compound | A-3 | B | C-3 | Yield (%) |
|---|---|---|---|---|
| 1 - 2 | 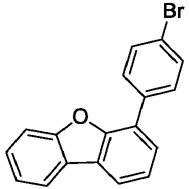 | 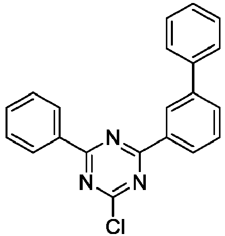 | 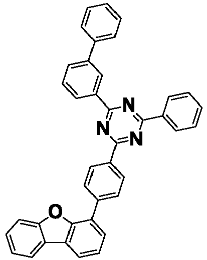 | 74 |
| 1 - 12 | 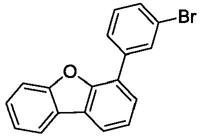 | 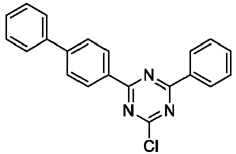 | 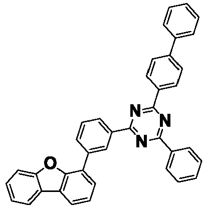 | 80 |
| 1 - 17 | 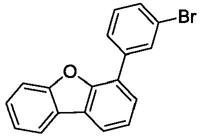 | 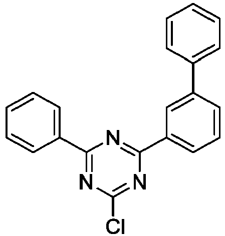 | 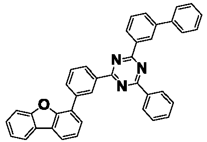 | 83 |
| 1 - 21 | 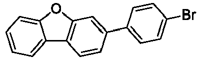 | 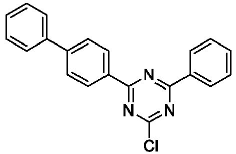 | 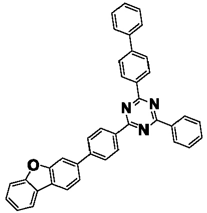 | 88 |
| 1 - 22 | 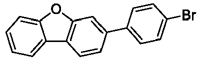 | 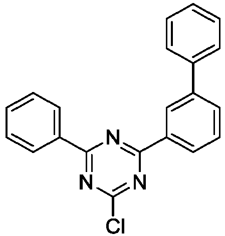 | 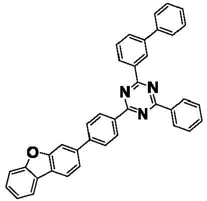 | 82 |
| 1 - 32 | 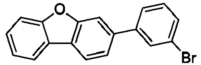 | 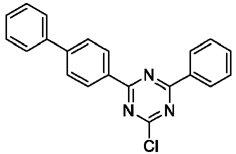 | 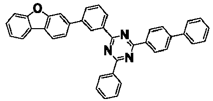 | 80 |
| 1 - 37 | 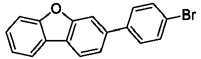 | 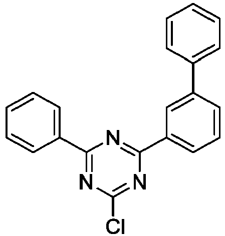 | 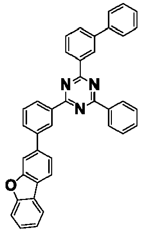 | 87 |
| 1 - 41 | 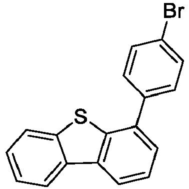 | 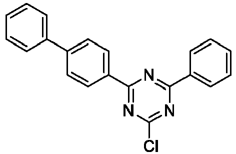 | 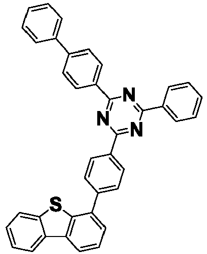 | 85 |
| 1 - 42 | 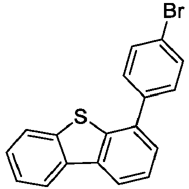 | 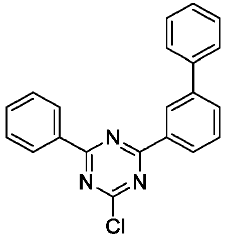 | 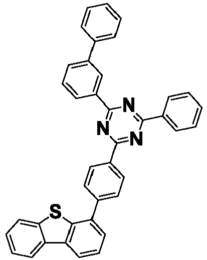 | 87 |
| 1 - 52 | 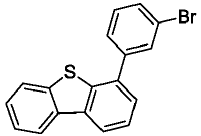 | 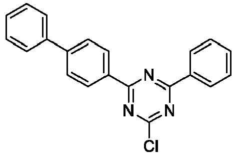 | 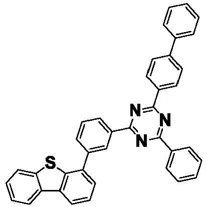 | 81 |
| 1 - 57 | 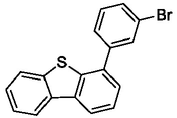 | 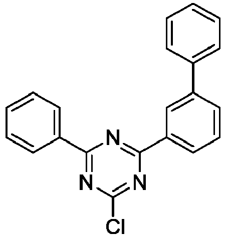 | 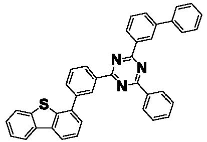 | 80 |
| 1 - 61 | 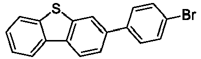 | 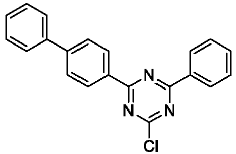 | 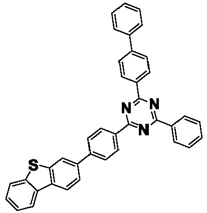 | 86 |
| 1 - 62 | 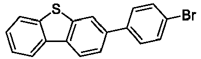 | 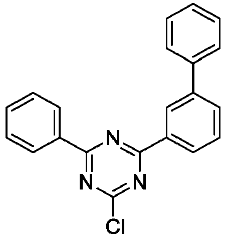 | 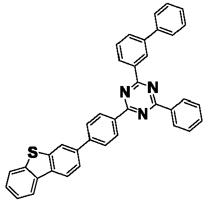 | 84 |
| 1 - 72 | 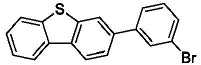 | 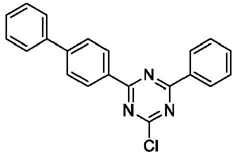 | 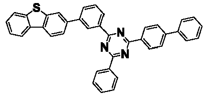 | 85 |
| 1 - 77 | 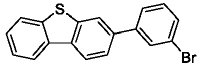 | 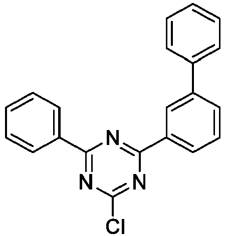 | 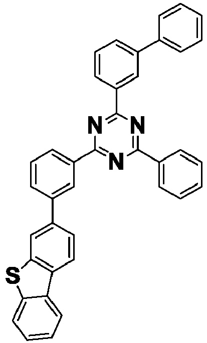 | 86 |

### [Preparation Example 4] Preparation of Compound 1-3 (C-4)

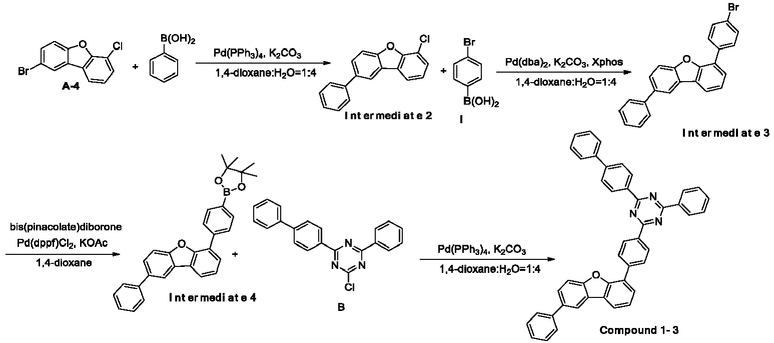

### Synthesis Method of Intermediate 2

After putting 20 g (71 mmol) of 2-bromo-6-chlorodibenzo[b,d]furan, 17.3 g (142 mmol) of phenylboronic acid, 8.2 g (7.1 mmol) of Pd(PPh₃)₄, and 29.4 g (213 mmol) of K₂CO₃ into a reaction flask and putting a 1,4-dioxane/water mixture (200 ml/50 ml) thereinto, the materials were heated at 120 °C for 4 hours. After completing the reaction, the temperature was lowered to room temperature, and the resulting solid was washed with distilled water and MeOH to obtain Intermediate 2 (15 g, 76%).

### Synthesis Method of Intermediate 3

After putting 15 g (53.8 mmol) of Intermediate 2, 12.9 g (64.5 mmol) of (4-bromophenyl)boronic acid, 0.91 g (2.69 mmol) of Pd(dba)₂, 22.3 g (161.4 mmol) of K₂CO₃, and 2.51 g (5.38 mmol) of Xphos into a reaction flask and putting a 1,4-dioxane/water mixture (150 ml/37.5 ml) thereinto, the materials were heated at 120 °C for 4 hours. After completing the reaction, the temperature was lowered to room temperature, and the resulting solid was washed with distilled water and MeOH to obtain Intermediate 3 (15 g, 70%).

### Synthesis Method of Intermediate 4

It was synthesized in the same manner as in Synthesis Method of Intermediate 1 of Preparation Example 3 above.

### Synthesis Method of Compound 1-3 (C-4)

It was synthesized in the same manner as in Synthesis Method of Compound 1-1 (C-3) of Preparation Example 3 above.

Compound (C-4) below was synthesized in the same manner except that Compounds A-4, I, and B of Table 4 below were used.

**[Table 4]**

| Compound | A-4 | I | B | C-4 | Yield (%) |
|---|---|---|---|---|---|
| 1 - 4 | 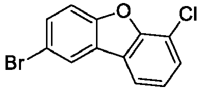 | 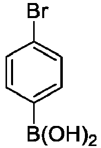 | 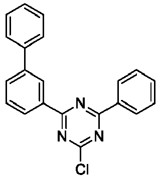 | 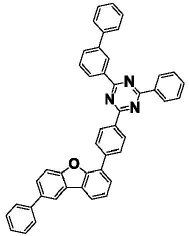 | 75 |
| 1 - 5 | 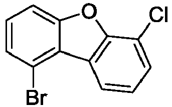 | 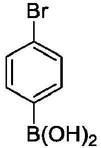 | 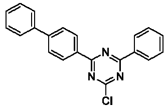 | 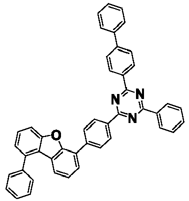 | 83 |
| 1 - 6 | 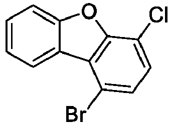 | 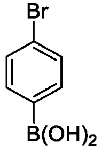 | 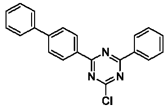 | 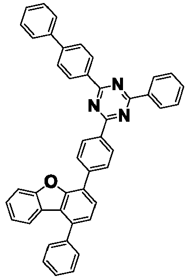 | 85 |
| 1 - 7 | 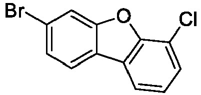 | 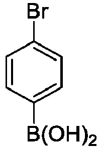 | 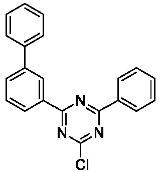 | 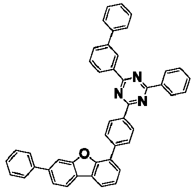 | 83 |
| 1 - 8 | 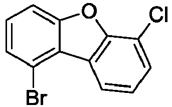 | 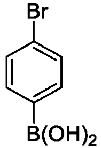 | 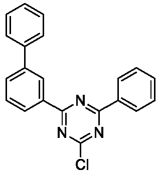 | 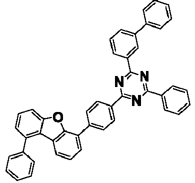 | 84 |
| 1 - 9 | 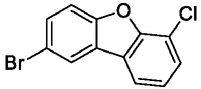 | 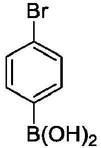 | 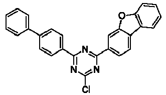 | 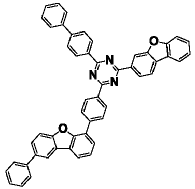 | 85 |
| 1 - 10 | 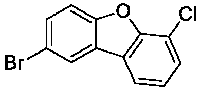 | 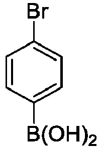 | 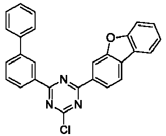 | 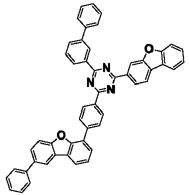 | 83 |
| 1 - 11 | 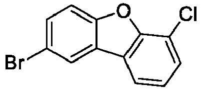 | 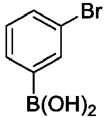 | 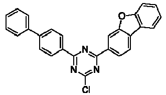 | 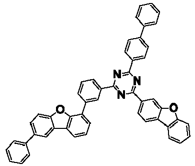 | 80 |
| 1 - 13 | 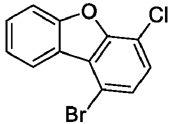 | 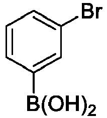 | 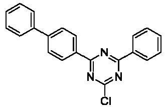 | 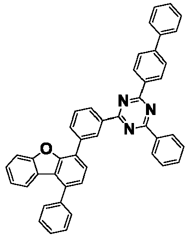 | 87 |
| 1 - 15 | 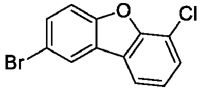 | 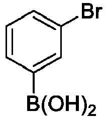 | 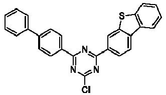 | 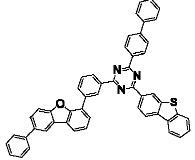 | 81 |
| 1 - 16 | 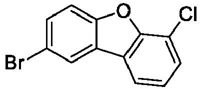 | 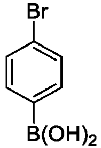 | 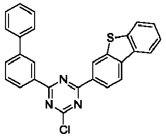 | 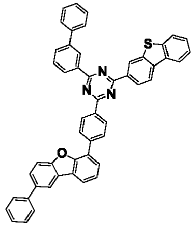 | 80 |
| 1 - 19 | 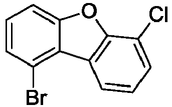 | 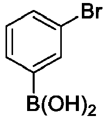 | 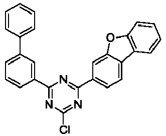 | 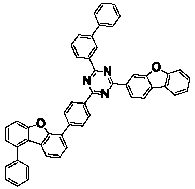 | 87 |
| 1 - 23 | 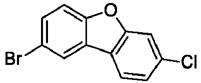 | 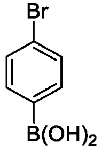 | 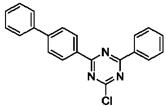 | 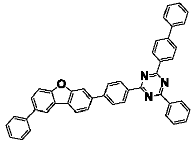 | 85 |
| 1 - 24 | 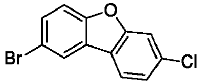 | 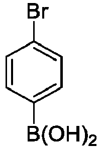 | 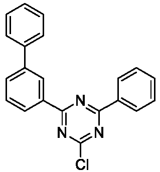 | 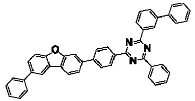 | 86 |
| 1 - 25 | 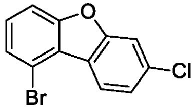 | 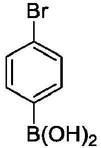 | 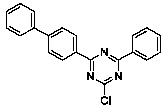 | 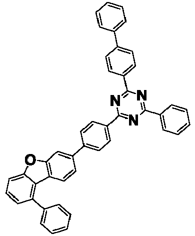 | 87 |
| 1 - 26 | 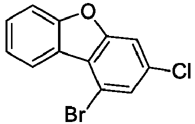 | 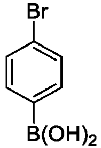 | 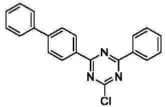 | 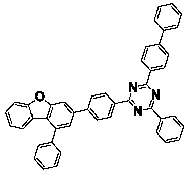 | 88 |
| 1 - 27 | 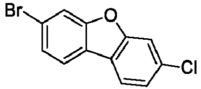 | 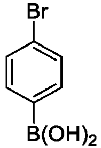 | 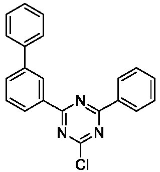 | 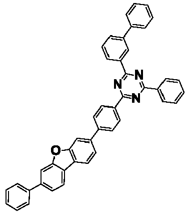 | 80 |
| 1 - 28 | 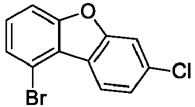 | 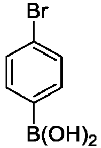 | 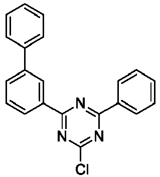 | 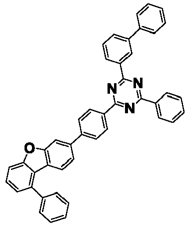 | 89 |
| 1 - 29 | 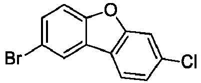 | 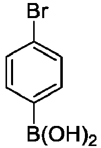 | 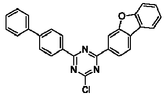 | 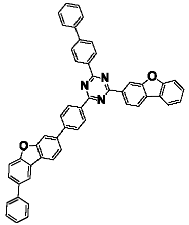 | 80 |
| 1 - 30 | 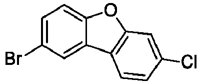 | 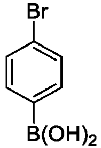 | 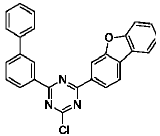 | 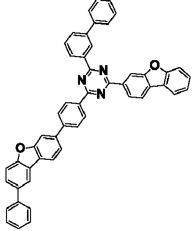 | 80 |
| 1 - 31 | 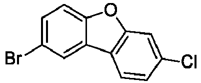 | 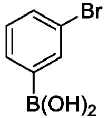 | 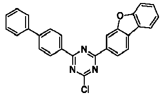 | 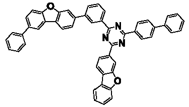 | 81 |
| 1 - 33 | 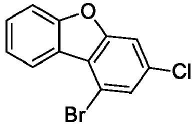 | 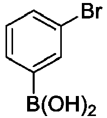 | 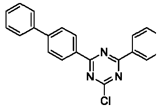 | 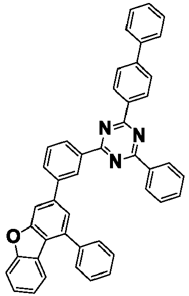 | 82 |
| 1 - 38 | 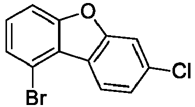 | 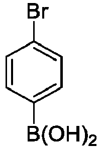 | 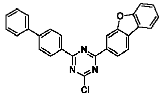 | 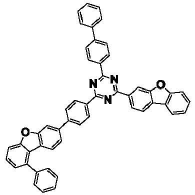 | 83 |
| 1 - 39 | 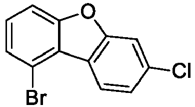 | 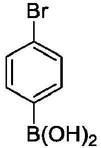 | 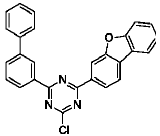 | 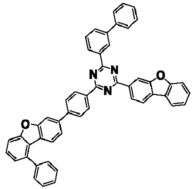 | 84 |
| 1 - 43 | 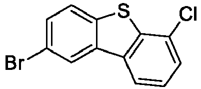 | 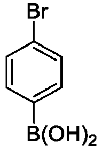 | 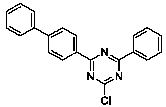 | 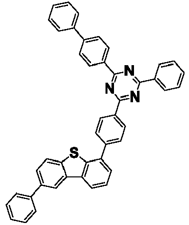 | 85 |
| 1 - 44 | 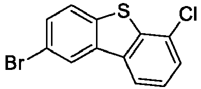 | 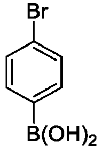 | 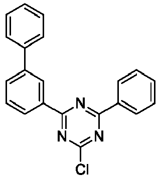 | 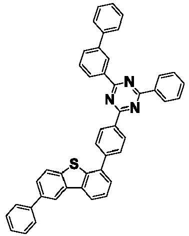 | 86 |
| 1 - 45 | 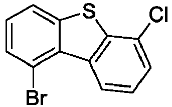 | 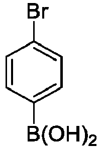 | 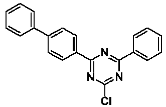 | 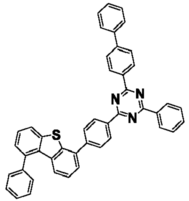 | 87 |
| 1 - 46 | 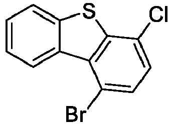 | 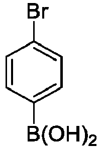 | 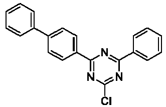 | 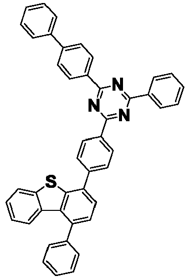 | 80 |
| 1 - 47 | 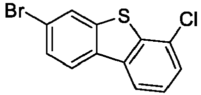 | 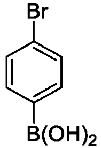 | 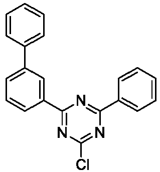 | 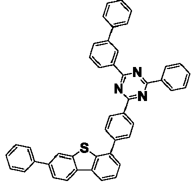 | 81 |
| 1 - 48 | 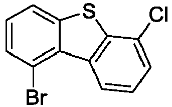 | 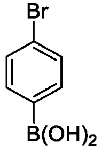 | 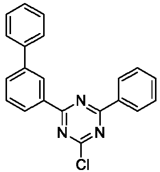 | 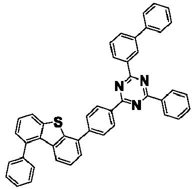 | 82 |
| 1 - 49 | 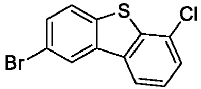 | 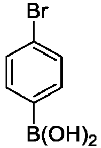 | 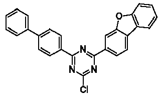 | 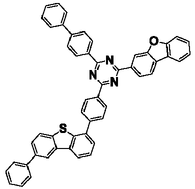 | 83 |
| 1 - 50 | 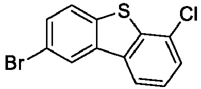 | 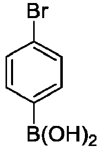 | 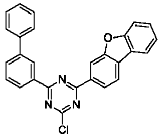 | 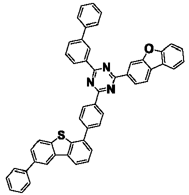 | 84 |
| 1 - 51 | 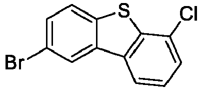 | 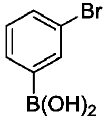 | 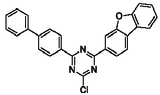 | 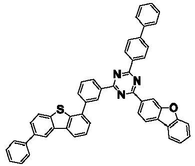 | 80 |
| 1 - 53 | 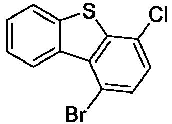 | 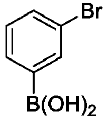 | 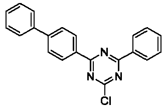 | 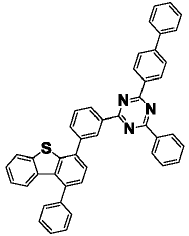 | 81 |
| 1 - 58 | 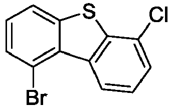 | 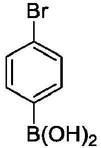 | 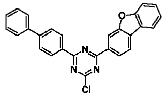 | 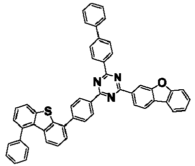 | 82 |
| 1 - 59 | 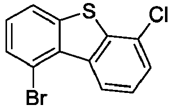 | 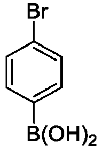 | 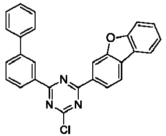 | 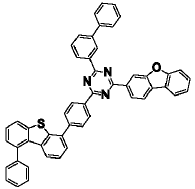 | 86 |
| 1 - 63 | 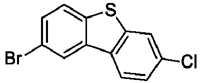 | 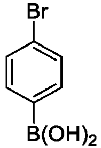 | 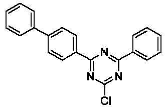 | 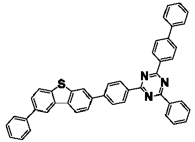 | 84 |
| 1 - 64 | 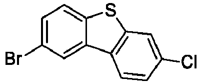 | 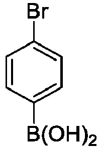 | 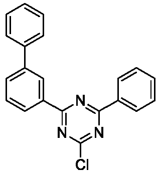 | 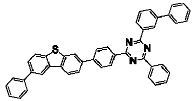 | 82 |
| 1 - 65 | 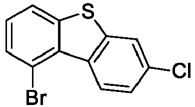 | 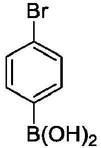 | 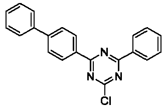 | 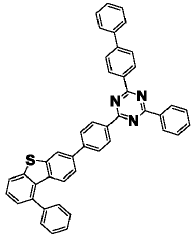 | 82 |
| 1 - 66 | 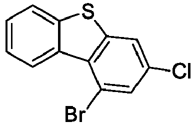 | 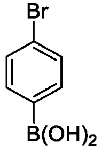 | 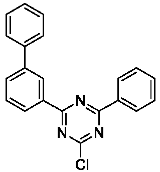 | 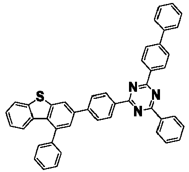 | 83 |
| 1 - 67 | 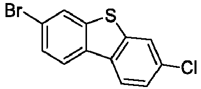 | 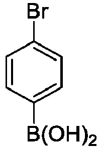 | 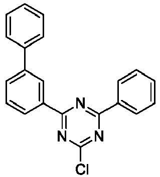 | 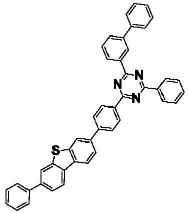 | 84 |
| 1 - 68 | 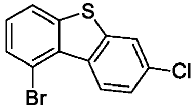 | 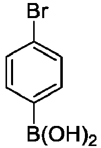 | 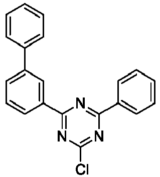 | 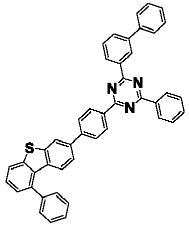 | 82 |
| 1 - 69 | 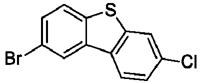 | 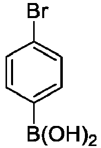 | 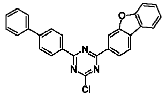 | 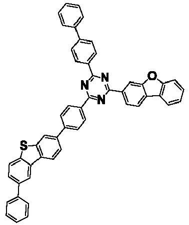 | 82 |
| 1 - 70 | 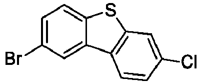 | 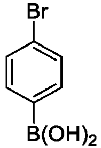 | 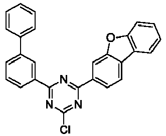 | 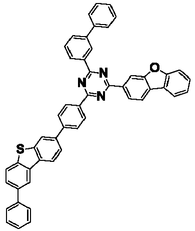 | 83 |
| 1 - 71 | 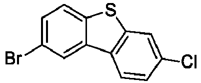 | 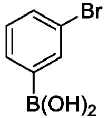 | 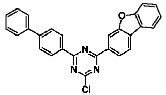 | 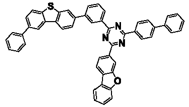 | 84 |
| 1 - 73 | 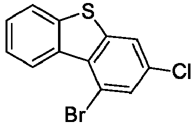 | 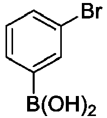 | 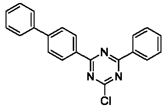 | 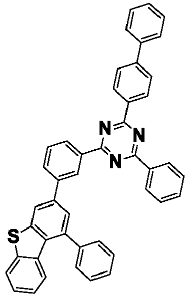 | 82 |
| 1 - 78 | 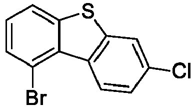 | 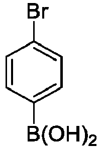 | 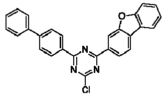 | 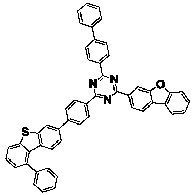 | 83 |
| 1 - 79 | 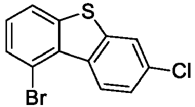 | 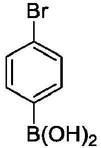 | 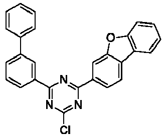 | 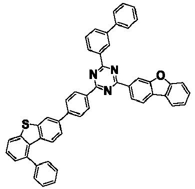 | 82 |

### <Preparation Example 5> Synthesis of Compound 2-3

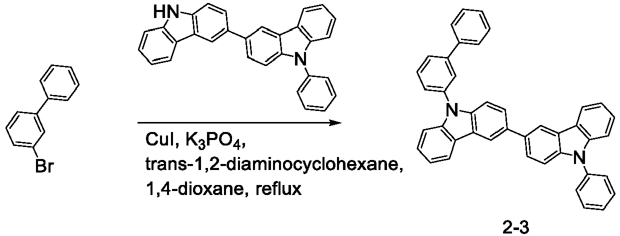

### 1) Preparation of Compound 2-3

3.7 g (15.8 mM) of 3-bromo-1,1'-biphenyl, 6.5 g (15.8 mM) of 9-phenyl-9H,9'H-3,3'-bicarbazole, 3.0 g (15.8 mM) of CuI, 1.9 mL (15.8 mM) of trans-1,2-diaminocyclohexane, and 3.3 g (31.6 mM) of K₃PO₄ were dissolved in 100 mL of 1,4-oxane and then refluxed for 24 hours. After the reaction was completed, distilled water and DCM were put at room temperature for extraction, the organic layer was dried with MgSO₄, and the solvent was removed by a rotary evaporator. The reaction product was purified by column chromatography (DCM:Hex=1:3) and recrystallized with methanol to obtain 7.5 g (85%) of Target Compound 2-3.

The target Compound A was synthesized by preparing Target Compound A in the same manner as in the preparation of Preparation Example 5 except that Intermediate A of Table 5 below was used instead of 3-bromo-1,1'-biphenyl, and Intermediate B of Table 5 below was used instead of 9-phenyl-9H, 9'H-3, 3'-bicarbazole in Preparation Example 5 above.

**[Table 5]**

| Compou nd No. | Intermediate A | Intermediate B | Target Compound A | Total yield |
|---|---|---|---|---|
| 2-4 | 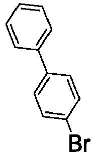 | 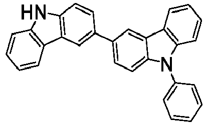 | 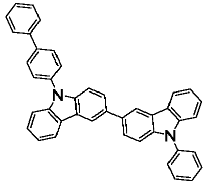 | 83% |
| 2-7 | 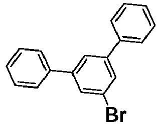 | | 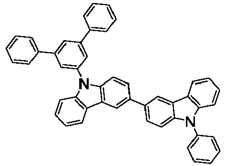 | 84% |
| 2-16 | 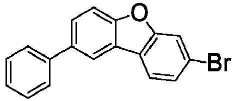 | | 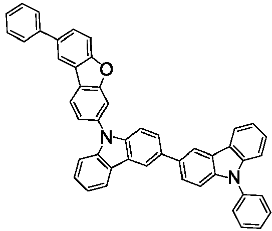 | 80% |
| 2-31 | 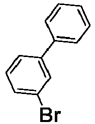 | 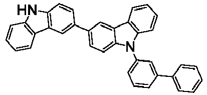 | 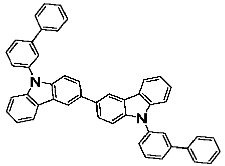 | 810 |
| 2-32 | 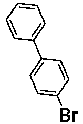 | | 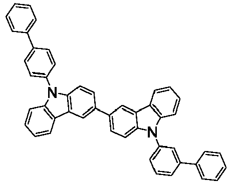 | 80% |
| 2-34 | 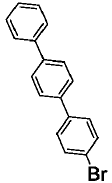 | | 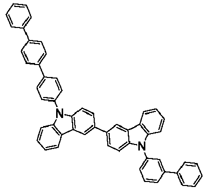 | 74% |
| 2-42 | 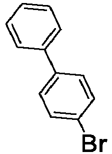 | 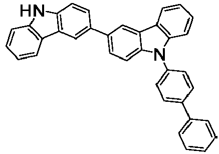 | 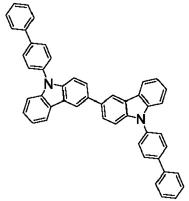 | 82% |
| 2-71 | 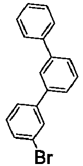 | | 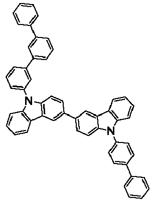 | 84% |

The heterocyclic compounds of Chemical Formulas 1 and 2 other than the compounds shown in Tables 1 to 5 above were also prepared in the same manner as described in the above-mentioned Preparation Examples.

Synthesis confirmation data of the compounds prepared above are as shown in [Table 6] and [Table 7] below.

**[Table 6]**

| Compou nd | FD-Mass | Compoun d | FD-Mass |
|---|---|---|---|
| 1-1 | m/z= 551.64 (C39H25N3O=551.20) | 1-2 | m/z= 551.64 (C39H25N3O=551.20) |
| 1-3 | m/z= 627.73 (C45H29N3O=627.23) | 1-4 | m/z= 627.73 (C45H29N3O=627.23) |
| 1-5 | m/z= 627.73 (C45H29N3O=627.23) | 1-6 | m/z= 627.73 (C45H29N3O627.23) |
| 1-7 | m/z= 627.73 (C45H29N3O=627.23) | 1-8 | m/z= 627.73 (C45H29N3O=627.23) |
| 1-9 | m/z= 717.81 (C51H31N3O2=717.24) | 1-10 | m/z= 717.81 (C51H31N3O2=717.24) |
| 1-11 | m/z= 717.81 (C51H31N3O2=717.24) | 1-12 | m/z= 551.64 (C39H25N3O=551.20) |
| 1-13 | m/z= 627.73 (C45H29N3O=627.23) | 1-14 | m/z= 733.88 (C51H31N3OS=733.22) |
| 1-15 | m/z= 733.88 (C51H31N3OS=733.22) | 1-16 | m/z= 733.88 (C51H31N3OS=733.22) |
| 1-17 | m/z= 551.64 (C39H25N3O=551.20) | 1-18 | m/z= 717.81 (C51H31N3O2=717.24) |
| 1-19 | m/z= 717.81 (C51H31N3O2=717.24) | 1-20 | m/z= 733.88 (C51H31N3OS=733.22) |
| 1-21 | m/z= 551.64 (C39H25N3O=551.20) | 1-22 | m/z= 551.64 (C39H25N3O=551.20) |
| 1-23 | m/z= 627.73 (C45H29N3O=627.23) | 1-24 | m/z= 627.73 (C45H29N3O=627.23) |
| 1-25 | m/z= 627.73 (C45H29N3O=627.23) | 1-26 | m/z= 627.73 (C45H29N3O=627.23) |
| 1-27 | m/z= 627.73 (C45H29N3O=627.23) | 1-28 | m/z= 627.73 (C45H29N3O=627.23) |
| 1-29 | m/z= 717.81 (C51H31N3O2=717.24) | 1-30 | m/z= 717.81 (C51H31N3O2=717.24) |
| 1-31 | m/z= 717.81 (C51H31N3O2=717.24) | 1-32 | m/z= 551.64 (C39H25N3O=551.20) |
| 1-33 | m/z= 627.73 (C45H29N3O=627.23) | 1-34 | m/z= 733.88 (C51H31N3OS=733.22) |
| 1-35 | m/z= 733.88 (C51H31N3OS=733.22) | 1-36 | m/z= 733.88 (C51H31N3OS=733.22) |
| 1-37 | m/z= 551.64 (C39H25N3O=551.20) | 1-38 | m/z= 717.81 (C51H31N3O2=717.24) |
| 1-39 | m/z= 717.81 (C51H31N3O2=717.24) | 1-40 | m/z= 733.88 (C51H31N3OS=733.22) |
| 1-41 | m/z= 567.70 (C39H25N3S=567.18) | 1-42 | m/z= 567.70 (C39H25N3S=567.18) |
| 1-43 | m/z= 643.80 (C45H29N3S=643.21) | 1-44 | m/z= 643.80 (C45H29N3S=643.21) |
| 1-45 | m/z= 643.80 (C45H29N3S=643.21) | 1-46 | m/z= 643.80 (C45H29N3S=643.21) |
| 1-47 | m/z= 643.80 (C45H29N3S=643.21) | 1-48 | m/z= 643.80 (C45H29N3S=643.21) |
| 1-49 | m/z= 733.88 (C51H31N3OS=733.22) | 1-50 | m/z= 733.88 (C51H31N3OS=733.22) |
| 1-51 | m/z= 733.88 (C51H31N3OS=733.22) | 1-52 | m/z= 567.70 (C39H25N3S=567.18) |
| 1-53 | m/z= 643.80 (C45H29N3S=643.21) | 1-54 | m/z= 749.94 (C51H31N3S2=749.20) |
| 1-55 | m/z= 749.94 (C51H31N3S2=749.20) | 1-56 | m/z= 749.94 (C51H31N3S2=749.20) |
| 1-57 | m/z= 567.70 (C39H25N3S=567.18) | 1-58 | m/z= 733.88 (C51H31N3OS=733.22) |
| 1-59 | m/z= 733.88 (C51H31N3OS=733.22) | 1-60 | m/z= 749.94 (C51H31N3S2=749.20) |
| 1-61 | m/z= 567.70 (C39H25N3S=567.18) | 1-62 | m/z= 567.70 (C39H25N3S=567.18) |
| 1-63 | m/z= 643.80 (C45H29N3S=643.21) | 1-64 | m/z= 643.80 (C45H29N3S=643.21) |
| 1-65 | m/z= 643.80 (C45H29N3S=643.21) | 1-66 | m/z= 643.80 (C45H29N3S=643.21) |
| 1-67 | m/z= 643.80 (C45H29N3S=643.21) | 1-68 | m/z= 643.80 (C45H29N3S=643.21) |
| 1-69 | m/z= 733.88 (C51H31N3OS=733.22) | 1-70 | m/z= 733.88 (C51H31N3OS=733.22) |
| 1-71 | m/z= 733.88 (C51H31N3OS=733.22) | 1-72 | m/z= 567.70 (C39H25N3S=567.18) |
| 1-73 | m/z= 643.80 (C45H29N3S=643.21) | 1-74 | m/z= 749.94 (C51H31N3S2=749.20) |
| 1-75 | m/z= 749.94 (C51H31N3S2=749.20) | 1-76 | m/z= 749.94 (C51H31N3S2=749.20) |
| 1-77 | m/z= 567.70 (C39H25N3S=567.18) | 1-78 | m/z= 733.88 (C51H31N3OS=733.22) |
| 1-79 | m/z= 733.88 (C51H31N3OS=733.22) | 1-80 | m/z= 749.94 (C51H31N3S2=749.20) |
| 1-81 | m/z= 627.73 (C45H29N3O=627.23) | 1-82 | m/z= 627.73 (C45H29N3O=627.23) |
| 1-83 | m/z= 703.83 (C51H33N3O= 703.26) | 1-84 | m/z= 627.73 (C45H29N3O=627.23) |
| 1-85 | m/z= 627.73 (C45H29N3O=627.23) | 1-86 | m/z= 779.92 (C57H374N3O=779.29) |
| 1-87 | m/z= 701.81 (C51H31N3O=701.25) | 1-88 | m/z= 627.73 (C45H29N3O=627.23) |
| 1-89 | m/z= 703.83 (C51H33N3O= 703.26) | 1-90 | m/z= 703.83 (C51H33N3O= 703.26) |
| 1-91 | m/z= 641.71 (C45H27N4O2=641.21) | 1-92 | m/z= 657.78 (C45H27N3OS=657.19) |
| 1-93 | m/z= 641.71 (C45H27N4O2=641.21) | 1-94 | m/z= 627.73 (C45H29N3O=627.23) |
| 1-95 | m/z= 703.83 (C51H33N3O= 703.26) | 1-96 | m/z= 627.73 (C45H29N3O=627.23) |
| 1-97 | m/z= 703.83 (C51H33N3O= 703.26) | 1-98 | m/z= 701.81 (C51H31N3O=701.25) |
| 1-99 | m/z= 703.83 (C51H33N3O= 703.26) | 1-100 | m/z= 703.83 (C51H33N3O= 703.26) |
| 1-101 | m/z= 703.83 (C51H33N3O= 703.26) | 1-102 | m/z= 641.71 (C45H27N4O2=641.21) |
| 1-103 | m/z= 641.71 (C45H27N4O2=641.21) | 1-104 | m/z= 657.78 (C45H27N3OS=657.19) |
| 1-105 | m/z= 627.73 (C45H29N3O=627.23) | 1-106 | m/z= 627.73 (C45H29N3O=627.23) |
| 1-107 | m/z= 703.83 (C51H33N3O= 703.26) | 1-108 | m/z= 627.73 (C45H29N3O=627.23) |
| 1-109 | m/z= 627.73 (C45H29N3O=627.23) | 1-110 | m/z= 779.92 (C57H374N3O=779.29) |
| 1-111 | m/z= 701.81 (C51H31N3O=701.25) | 1-112 | m/z= 627.73 (C45H29N3O=627.23) |
| 1-113 | m/z= 703.83 (C51H33N3O= 703.26) | 1-114 | m/z= 703.83 (C51H33N3O= 703.26) |
| 1-115 | m/z= 641.71 (C45H27N4O2=641.21) | 1-116 | m/z= 657.78 (C45H27N3OS=657.19) |
| 1-117 | m/z= 641.71 (C45H27N4O2=641.21) | 1-118 | m/z= 627.73 (C45H29N3O=627.23) |
| 1-119 | m/z= 703.83 (C51H33N3O= 703.26) | 1-120 | m/z= 627.73 (C45H29N3O=627.23) |
| 1-121 | m/z= 703.83 (C51H33N3O= 703.26) | 1-122 | m/z= 701.81 (C51H31N3O=701.25) |
| 1-123 | m/z= 703.83 (C51H33N3O= 703.26) | 1-124 | m/z= 703.83 (C51H33N3O= 703.26) |
| 1-125 | m/z= 703.83 (C51H33N3O= 703.26) | 1-126 | m/z= 641.71 (C45H27N4O2=641.21) |
| 1-127 | m/z= 641.71 (C45H27N4O2=641.21) | 1-128 | m/z= 657.78 (C45H27N3OS=657.19) |
| 1-129 | m/z= 627.73 (C45H29N3O=627.23) | 1-130 | m/z= 627.73 (C45H29N3O=627.23) |
| 1-131 | m/z= 703.83 (C51H33N3O= 703.26) | 1-132 | m/z= 627.73 (C45H29N3O=627.23) |
| 1-133 | m/z= 627.73 (C45H29N3O=627.23) | 1-134 | m/z= 779.92 (C57H374N3O=779.29) |
| 1-135 | m/z= 701.81 (C51H31N3O=701.25) | 1-136 | m/z= 627.73 (C45H29N3O=627.23) |
| 1-137 | m/z= 703.83 (C51H33N3O= 703.26) | 1-138 | m/z= 703.83 (C51H33N3O= 703.26) |
| 1-139 | m/z= 641.71 (C45H27N4O2=641.21) | 1-140 | m/z= 657.78 (C45H27N3OS=657.19) |
| 1-141 | m/z= 641.71 (C45H27N4O2=641.21) | 1-142 | m/z= 627.73 (C45H29N3O=627.23) |
| 1-143 | m/z= 703.83 (C51H33N3O= 703.26) | 1-144 | m/z= 627.73 (C45H29N3O=627.23) |
| 1-145 | m/z= 703.83 (C51H33N3O= 703.26) | 1-146 | m/z= 701.81 (C51H31N3O=701.25) |
| 1-147 | m/z= 703.83 (C51H33N3O= 703.26) | 1-148 | m/z= 703.83 (C51H33N3O= 703.26) |
| 1-149 | m/z= 703.83 (C51H33N3O= 703.26) | 1-150 | m/z= 641.71 (C45H27N4O2=641.21) |
| 1-151 | m/z= 641.71 (C45H27N4O2=641.21) | 1-152 | m/z= 657.78 (C45H27N3OS=657.19) |
| 1-153 | m/z= 643.80 (C45H29N3S=643.21) | 1-154 | m/z= 643.80 (C45H29N3S=643.21) |
| 1-155 | m/z= 719.89 (C51H33N3S= 719.24) | 1-156 | m/z= 643.80 (C45H29N3S=643.21) |
| 1-157 | m/z= 643.80 (C45H29N3S=643.21) | 1-158 | m/z= 795.99 (C57H37N3S=795.27) |
| 1-159 | m/z= 717.88 (C51H31N3S=717.22) | 1-160 | m/z= 643.80 (C45H29N3S=643.21) |
| 1-161 | m/z= 719.89 (C51H33N3S= 719.24) | 1-162 | m/z= 719.89 (C51H33N3S= 719.24) |
| 1-163 | m/z= 657.78 (C45H27N3OS=657.19) | 1-164 | m/z= 678.85 (C45H27N3S2=673.16) |
| 1-165 | m/z= 657.78 (C45H27N3OS=657.19) | 1-166 | m/z= 643.80 (C45H29N3S=643.21) |
| 1-167 | m/z= 719.89 (C51H33N3S= 719.24) | 1-168 | m/z= 643.80 (C45H29N3S=643.21) |
| 1-169 | m/z= 719.89 (C51H33N3S= 719.24) | 1-170 | m/z= 717.88 (C51H31N3S=717.22) |
| 1-171 | m/z= 719.89 (C51H33N3S= 719.24) | 1-172 | m/z= 719.89 (C51H33N3S= 719.24) |
| 1-173 | m/z= 703.83 (C51H33N3O=703.26) | 1-174 | m/z= 641.71 (C45H27N3O2=641.21) |
| 1-175 | m/z= 641.71 (C45H27N3O2=641.21) | 1-176 | m/z= 657.78 (C45H27N3OS=657.19) |
| 2-1 | m/z= 484.19 (O₃₆H₂₄N₂=484.60) | 2-2 | m/z= 560.23 (C₄₂H₂₈N₂=560.70) |
| 2-3 | m/z= 560.23 (C₄₂H₂₈N₂=560.70) | 2-4 | m/z= 560.23 (C₄₂H₂₈N₂=560.70) |
| 2-5 | m/z= 636.26 (C₄₈H₃₂N₂=636.80) | 2-6 | m/z= 636.26 (C₄₈H₃₂N₂=636.80) |
| 2-7 | m/z= 636.26 (C₄₈H₃₂N₂=636.80) | 2-8 | m/z= 585.69 (C43H20N3=585.22) |
| 2-9 | m/z= 661.79(C49H31N3= 661.25) | 2-10 | m/z= 600.26 (C₄₅H₃₂N₂=600.76) |
| 2-11 | m/z= 600.26 (C₄₅H₃₂N₂=600.76) | 2-12 | m/z= 724.29 (C₅₅H₃₆N₂=724.91) |
| 2-13 | m/z= 724.29 (C₅₅H₃₆N₂=724.91) | 2-14 | m/z= 722.27 (C₅₅H₃₄N₂=722.89) |
| 2-15 | m/z= 722.27 (C₅₅H₃₄N₂=722.89) | 2-16 | m/z= 650.76 (C48H30N2O=650.24) |
| 2-17 | m/z= 509.19 (C₃₇H₂₃N₃=509.61) | 2-18 | m/z= 742.28 (C₅₄H₃₈N₂Si=743.00) |
| 2-19 | m/z= 636.26 (C₄₈H₃₂N₂=636.80) | 2-20 | m/z= 636.26 (C₄₈H₃₂N₂=636.80) |
| 2-21 | m/z= 636.26 (C₄₈H₃₂N₂=636.80) | 2-22 | m/z= 712.29 (C₅₄H₃₆N₂=712.90) |
| 2-23 | m/z= 712.29 (C₅₄H₃₆N₂=712.90) | 2-24 | m/z= 712.29 (C₅₄H₃₆N₂=712.90) |
| 2-25 | m/z= 661.79 (C49H31N3=661.25) | 2-26 | m/z= 726.86(C54H34N2O=726.27) |
| 2-27 | m/z= 676.29 (C₅₁H₃₆N₂=676.86) | 2-28 | m/z= 710.27 (C₅₄H₃₄N₂=710.88) |
| 2-29 | m/z= 585.22 (C₄₃H₂₇N₃=585.71) | 2-30 | m/z= 818.31 (C₆₀H₄₂N₂Si=819.10) |
| 2-31 | m/z= 636.26 (C₄₈H₃₂N₂=636.80) | 2-32 | m/z= 636.26 (C₄₈H₃₂N₂=636.80) |
| 2-33 | m/z= 712.29 (C₅₄H₃₆N₂=712.90) | 2-34 | m/z= 712.29 (C₅₄H₃₆N₂=712.90) |
| 2-35 | m/z= 712.29 (C₅₄H₃₆N₂=712.90) | 2-36 | m/z= 650.76 (C48H30N2O=650.24) |
| 2-37 | m/z= 650.76 (C48H30N4O2=650.24) | 2-38 | m/z= 676.29 (C₅₁H₃₆N₂=676.86) |
| 2-39 | m/z= 650.76 (C48H30N2O= 650.24) | 2-40 | m/z= 585.22 (C₄₃H₂₇N₃=585.71) |
| 2-41 | m/z= 818.31 (C₆₀H₄₂N₂Si=819.10) | 2-42 | m/z= 636.26 (C₄₈H₃₂N₂=636.80) |
| 2-43 | m/z= 712.29 (C₅₄H₃₆N₂=712.90) | 2-44 | m/z= 712.29 (C₅₄H₃₆N₂=712.90) |
| 2-45 | m/z= 712.29 (C₅₄H₃₆N₂=712.90) | 2-46 | m/z= 726.86 (C54H3452O=726.27) |
| 2-47 | m/z= 726.86 (C₅₄H₃₄N₂O=610.76) | 2-48 | m/z= 676.29 (C₅₁H₃₆N₂=676.86) |
| 2-49 | m/z= 661.79 (C₄₉H₃₁N₃=661.25) | 2-50 | m/z= 585.22 (C₄₃H₂₇N₃=585.71) |
| 2-51 | m/z= 818.31 (C₆₀H₄₂N₂Si=819.10) | 2-52 | m/z= 788.32 (C₆₀H₄₀N₂=788.99) |
| 2-53 | m/z= 788.32 (C₆₀H₄₀N₂=788.99) | 2-54 | m/z= 788.32 (C₆₀H₄₀N₂=788.99) |
| 2-55 | m/z= 895.17 (C₆₆H₄₆N₂Si=894.34) | 2-56 | m/z= 716.91 (C₅₄H₄₀N₂=716.32) |
| 2-57 | m/z= 752.32 (C₅₇H₄₀N₂=752.96) | 2-58 | m/z= 625.76 (C₄₆H₃₁N₃=625.25) |
| 2-59 | m/z= 661.25 (C₄₉H₃₁N₃=661.81) | 2-60 | m/z= 894.34 (C₆₆H₄₆N₂Si=895.19) |
| 2-61 | m/z= 788.32 (C₆₀H₄₀N₂=788.99) | 2-62 | m/z= 788.32 (C₆₀H₄₀N₂=788.99) |
| 2-63 | m/z= 859.14 (C₆₃H₄₆N₂Si=858.34) | 2-64 | m/z= 534.61 (C₃₈H₂₂N₄=534.18) |
| 2-65 | m/z= 752.32 (C₅₇H₄₀N₂=752.96) | 2-66 | m/z= 767.99 (C₅₅H₃₇N₃Si=767.28) |
| 2-67 | m/z= 661.25 (C₄₉H₃₁N₃=661.81) | 2-68 | m/z= 894.34 (C₆₆H₄₆N₂Si=895.19) |
| 2-69 | m/z= 788.32 (C₆₀H₄₀N₂=788.99) | 2-70 | m/z= 1001.37 (C₇₂H₅₂N₂Si₂=1000.37) |
| 2-71 | m/z= 712.88 (C₅₄H₃₆N₂=712.29) | 2-72 | m/z= 752.32 (C₅₇H₄₀N₂=752.96) |

**[Table 7]**

| Compound | ¹H NMR(CDCl₃, 300Mz) |
|---|---|
| 1-1 | δ = 8.22(d, 2H), 7.89-7.81(m, 7H), 7.66(d, 1H), 7.52-7.25(m, 15H) |
| 1-2 | δ = 8.28-8.24(m, 3H), 7.89-7.81(m, 5H), 7.70-7.66(m, 2H), 7.57-7.32(m, 15H) |
| 1-3 | δ =8.28(m, 2H), 7.85-7.81(m, 7H), 7.72-7.71(m, 2H), 7.52-7.38(m, 14H), 7.25(m, 4H) |
| 1-8 | δ =8.24-8.28(m, 3H), 7.85-7.70(m, 8H), 7.57-7.38(m,16H) |
| 1-10 | δ =8.24(d, 1H), 7.95-7.32(m, 30H) |
| 1-12 | δ = 8.28-8.24(m, 3H), 7.89-7.81(m, 5H), 7.70-7.66(m, 2H), 7.51-7.25(m, 15H) |
| 1-17 | δ =8.28-8.24(m, 4H), 7.89-7.81(m, 3H), 7.70-7.66(m, 3H), 7.57-7.32(m, 15H) |
| 1-23 | δ =8.28(m, 2H), 7.95(d, 1H), 7.85-7.71(m, 8H), 7.64(s, 1H), 7.52-7.41(m, 13H), 7.25(m, 4H) |
| 1-24 | δ =8.28-8.24(m, 3H), 7.95(m, 1H), 7.85-7.70(m, 7H), 7.64(s, 1H), 7.57-7.41(m, 15H), 7.25(d, 2H) |
| 1-26 | δ =8.28(d, 2H), 7.89-7.79(m, 7H), 7.60-7.25(m, 20H) |
| 1-29 | δ =7.95-7.64(m, 15H), 7.52-7.25(m, 16H) |
| 1-33 | δ =8.28-8.24(m, 3H), 7.89-7.79(m, 5H), 7.70-7.25(m, 21H) |
| 1-39 | δ = 8.24(d, 1H), 7.95-7.25(m, 31H) |
| 1-41 | δ =8.45-8.41(m, 2H), 8.28(m, 2H), 8.20(m, 1H), 7.98(m, 1H), 7.85(m, 4H), 7.58-7.41(m, 11H), 7.25(m, 4H) |
| 1-43 | δ = 8.41(d,1H), 8.28(m, 2H), 8.20(d, 1H), 8.00(d, 2H), 7.86-7.85(m, 5H), 7.58-7.41(m, 14H), 7.25(m, 4H) |
| 1-44 | δ =8.41(d, 1H), 8.28-8.20(m, 4H), 8.00(d, 2H), 7.8-7.85(m, 3H), 7.58-7.41(m, 16H), 7.25(m, 2H) |
| 1-68 | δ =8.28-8.24(m, 3H), 8.11-8.05(m, 3H), 7.94(d, 1H), 7.85-7.79(m, 5H), 7.70(s, 1H), 7.57-7.41(m, 14H), 7.25(d, 2H) |
| 1-72 | δ =8.45(d, 1H), 8.28-8.24(m, 3H), 8.11-7.98(m, 4H), 7.85(m, 2H), 7.70(s, 1H), 7.57-7.48(m, 12H), 7.25(m, 2H) |
| 1-81 | δ = 7.89-7.81(m, 9H), 7.66(d, 1H), 7.52-7.25(m, 19H) |
| 1-87 | δ = 9.15(s, 1H), 8.93(d, 2H), 8.18-8.12(m, 3H), 8.04(d, 1H), 7.89-7.81(m, 11H), 7.66(d, 1H), 7.51-7.25(m, 12H) |
| 1-92 | δ = 8.45(d, 1H), 8.11-8.08(m, 2H), 7.98(d, 1H), 7.89-7.81(m, 8H), 7.66(d, 1H), 7.52-7.25(m, 14H) |
| 1-98 | δ = 9.15(s, 1H), 8.93(d, 2H), 8.24-8.04(m, 5H), 7.89-7.81(m, 9H), 7.70-7.66(m, 2H), 7.57-7.25(m, 12H) |
| 1-102 | δ = 8.24(d, 2H), 7.95-7.89(m, 4H), 7.70-7.64(m, 10H), 7.52-7.32(m, 11H) |
| 1-153 | δ = 8.45-8.41(m, 2H), 8.20(m, 1H), 7.98(m, 1H), 7.85(m, 6H), 7.58-7.41(m, 13H), 7.25(m, 6H) |
| 1-166 | δ = 8.24(m, 2H), 7.89-7.85(m, 3H), 7.57-7.25(m, 24H) |
| 1-173 | δ = 8.24(m, 2H), 7.89-7.85(m, 3H), 7.70-7.25(m, 28H) |
| 2-3 | δ = 8.55(1H, d), 8.30(1H, d), 8.21-8.13(3H, m), 7.99-7.89(4H, m), 7.77-7.35(17H, m), 7.20-7.16(2H, m) |
| 2-4 | δ = 8.55(1H, d), 8.30(1H, d), 8.19-8.13(2H, m), 7.99-7.89(8H, m), 7.77-7.75(3H, m), 7.62-7.35(11H, m), 7.20-7.16(2H, m) |
| 2-7 | δ = 8.55(1H, d), 8.31-8.30(3H, d), 8.19-8.13(2H, m), 7.99-7.89(5H, m), 7.77-7.75(5H, m), 7.62-7.35(14H, m), 7.20-7.16(2H, m) |
| 2-16 | δ = 8.93-8.90 (3H, d), 8.55 (1H, d), 8.18-8.10 (5H, m), 8.00-7.77 (10H, m), 7.58-7.45 (8H, m), 7.33-7.29 (2H, m) |
| 2-31 | δ = 8.55(1H, d), 8.30(1H, d), 8.21-8.13(4H, m), 7.99-7.89(4H, m), 7.77-7.35(20H, m), 7.20-7.16(2H, m) |
| 2-32 | δ = 8.55(1H, d), 8.30(1H, d), 8.21-8.13(3H, m), 7.99-7.89(8H, m), 7.77-7.35(17H, m), 7.20-7.16(2H, m) |
| 2-34 | δ = 8.55 (1H, d), 8.18-8.09 (3H, m), 8.00-7.94 (2H, m), 7.87 (1H, d), 7.79-7.77 (4H, m), 7.69-7.63 (4H, m), 7.51-7.25 (21H, m) |
| 2-42 | δ = 8.55 (1H, d), 8.18-8.12 (2H, m), 8.00-7.87 (3H, m), 7.79-7,77(6H, m), 7.69-7.63 (6H, m), 7.52-7.25 (14H, m) |

### <Experimental Example 1> - Fabrication of Organic Light Emitting Devices

### 1) Fabrication of Organic Light Emitting Devices

Glass substrates coated with a thin film of ITO to a thickness of 1,500 Å were washed with distilled water and ultrasonic waves. After washing the substrates with distilled water, ultrasonic cleaning was performed on the washed substrates with solvents such as acetone, methanol, isopropyl alcohol, etc., the ultrasonic cleaned substrates were dried, and UVO-treated for 5 minutes using UV in a UV cleaner. Thereafter, after transferring the substrates to a plasma cleaner (PT), plasma treatment was performed to remove the ITO work function and residual film in a vacuum state, and the substrates were transferred to thermal deposition equipment for organic deposition.

As common layers on the ITO transparent electrodes (anodes), a hole injection layer 2-TNATA (4,4',4"-Tris[2-naphthyl(phenyl)amino]triphenylamine) and a hole transport layer NPB (N,N'-Di(1-naphthyl)-N,N'-diphenyl-(1,1'-biphenyl)-4,4'-diamine) were formed.

A light emitting layer was thermal vacuum deposited thereon as follows. The light emitting layer was deposited to a thickness of 400 Å by doping the host with 7% of Ir(ppy)₃ using the compounds shown in Table 8 below as a host and Ir(ppy)₃ (tris(2-phenylpyridine)iridium) as a green phosphorescent dopant. Thereafter, BCP was deposited to a thickness of 60 Å as a hole blocking layer, and Alq₃ was deposited to a thickness of 200 Å thereon as an electron transport layer. Finally, after forming an electron injection layer by depositing lithium fluoride (LiF) to a thickness of 10 Å on the electron transport layer, an aluminum (Al) cathode was deposited to a thickness of 1,200 Å on the electron injection layer to form cathodes, thereby manufacturing organic electroluminescent devices.

Meanwhile, all organic compounds required for fabricating OLED devices were vacuum sublimated and purified under 10⁻⁶ to 10⁻⁸ torr for each material respectively, and used for OLED fabrication.

### 2) Driving Voltage and Luminous Efficiency of Organic Electroluminescent Devices

Electroluminescence (EL) characteristics were measured for the organic electroluminescent devices fabricated as described above with M7000 of McScience, and T₉₀ values were measured with the measurement results when the reference luminance was 6,000 cd/m² through the device lifespan measuring system (M6000) manufactured by McScience. The properties of the organic electroluminescent devices of the present disclosure were as shown in Table 8.

**[Table 8]**

| | Compound | Driving voltage (V) | Yield (cd/A) | Color coordinate (x, y) | Lifespan (T₉₀) |
|---|---|---|---|---|---|
| Comparative Example 1 | 1-1 | 4.28 | 65.3 | (0.277, 0.669) | 138 |
| Comparative Example 2 | 1-2 | 4.26 | 66.7 | (0.281, 0.679) | 139 |
| Comparative Example 3 | 1-3 | 4.12 | 63.2 | (0.280, 0.677) | 137 |
| Comparative Example 4 | 1-4 | 4.20 | 67.8 | (0.279, 0.676) | 138 |
| Comparative Example 5 | 1-5 | 4.22 | 68.9 | (0.272, 0.669) | 139 |
| Comparative Example 6 | 1-6 | 4.28 | 69.0 | (0.271, 0.671) | 140 |
| Comparative Example 7 | 1-7 | 4.29 | 66.7 | (0.275, 0.672) | 141 |
| Comparative Example 8 | 1-8 | 4.21 | 67.8 | (0.279, 0.675) | 141 |
| Comparative Example 9 | 1-9 | 4.18 | 69.1 | (0.280, 0.676) | 138 |
| Comparative Example 10 | 1-10 | 4.17 | 70.1 | (0.278, 0.672) | 139 |
| Comparative Example 11 | 1-11 | 4.15 | 63.2 | (0.283, 0.687) | 140 |
| Comparative Example 12 | 1-12 | 4.23 | 64.5 | (0.286, 0.686) | 141 |
| Comparative Example 13 | 1-13 | 4.17 | 65.5 | (0.274, 0.678) | 140 |
| Comparative Example 14 | 1-15 | 4.15 | 66.7 | (0.279, 0.677) | 141 |
| Comparative Example 15 | 1-17 | 4.10 | 67.8 | (0.278, 0.677) | 141 |
| Comparative Example 16 | 1-19 | 4.18 | 69.2 | (0.276, 0.671) | 139 |
| Comparative Example 17 | 1-21 | 4.18 | 69.9 | (0.284, 0.687) | 138 |
| Comparative Example 18 | 1-22 | 4.19 | 68.9 | (0.281, 0.684) | 139 |
| Comparative Example 19 | 1-23 | 4.20 | 68.8 | (0.283, 0.681) | 138 |
| Comparative Example 20 | 1-24 | 4.21 | 69.2 | (0.279, 0.681) | 137 |
| Comparative Example 21 | 1-25 | 4.08 | 77.3 | (0.280, 0.679) | 142 |
| Comparative Example 22 | 1-26 | 4.07 | 78.8 | (0.281, 0.678) | 146 |
| Comparative Example 23 | 1-27 | 4.00 | 78.9 | (0.278, 0.682) | 148 |
| Comparative Example 24 | 1-28 | 4.06 | 72.3 | (0.286, 0.692) | 148 |
| Comparative Example 25 | 1-29 | 3.98 | 77.4 | (0.274, 0.672) | 149 |
| Comparative Example 26 | 1-30 | 3.96 | 78.3 | (0.278, 0.678) | 150 |
| Comparative Example 27 | 1-31 | 3.99 | 79.2 | (0.277, 0.674) | 147 |
| Comparative Example 28 | 1-32 | 3.92 | 80.4 | (0.279, 0.674) | 149 |
| Comparative Example 29 | 1-33 | 4.08 | 80.3 | (0.278, 0.677) | 151 |
| Comparative Example 30 | 1-34 | 4.09 | 81.2 | (0.283, 0.677) | 150 |
| Comparative Example 31 | 1-35 | 4.11 | 81.4 | (0.273, 0.678) | 151 |
| Comparative Example 32 | 1-36 | 4.07 | 80.7 | (0.277, 0.668) | 152 |
| Comparative Example 33 | 1-37 | 3.88 | 83.1 | (0.271, 0.673) | 157 |
| Comparative Example 34 | 1-38 | 3.89 | 84.7 | (0.276, 0.671) | 158 |
| Comparative Example 35 | 1-39 | 3.87 | 83.9 | (0.272, 0.666) | 159 |
| Comparative Example 36 | 1-40 | 3.85 | 82.9 | (0.274, 0.667) | 160 |
| Comparative Example 37 | 1-41 | 4.26 | 68.9 | (0.279, 0.676) | 137 |
| Comparative Example 38 | 1-48 | 4.28 | 69.7 | (0.272, 0.669) | 138 |
| Comparative Example 39 | 1-52 | 4.18 | 67.7 | (0.271, 0.671) | 139 |
| Comparative Example 40 | 1-58 | 4.16 | 69.9 | (0.275, 0.672) | 141 |
| Comparative Example 41 | 1-61 | 3.92 | 78.9 | (0.279, 0.675) | 150 |
| Comparative Example 42 | 1-63 | 3.90 | 79.2 | (0.280, 0.676) | 149 |
| Comparative Example 43 | 1-77 | 3.82 | 85.0 | (0.277, 0.669) | 149 |
| Comparative Example 44 | 1-78 | 3.83 | 84.7 | (0.281, 0.679) | 151 |
| Comparative Example 45 | 1-81 | 4.16 | 68.7 | (0.280, 0.677) | 138 |
| Comparative Example 46 | 1-82 | 4.18 | 69.2 | (0.279, 0.676) | 138 |
| Comparative Example 47 | 1-83 | 4.13 | 69.3 | (0.272, 0.669) | 137 |
| Comparative Example 48 | 1-84 | 4.21 | 67.2 | (0.271, 0.671) | 132 |
| Comparative Example 49 | 1-85 | 4.22 | 70.3 | (0.275, 0.672) | 137 |
| Comparative Example 50 | 1-88 | 4.18 | 69.7 | (0.279, 0.675) | 135 |
| Comparative Example 51 | 1-89 | 4.17 | 71.2 | (0.280, 0.676) | 136 |
| Comparative Example 52 | 1-90 | 4.18 | 70.3 | (0.278, 0.672) | 134 |
| Comparative Example 53 | 1-92 | 4.15 | 69.4 | (0.283, 0.687) | 138 |
| Comparative Example 54 | 1-93 | 4.11 | 69.9 | (0.286, 0.686) | 139 |
| Comparative Example 55 | 1-97 | 4.24 | 71.9 | (0.274, 0.678) | 137 |
| Comparative Example 56 | 1-99 | 4.22 | 70.3 | (0.279, 0.677) | 136 |
| Comparative Example 57 | 1-101 | 3.99 | 78.9 | (0.278, 0.677) | 150 |
| Comparative Example 58 | 1-102 | 3.97 | 77.3 | (0.276, 0.671) | 149 |
| Comparative Example 59 | 1-103 | 3.99 | 78.8 | (0.273, 0.678) | 148 |
| Comparative Example 60 | 1-104 | 3.98 | 79.2 | (0.277, 0.668) | 148 |
| Comparative Example 61 | 1-105 | 3.92 | 73.7 | (0.271, 0.673) | 147 |
| Comparative Example 62 | 1-106 | 3.90 | 75.9 | (0.276, 0.671) | 149 |
| Comparative Example 63 | 1-108 | 3.91 | 76.6 | (0.272, 0.666) | 148 |
| Comparative Example 64 | 1-113 | 3.89 | 76.7 | (0.274, 0.667) | 151 |
| Comparative Example 65 | 1-114 | 3.90 | 77.7 | (0.279, 0.676) | 150 |
| Comparative Example 66 | 1-115 | 3.88 | 79.2 | (0.272, 0.669) | 151 |
| Comparative Example 67 | 1-177 | 3.86 | 81.2 | (0.271, 0.671) | 158 |
| Comparative Example 68 | 1-118 | 3.85 | 83.4 | (0.275, 0.672) | 159 |
| Comparative Example 69 | 1-119 | 3.87 | 82.9 | (0.280, 0.676) | 159 |
| Comparative Example 70 | 1-120 | 3.88 | 84.0 | (0.278, 0.672) | 161 |
| Comparative Example 71 | 1-125 | 4.12 | 70.2 | (0.283, 0.687) | 134 |
| Comparative Example 72 | 1-128 | 4.18 | 69.3 | (0.286, 0.686) | 137 |
| Comparative Example 73 | 1-130 | 4.17 | 68.8 | (0.274, 0.678) | 139 |
| Comparative Example 74 | 1-141 | 3.97 | 77.3 | (0.279, 0.677) | 149 |
| Comparative Example 75 | 1-143 | 3.92 | 78.8 | (0.278, 0.677) | 148 |
| Comparative Example 76 | 1-145 | 3.94 | 79.2 | (0.276, 0.671) | 144 |
| Comparative Example 77 | 1-146 | 3.90 | 77.3 | (0.273, 0.678) | 149 |
| Comparative Example 78 | 1-157 | 3.85 | 83.1 | (0.277, 0.668) | 153 |
| Comparative Example 79 | 1-158 | 3.88 | 82.2 | (0.274, 0.678) | 155 |
| Comparative Example 80 | 1-159 | 3.87 | 81.4 | (0.279, 0.677) | 157 |
| Comparative Example 81 | 1-160 | 3.85 | 83.2 | (0.278, 0.677) | 159 |

### <Experimental Example 2> - Fabrication of Organic Light Emitting Devices

Glass substrates coated with a thin film of ITO to a thickness of 1,500 Å were washed with distilled water and ultrasonic waves. After washing the substrates with distilled water, ultrasonic cleaning was performed on the washed substrates with solvents such as acetone, methanol, isopropyl alcohol, etc., the ultrasonic cleaned substrates were dried, and UVO-treated for 5 minutes using UV in a UV cleaner. Thereafter, after transferring the substrates to a plasma cleaner (PT), plasma treatment was performed to remove the ITO work function and residual film in a vacuum state, and the substrates were transferred to thermal deposition equipment for organic deposition.

As common layers on the ITO transparent electrodes (anodes), a hole injection layer 2-TNATA (4,4',4"-tris[2-naphthyl(phenyl)amino]triphenylamine) and a hole transport layer NPB (N,N'-di(1-naphthyl)-N,N'-diphenyl-(1,1'-biphenyl)-4,4'-diamine) were formed.

A light emitting layer was thermal vacuum deposited thereon as follows. The light emitting layer was deposited to a thickness of 400 Å on one container after premixing one compound described in Chemical Formula 1 and one compound described in Chemical Formula 2 in Table 9 below as a host, and the green phosphorescent dopant was deposited by doping Ir(ppy)₃ to a thickness corresponding to 7% of the deposition thickness of the light emitting layer. Thereafter, BCP was deposited to a thickness of 60 Å as a hole blocking layer, and Alq₃ was deposited to a thickness of 200 Å thereon as an electron transport layer. Finally, after forming an electron injection layer by depositing lithium fluoride (LiF) to a thickness of 10 Å on the electron transport layer, an aluminum (Al) cathode was deposited to a thickness of 1,200 Å on the electron injection layer to form cathodes, thereby manufacturing organic electroluminescent devices.

Meanwhile, all organic compounds required for fabricating OLED devices were vacuum sublimated and purified under 10⁻⁶ to 10⁻⁸ torr for each material respectively, and used for OLED fabrication.

Electroluminescence (EL) characteristics were measured for the organic electroluminescent devices fabricated as described above with M7000 of McScience, and T₉₀ values were measured with the measurement results when the reference luminance was 6,000 cd/m² through the device lifespan measuring system (M6000) manufactured by McScience.

The results of measuring driving voltage values, luminous efficiency values, color coordinate (CIE) values, and lifespan values of the organic light emitting devices manufactured according to the present disclosure were as shown in Table 9 below.

**[Table 9]**

| | Light emitting layer compound | Ratio | Driving voltage (V) | Efficiency (cd/A) | Color coordinate (x, y) | Lifespan (T₉₀) |
|---|---|---|---|---|---|---|
| Example 1 | 1-1:2-5 | 1:3.5 | 3.81 | 125.3 | (0.279, 0.677) | 380 |
| Example 2 | | 1:3 | 3.80 | 127.6 | (0.272, 0.666) | 378 |
| Example 3 | | 1:2 | 3.79 | 130.2 | (0.274, 0.667) | 377 |
| Example 4 | 1-1:2-33 | 1:3 | 3.80 | 128.4 | (0.277, 0.677) | 381 |
| Example 5 | | 1:2 | 3.79 | 130.2 | (0.277, 0.680) | 379 |
| Example 6 | 1-1:2.24 | 1:3 | 3.72 | 140.6 | (0.278, 0.681) | 385 |
| Example 7 | | 1:2 | 3.70 | 144.4 | (0.277, 0.682) | 382 |
| Example 8 | 1-1:2-53 | 1:3 | 3.73 | 142.3 | (0.281, 0.676) | 384 |
| Example 9 | | 1:2 | 3.71 | 145.4 | (0.280, 0.676) | 380 |
| Example 10 | 1-1:2-69 | 1:3 | 3.61 | 150.6 | (0.276, 0.679) | 390 |
| Example 11 | | 1:2 | 3.60 | 154.6 | (0.275, 0.679) | 387 |
| Example 12 | 1-1:2-72 | 1:3 | 3.63 | 153.7. | (0.278, 0.678) | 389 |
| Example 13 | | 1:2 | 3.60 | 155.8 | (0.279, 0.677) | 385 |
| Example 14 | 1-1:2-73 | 1:3.5 | 3.53 | 157.2 | (0.275, 0.680) | 393 |
| Example 15 | | 1:3 | 3.51 | 158.3 | (0.274, 0.678) | 391 |
| Example 16 | | 1:2 | 3.50 | 160.6 | (0.276, 0.676) | 390 |
| Example 17 | 1-1:2-15 | 1:3 | 3.51 | 155.6 | (0.274, 0.679) | 393 |
| Example 18 | | 1:2 | 3.50 | 161.7 | (0.288, 0.681) | 390 |
| Example 19 | 1-2:2-45 | 1:3.5 | 3.74 | 140.4 | (0.278, 0.677) | 385 |
| Example 20 | | 1:2.5 | 3.71 | 143.7 | (0.276, 0.671) | 384 |
| Example 21 | | 1:2 | 3.70 | 145.1 | (0.273, 0.678) | 381 |
| Example 22 | 1-2:2-63 | 1:4 | 3.67 | 150.9 | (0.277, 0.668) | 390 |
| Example 23 | | 1:3 | 3.62 | 153.7 | (0.271, 0.673) | 389 |
| Example 24 | | 1:2.5 | 3.60 | 155.9 | (0.276, 0.671) | 387 |
| Example 25 | 1-2:2-49 | 1:3.5 | 3.56 | 157.2 | (0.272, 0.666) | 393 |
| Example 26 | | 1:2.5 | 3.51 | 159.3 | (0.274, 0.667) | 393 |
| Example 27 | | 1:2 | 3.50 | 160.2 | (0.277, 0.677) | 392 |
| Example 28 | 1-2:2-40 | 1:3.5 | 3.54 | 156.4 | (0.277, 0.680) | 393 |
| Example 29 | | 1:2.5 | 3.52 | 158.3 | (0.278, 0.681) | 392 |
| Example 30 | | 1:2 | 3.51 | 159.9 | (0.277, 0.682) | 390 |
| Example 31 | 1-3:2-65 | 1:3 | 3.63 | 153.7 | (0.276, 0.671) | 390 |
| Example 32 | | 1:2 | 3.60 | 155.9 | (0.276, 0.671) | 388 |
| Example 33 | 1-3:2-33 | 1:3 | 3.52 | 157.9 | (0.272, 0.666) | 393 |
| Example 34 | | 1:2 | 3.50 | 160.7 | (0.274, 0.667) | 392 |
| Example 35 | 1-5:2-30 | 1:3 | 3.55 | 158.9 | (0.273, 0.678) | 393 |
| Example 36 | | 1:2 | 3.50 | 159.9 | (0.277, 0.668) | 391 |
| Example 37 | 1-5:2-44 | 1:3 | 3.53 | 157.9 | (0.271, 0.673) | 391 |
| Example 38 | | 1:2 | 3.52 | 158.9 | (0.276, 0.671) | 390 |
| Example 39 | 1-8:2-68 | 1:3.5 | 3.53 | 156.4 | (0.272, 0.666) | 393 |
| Example 40 | | 1:2.5 | 3.52 | 157.9 | (0.274, 0.667) | 391 |
| Example 41 | | 1:2 | 3.50 | 159.9 | (0.277, 0.677) | 390 |
| Example 42 | 1-8:2-34 | 1:3 | 3.52 | 156.7 | (0.277, 0.680) | 391 |
| Example 43 | | 1:2 | 3.51 | 158.9 | (0.278, 0.681) | 390 |
| Example 44 | 1-10:2-66 | 1:3 | 3.63 | 153.7 | (0.277, 0.682) | 390 |
| Example 45 | | 1:2 | 3.62 | 155.2 | (0.276, 0.671) | 387 |
| Example 46 | 1-10:2-38 | 1:3 | 3.53 | 156.7 | (0.276, 0.671) | 393 |
| Example 47 | | 1:2 | 3.50 | 159.9 | (0.277, 0.668) | 391 |
| Example 48 | 1-17:2-32 | 1:3 | 3.54 | 157.8 | (0.271, 0.673) | 393 |
| Example 49 | | 1:2 | 3.52 | 159.9 | (0.276, 0.671) | 392 |
| Example 50 | 1-17:2-13 | 1:3 | 3.53 | 156.7 | (0.272, 0.666) | 393 |
| Example 51 | | 1:2 | 3.50 | 159.9 | (0.274, 0.667) | 392 |
| Example 52 | 1-18:2-23 | 1:3 | 3.51 | 157.7 | (0.277, 0.677) | 393 |
| Example 53 | | 1:2 | 3.50 | 160.4 | (0.277, 0.680) | 390 |
| Example 54 | 1-18:2-24 | 1:3 | 3.53 | 158.9 | (0.278, 0.681) | 394 |
| Example 55 | | 1:2 | 3.52 | 160.7 | (0.277, 0.682) | 392 |
| Example 56 | 1-21:2-33 | 1:3 | 3.51 | 159.3 | (0.276, 0.671) | 393 |
| Example 57 | | 1:2 | 3.50 | 161.2 | (0.276, 0.671) | 390 |
| Example 58 | 1-22:2-5 | 1:3 | 3.71 | 143.3 | (0.277, 0.677) | 443 |
| Example 59 | | 1:2 | 3.70 | 145.7 | (0.277, 0.680) | 380 |
| Example 60 | 1-22:2-25 | 1:3 | 3.72 | 144.6 | (0.276, 0.671) | 385 |
| Example 61 | | 1:2 | 3.70 | 145.9 | (0.272, 0.666) | 383 |
| Example 62 | 1-22:2-42 | 1:3 | 3.63 | 154.3 | (0.274, 0.667) | 385 |
| Example 63 | | 1:2 | 3.60 | 155.7 | (0.277, 0.677) | 381 |
| Example 64 | 1-22:2-50 | 1:3 | 3.61 | 153.3 | (0.277, 0.680) | 390 |
| Example 65 | | 1:2 | 3.60 | 155.6 | (0.278, 0.681) | 388 |
| Example 66 | 1-22:2-66 | 1:3 | 3.53 | 158.7 | (0.277, 0.682) | 393 |
| Example 67 | | 1:2 | 3.50 | 160.3 | (0.276, 0.671) | 390 |
| Example 68 | 1-22:2-72 | 1:3 | 3.52 | 157.9 | (0.276, 0.671) | 394 |
| Example 69 | | 1:2 | 3.50 | 161.0 | (0.277, 0.677) | 391 |
| Example 70 | 1-22:2-33 | 1:3 | 3.43 | 168.7 | (0.277, 0.680) | 420 |
| Example 71 | | 1:2 | 3.41 | 169.3 | (0.276, 0.671) | 418 |
| Example 72 | 1-22:2-36 | 1:3 | 3.42 | 166.4 | (0.272, 0.666) | 421 |
| Example 73 | | 1:2 | 3.40 | 170.2 | (0.274, 0.667) | 419 |
| Example 74 | 1-23:2-10 | 1:3 | 3.43 | 167.7 | (0.277, 0.677) | 393 |
| Example 75 | | 1:2 | 3.42 | 170.3 | (0.277, 0.680) | 382 |
| Example 76 | 1-23:2-68 | 1:3 | 3.44 | 168.8 | (0.277, 0.680) | 420 |
| Example 77 | | 1:2 | 3.41 | 170.2 | (0.276, 0.671) | 418 |
| Example 78 | 1-24:2-64 | 1:3 | 3.51 | 158.9 | (0.272, 0.666) | 393 |
| Example 79 | | 1:2 | 3.50 | 160.2 | (0.274, 0.667) | 391 |
| Example 80 | 1-24:2-24 | 1:3 | 3.41 | 169.2 | (0.277, 0.677) | 421 |
| Example 81 | | 1:2 | 3.40 | 171.3 | (0.277, 0.680) | 419 |
| Example 82 | 1-26:2-11 | 1:3 | 3.42 | 168.3 | (0.278, 0.681) | 422 |
| Example 83 | | 1:2 | 3.40 | 170.9 | (0.277, 0.682) | 420 |
| Example 84 | 1-26:2-33 | 1:3 | 3.40 | 168.3 | (0.276, 0.671) | 421 |
| Example 85 | | 1:2 | 3.39 | 171.2 | (0.276, 0.671) | 419 |
| Example 86 | 1-27:2-24 | 1:3 | 3.41 | 169.9 | (0.277, 0.677) | 420 |
| Example 87 | | 1:2 | 3.40 | 171.2 | (0.277, 0.680) | 419 |
| Example 88 | 1-27:2-18 | 1:3 | 3.41 | 168.2 | (0.276, 0.671) | 422 |
| Example 89 | | 1:2 | 3.39 | 170.3 | (0.272, 0.666) | 420 |
| Example 90 | 1-37:2-22 | 1:3 | 3.53 | 158.2 | (0.274, 0.667) | 393 |
| Example 91 | | 1:2 | 3.50 | 160.3 | (0.277, 0.677) | 391 |
| Example 92 | 1-37:2-33 | 1:3 | 3.52 | 159.2 | (0.278, 0.681) | 394 |
| Example 93 | | 1:2 | 3.51 | 162.1 | (0.277, 0.682) | 391 |
| Example 94 | 1-37:2-45 | 1:3 | 3.42 | 168.2 | (0.276, 0.671) | 420 |
| Example 95 | | 1:2 | 3.40 | 170.3 | (0.276, 0.671) | 418 |
| Example 96 | 1-37:2-55 | 1:3 | 3.41 | 169.2 | (0.277, 0.677) | 422 |
| Example 97 | | 1:2 | 3.40 | 172.2 | (0.277, 0.680) | 420 |
| Example 98 | 1-37:2-65 | 1:3 | 3.32 | 178.2 | (0.276, 0.671) | 480 |
| Example 99 | | 1:2 | 3.30 | 180.3 | (0.272, 0.666) | 478 |
| Example 100 | 1-37:2-72 | 1:3 | 3.31 | 179.2 | (0.274, 0.667) | 482 |
| Example 101 | | 1:2 | 3.30 | 181.1 | (0.277, 0.677) | 480 |
| Example 102 | 1-37:2-73 | 1:3 | 3.21 | 188.3 | (0.278, 0.681) | 500 |
| Example 103 | | 1:2 | 3.20 | 190.2 | (0.277, 0.682) | 492 |
| Example 104 | 1-37:2-35 | 1:3 | 3.22 | 190.3 | (0.276, 0.671) | 500 |
| Example 105 | | 1:2 | 3.20 | 192.7 | (0.276, 0.671) | 493 |
| Example 106 | 1-39:2-31 | 1:3 | 3.18 | 190.7 | (0.277, 0.677) | 501 |
| Example 107 | | 1:2 | 3.16 | 192.2 | (0.277, 0.680) | 499 |
| Example 108 | 1-39: 2-28 | 1:3 | 3.19 | 191.3 | (0.277, 0.682) | 500 |
| Example 109 | | 1:2 | 3.17 | 193.2 | (0.276, 0.671) | 498 |
| Example 110 | 1-39:2-30 | 1:3 | 3.20 | 190.7 | (0.276, 0.671) | 502 |
| Example 111 | | 1:2 | 3.18 | 192.7 | (0.277, 0.677) | 500 |
| Example 112 | 1-41:2-43 | 1:3 | 3.50 | 158.8 | (0.277, 0.680) | 393 |
| Example 113 | | 1:2 | 3.48 | 160.2 | (0.272, 0.666) | 391 |
| Example 114 | 1-41:2-28 | 1:3 | 3.51 | 156.7 | (0.277, 0.680) | 393 |
| Example 115 | | 1:2 | 3.50 | 159.3 | (0.272, 0.666) | 392 |
| Example 116 | 1-43:2-44 | 1:3 | 3.52 | 158.3 | (0.274, 0.667) | 394 |
| Example 117 | | 1:2 | 3.50 | 161.1 | (0.277, 0.677) | 393 |
| Example 118 | 1-43:2-30 | 1:3 | 3.50 | 158.8 | (0.277, 0.680) | 394 |
| Example 119 | | 1:2 | 3.48 | 160.2 | (0.272, 0.666) | 393 |
| Example 120 | 1-46:2-63 | 1:3 | 3.51 | 157.8 | (0.279, 0.677) | 392 |
| Example 121 | | 1:2 | 3.49 | 161.1 | (0.277, 0.680) | 390 |
| Example 122 | 1-46:2-43 | 1:3 | 3.50 | 159.2 | (0.272, 0.666) | 393 |
| Example 123 | | 1:2 | 3.49 | 162.0 | (0.279, 0.677) | 390 |
| Example 124 | 1-50:2-52 | 1:3 | 3.51 | 158.3 | (0.279, 0.677) | 392 |
| Example 125 | | 1:2 | 3.48 | 159.2 | (0.277, 0.680) | 390 |
| Example 126 | 1-50:2-61 | 1:3 | 3.51 | 157.3 | (0.272, 0.666) | 394 |
| Example 127 | | 1:2 | 3.50 | 160.3 | (0.279, 0.677) | 392 |
| Example 128 | 1-57:2-37 | 1:3 | 3.52 | 158.9 | (0.277, 0.668) | 392 |
| Example 129 | | 1:2 | 3.51 | 160.1 | (0.277, 0.680) | 390 |
| Example 130 | 1-57:2-20 | 1:3 | 3.51 | 156.6 | (0.272, 0.666) | 391 |
| Example 131 | | 1:2 | 3.49 | 159.9 | (0.279, 0.677) | 390 |
| Example 132 | 1-61:2-71 | 1:3 | 3.40 | 168.3 | (0.273, 0.678) | 422 |
| Example 133 | | 1:2 | 3.39 | 170.2 | (0.277, 0.680) | 420 |
| Example 134 | 1-61:2-33 | 1:3 | 3.38 | 169.8 | (0.272, 0.666) | 423 |
| Example 135 | | 1:2 | 3.37 | 171.1 | (0.279, 0.677) | 421 |
| Example 136 | 1-65:2-25 | 1:3 | 3.41 | 166.8 | (0.277, 0.680) | 420 |
| Example 137 | | 1:2 | 3.39 | 170.2 | (0.272, 0.666) | 418 |
| Example 138 | 1-65:2-64 | 1:3 | 3.40 | 169.7 | (0.279, 0.677) | 422 |
| Example 139 | | 1:2 | 3.38 | 172.5 | (0.279, 0.677) | 420 |
| Example 140 | 1-66:2-11 | 1:3 | 3.41 | 168.6 | (0.277, 0.680) | 422 |
| Example 141 | | 1:2 | 3.40 | 170.5 | (0.272, 0.666) | 421 |
| Example 142 | 1-66:2-23 | 1:3 | 3.39 | 171.4 | (0.279, 0.677) | 423 |
| Example 143 | | 1:2 | 3.38 | 172.3 | (0.277, 0.668) | 420 |
| Example 144 | 1-68:2-23 | 1:3 | 3.41 | 169.2 | (0.277, 0.680) | 422 |
| Example 145 | | 1:2 | 3.40 | 171.6 | (0.272, 0.666) | 420 |
| Example 146 | 1-68:2-33 | 1:3 | 3.41 | 168.6 | (0.279, 0.677) | 423 |
| Example 147 | | 1:2 | 3.40 | 170.3 | (0.273, 0.678) | 421 |
| Example 148 | 1-77:2-58 | 1:3 | 3.21 | 188.6 | (0.272, 0.666) | 500 |
| Example 149 | | 1:2 | 3.20 | 190.5 | (0.277, 0.680) | 495 |
| Example 150 | 1-77:2-49 | 1:3 | 3.19 | 190.6 | (0.272, 0.666) | 498 |
| Example 151 | | 1:2 | 3.18 | 192.5 | (0.279, 0.677) | 496 |
| Example 152 | 1-78:2-20 | 1:3 | 3.20 | 189.5 | (0.277, 0.680) | 499 |
| Example 153 | | 1:2 | 3.18 | 191.7 | (0.272, 0.666) | 492 |
| Example 154 | 1-78:2-33 | 1:3 | 3.21 | 187.7 | (0.279, 0.677) | 498 |
| Example 155 | | 1:2 | 3.20 | 189.9 | (0.279, 0.677) | 497 |
| Example 156 | 1-79:2-10 | 1:3 | 3.18 | 188.7 | (0.277, 0.680) | 499 |
| Example 157 | | 1:2 | 3.16 | 191.9 | (0.272, 0.666) | 490 |
| Example 158 | 1-79:2-28 | 1:3 | 3.21 | 187.8 | (0.279, 0.677) | 493 |
| Example 159 | | 1:2 | 3.20 | 190.8 | (0.277, 0.668) | 490 |
| Example 160 | 1-80:2-32 | 1:3 | 3.21 | 188.9 | (0.277, 0.680) | 501 |
| Example 161 | | 1:2 | 3.20 | 190.7 | (0.272, 0.666) | 498 |
| Example 162 | 1-80:2-53 | 1:3 | 3.19 | 191.5 | (0.279, 0.677) | 499 |
| Example 163 | | 1:2 | 3.18 | 192.3 | (0.273, 0.678) | 492 |
| Example 164 | 1-81:2-43 | 1:3 | 3.50 | 158.8 | (0.276, 0.676) | 393 |
| Example 165 | | 1:2 | 3.48 | 160.2 | (0.277, 0.668) | 391 |
| Example 166 | 1-81:2-38 | 1:3 | 3.51 | 156.7 | (0.277, 0.680) | 393 |
| Example 167 | | 1:2 | 3.49 | 159.3 | (0.272, 0.666) | 392 |
| Example 168 | 1-83:2-22 | 1:3 | 3.50 | 158.3 | (0.279, 0.677) | 394 |
| Example 169 | | 1:2 | 3.49 | 161.1 | (0.273, 0.678) | 393 |
| Example 170 | 1-83:2-47 | 1:3 | 3.51 | 158.8 | (0.276, 0.676) | 394 |
| Example 171 | | 1:2 | 3.48 | 160.2 | (0.277, 0.668) | 393 |
| Example 172 | 1-84:2-32 | 1:3 | 3.51 | 157.8 | (0.277, 0.680) | 392 |
| Example 173 | | 1:2 | 3.50 | 161.1 | (0.272, 0.666) | 390 |
| Example 174 | 1-84:2-29 | 1:3 | 3.52 | 159.2 | (0.279, 0.677) | 393 |
| Example 175 | | 1:2 | 3.51 | 162.0 | (0.273, 0.678) | 390 |
| Example 176 | 1-90:2-43 | 1:3 | 3.51 | 158.3 | (0.276, 0.676) | 392 |
| Example 177 | | 1:2 | 3.49 | 159.2 | (0.277, 0.668) | 390 |
| Example 178 | 1-90:2-38 | 1:3 | 3.50 | 157.3 | (0.277, 0.680) | 394 |
| Example 179 | | 1:2 | 3.48 | 160.3 | (0.272, 0.666) | 392 |
| Example 180 | 1-101:2-49 | 1:3 | 3.40 | 168.3 | (0.279, 0.677) | 422 |
| Example 181 | | 1:2 | 3.39 | 170.2 | (0.273, 0.678) | 420 |
| Example 182 | 1-101:2-27 | 1:3 | 3.38 | 169.8 | (0.276, 0.676) | 423 |
| Example 183 | | 1:2 | 3.37 | 171.1 | (0.277, 0.668) | 421 |
| Example 184 | 1-101:2-17 | 1:3 | 3.41 | 166.8 | (0.277, 0.680) | 420 |
| Example 185 | | 1:2 | 3.39 | 170.2 | (0.272, 0.666) | 418 |
| Example 186 | 1-105:2-24 | 1:3 | 3.40 | 169.7 | (0.279, 0.677) | 422 |
| Example 187 | | 1:2 | 3.38 | 172.5 | (0.273, 0.678) | 420 |
| Example 188 | 1-105:2-48 | 1:3 | 3.41 | 168.6 | (0.276, 0.676) | 422 |
| Example 189 | | 1:2 | 3.40 | 170.5 | (0.273, 0.678) | 421 |
| Example 190 | 1-105:2-66 | 1:3 | 3.39 | 171.4 | (0.276, 0.676) | 423 |
| Example 191 | | 1:2 | 3.38 | 172.3 | (0.277, 0.668) | 420 |
| Example 192 | 1-105:2-58 | 1:3 | 3.41 | 169.2 | (0.277, 0.680) | 422 |
| Example 193 | | 1:2 | 3.40 | 171.6 | (0.272, 0.666) | 420 |
| Example 194 | 1-106:2-22 | 1:3 | 3.41 | 168.6 | (0.279, 0.677) | 423 |
| Example 195 | | 1:2 | 3.40 | 170.3 | (0.273, 0.678) | 421 |
| Example 196 | 1-106:2-37 | 1:3 | 3.40 | 168.3 | (0.273, 0.678) | 420 |
| Example 197 | | 1:2 | 3.39 | 170.2 | (0.276, 0.676) | 418 |
| Example 198 | 1-107:2-22 | 1:3 | 3.41 | 169.9 | (0.277, 0.668) | 393 |
| Example 199 | | 1:2 | 3.40 | 171.2 | (0.277, 0.680) | 391 |
| Example 200 | 1-108:2-24 | 1:3 | 3.41 | 168.2 | (0.272, 0.666) | 421 |
| Example 201 | | 1:2 | 3.39 | 170.3 | (0.279, 0.677) | 419 |
| Example 202 | 1-118:2-34 | 1:3 | 3.18 | 188.7 | (0.277, 0.680) | 499 |
| Example 202 | | 1:2 | 3.16 | 191.9 | (0.272, 0.666) | 490 |
| Example 203 | 1-119:2-38 | 1:3 | 3.21 | 187.8 | (0.279, 0.677) | 493 |
| Example 204 | | 1:2 | 3.20 | 190.8 | (0.277, 0.668) | 490 |
| Example 205 | 1-141:2-45 | 1:3 | 3.40 | 169.7 | (0.279, 0.677) | 422 |
| Example 206 | | 1:2 | 3.38 | 172.5 | (0.279, 0.677) | 420 |
| Example 207 | 1-145:2-66 | 1:3 | 3.41 | 168.6 | (0.277, 0.680) | 422 |
| Example 208 | | 1:2 | 3.40 | 170.5 | (0.272, 0.666) | 421 |
| Example 209 | 1-158:2-62 | 1:3 | 3.21 | 187.8 | (0.279, 0.677) | 493 |
| Example 210 | | 1:2 | 3.20 | 190.8 | (0.277, 0.668) | 490 |
| Example 211 | 1-158:2-32 | 1:3 | 3.21 | 188.9 | (0.277, 0.680) | 501 |
| Example 212 | | 1:2 | 3.20 | 190.7 | (0.272, 0.666) | 498 |

Looking at results of Tables 8 and 9 above, the results of Tables 8 and 9 above exhibit better efficiency and lifespan effects when the compound of Chemical Formula 1 and the compound of Chemical Formula 2 are included at the same time. These results may expect an exciplex phenomenon to occur when both of the compounds are included at the same time.

The exciplex phenomenon is a phenomenon in which energy having a size of a HOMO level of a donor (p-host) and a LUMO level of an acceptor (n-host) is emitted through electron exchange between two molecules. When a donor (p-host) with good hole transport ability and an acceptor (n-host) with good electron transport ability are used as a host of the light emitting layer, since holes are injected into the p-host and electrons are injected into the n-host, the driving voltage may be lowered, thereby helping to improve the lifespan. In the present disclosure, it could be confirmed that excellent device properties are exhibited when the compound of Chemical Formula 2 above acting as a donor and the compound of Chemical Formula 1 above acting as an acceptor are used as a host for the light emitting layer.

### <Reference Numeral>

- 100:: Substrate
- 200:: Anode
- 300:: Organic Material Layer
- 301:: Hole Injection Layer
- 302:: Hole Transport Layer
- 303:: Light Emitting Layer
- 304:: Hole Blocking Layer
- 305:: Electron Transport Layer
- 306:: Electron Injection Layer
- 400:: Cathode

## Claims

1. An organic light emitting device comprising a first electrode, a second electrode, and an organic material layer comprising one or more layers provided between the first electrode and the second electrode, wherein the one or more layers of the organic material layer comprise a heterocyclic compound represented by the following Chemical Formula 1 and a heterocyclic compound represented by the following Chemical Formula 2: 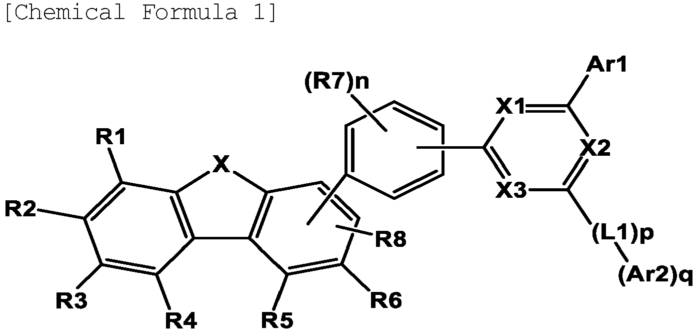 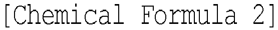 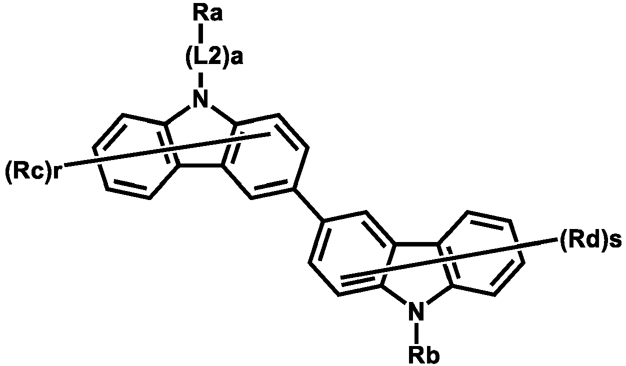 In Chemical Formulas 1 and 2 above,
X is O; or S,
R1 to R8, and Rc and Rd are the same as or different from each other, and are each independently selected from the group including hydrogen; deuterium; a halogen; a cyano group; a substituted or unsubstituted C1-C60 alkyl group; a substituted or unsubstituted C2-C60 alkenyl group; a substituted or unsubstituted C2-C60 alkynyl group; a substituted or unsubstituted C1-C60 alkoxy group; a substituted or unsubstituted C3-C60 cycloalkyl group; a substituted or unsubstituted C2-C60 heterocycloalkyl group; a substituted or unsubstituted C6-C60 aryl group; a substituted or unsubstituted C2-C60 heteroaryl group; -SiRR'R"; -P(=O)RR'; and -NRR', or two or more groups adjacent to each other are bonded to each other to form a substituted or unsubstituted aromatic hydrocarbon ring or a substituted or unsubstituted heterocycle ring,
Xl to X3 are N; or CRe, and at least one of Xl to X3 is N,
L1 and L2 are the same as or different from each other and are each independently a direct bond; a substituted or unsubstituted C6-C60 arylene group; or a substituted or unsubstituted C2-C60 heteroarylene group,
Ar1 and Ar2 are the same as or different from each other and are each independently a substituted or unsubstituted C1-C60 alkyl group; a substituted or unsubstituted C6-C60 aryl group; or a substituted or unsubstituted C2-C60 heteroaryl group,
Ra and Rb are the same as or different from each other and are each independently -CN; -SiRR'R"; a substituted or unsubstituted C6-C60 aryl group; or a substituted or unsubstituted C2-C60 heteroaryl group,
R, R', R", and Re are the same as or different from each other and are each independently hydrogen; deuterium; a substituted or unsubstituted C1-C60 alkyl group; or a substituted or unsubstituted C6-C60 aryl group,
n, p, and a are an integer of 0 to 4,
r and s are an integer of 0 to 7,
q is an integer of 1 to 5, and
when n, p, a, s, q, and r are 2 or more, the substituents in parentheses are the same as or different from each other.

2. The organic light emitting device of claim 1, wherein Chemical Formula 1 above is represented by the following Chemical Formula 3 or 4: 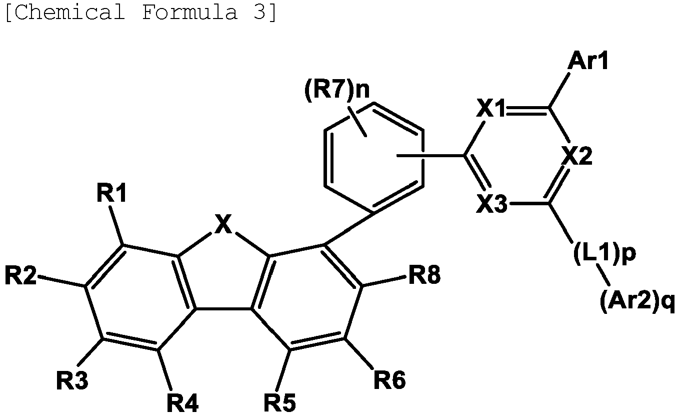 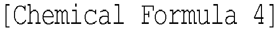 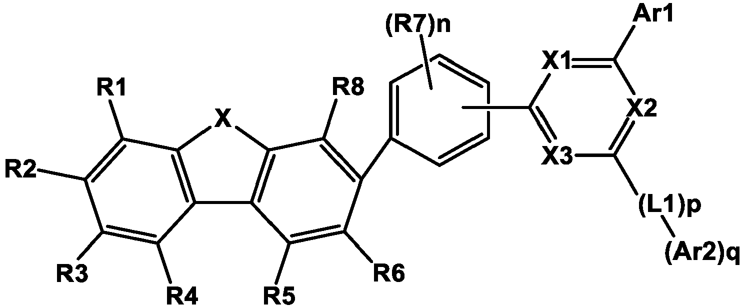 In Chemical Formulas 3 and 4 above,
the definition of each substituent is the same as defined in Chemical Formula 1 above.

3. The organic light emitting device of claim 1, wherein 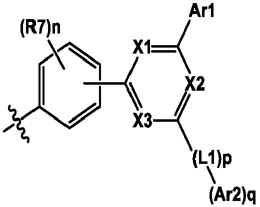 of Chemical Formula 1 above is represented by any one of the following Chemical Formulas 1-1 to 1-3: 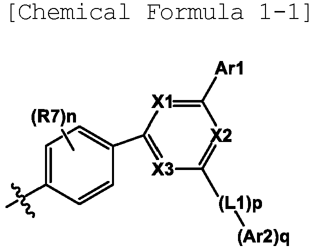 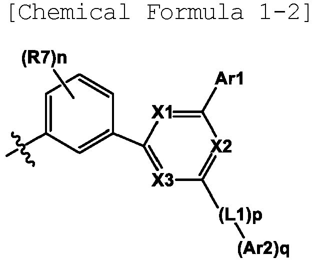 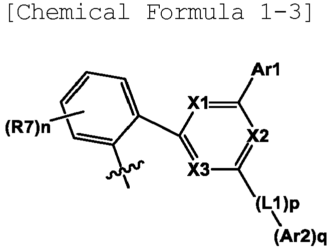 In Chemical Formulas 1-1 to 1-3 above,
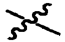 means a position connected to Chemical Formula 1, and
the definition of each substituent is the same as defined in Chemical Formula 1 above.

4. The organic light emitting device of claim 1, wherein Ar1 is represented by any one of the following Chemical Formulas 1-1-1 to 1-1-3: 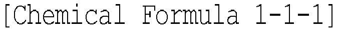 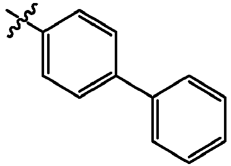 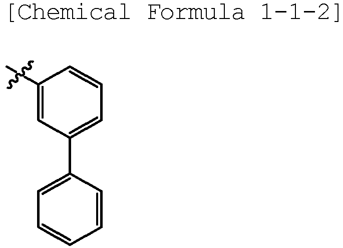 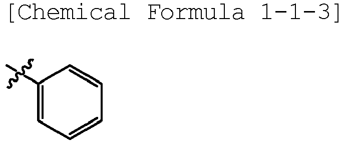 In Chemical Formulas 1-1-1 to 1-1-3 above,
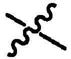 means a position connected to Chemical Formula 1.

5. The organic light emitting device of claim 1, wherein Ar2 is represented by the following Chemical Formula 1-2-1 or 1-2-2: 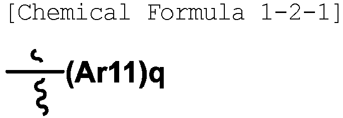 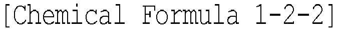 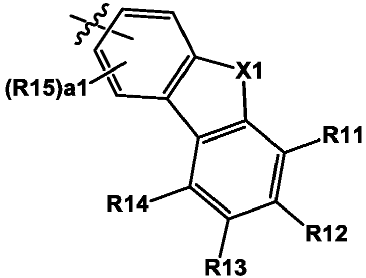 In Chemical Formulas 1-2-1 and 1-2-2 above,
q is the same as the definition in Chemical Formula 1 above,
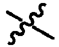 means a position connected to Chemical Formula 1,
X1 is O; or S,
R11 to R15 are the same as or different from each other, and are each independently selected from the group comprising hydrogen; deuterium; a halogen; a cyano group; a substituted or unsubstituted C1-C60 alkyl group; a substituted or unsubstituted C2-C60 alkenyl group; a substituted or unsubstituted C2-C60 alkynyl group; a substituted or unsubstituted C1-C60 alkoxy group; a substituted or unsubstituted C3-C60 cycloalkyl group; a substituted or unsubstituted C2-C60 heterocycloalkyl group; a substituted or unsubstituted C6-C60 aryl group; a substituted or unsubstituted C2-C60 heteroaryl group; -SiRR'R"; -P(=O)RR'; and -NRR', or two or more groups adjacent to each other are bonded to each other to form a substituted or unsubstituted aromatic hydrocarbon ring or a substituted or unsubstituted heterocycle ring,
al is an integer of 0 to 3, and when it is 2 or more, the substituents in parentheses are the same as or different from each other,
the definitions of R, R', and R" are the same as those in Chemical Formula 1 above, and
Ar11 is a substituted or unsubstituted C6-C60 aryl group.

6. The organic light emitting device of claim 1, wherein Chemical Formula 1 above is represented by any one of the following compounds:

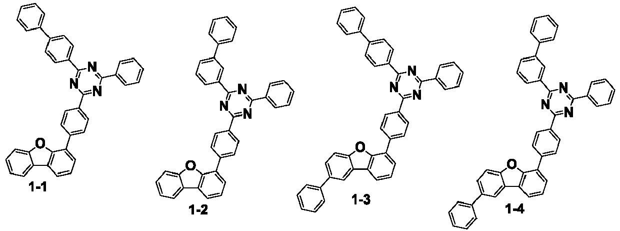

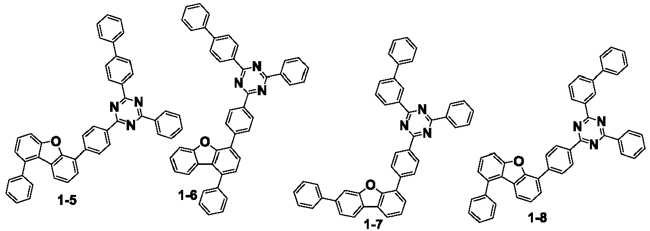

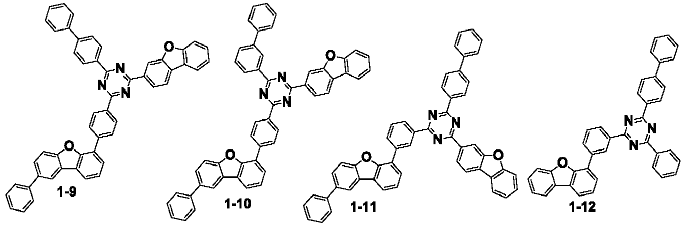

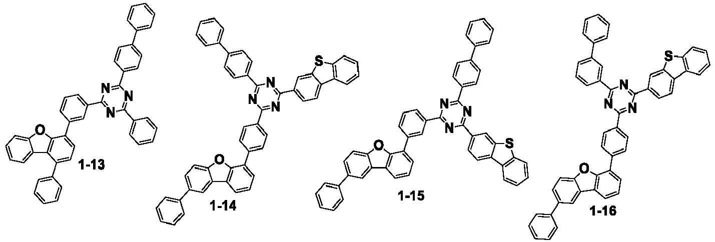

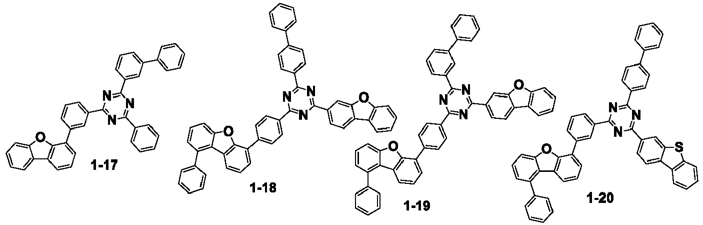

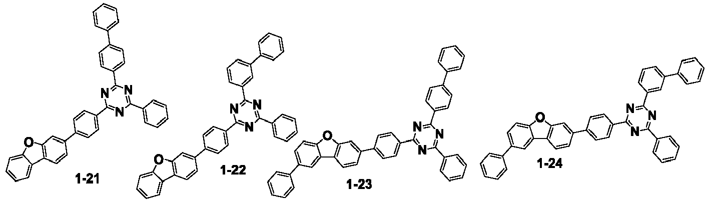

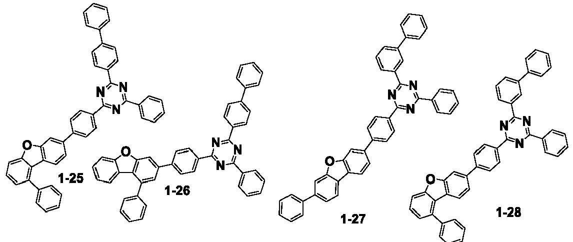

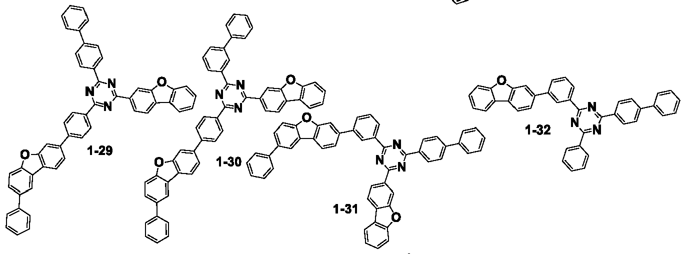

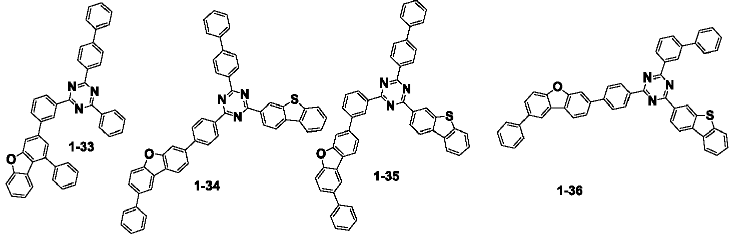

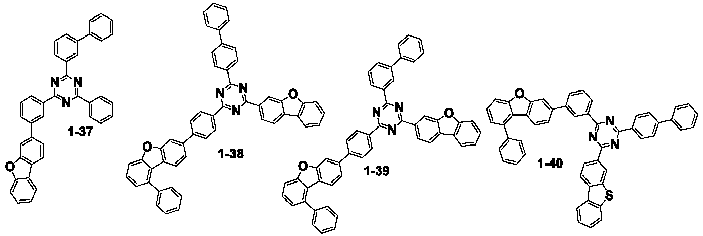

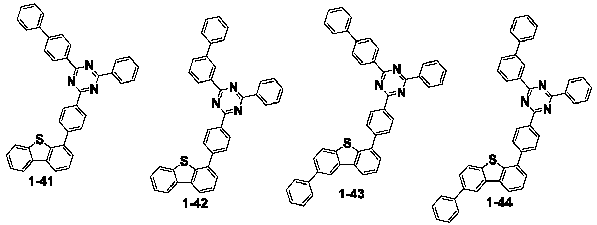

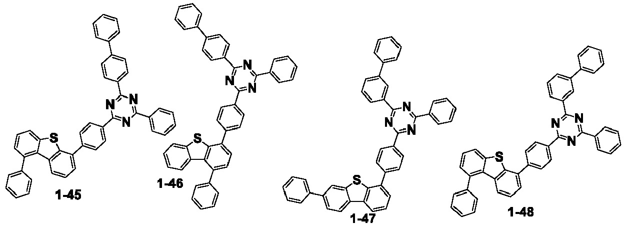

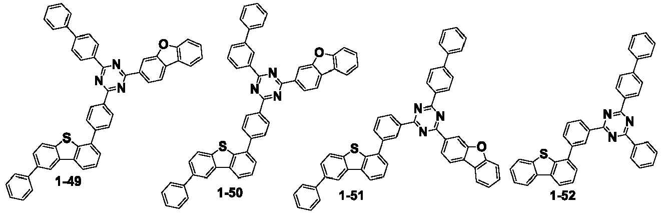

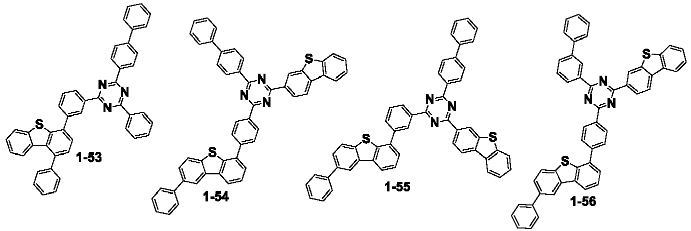

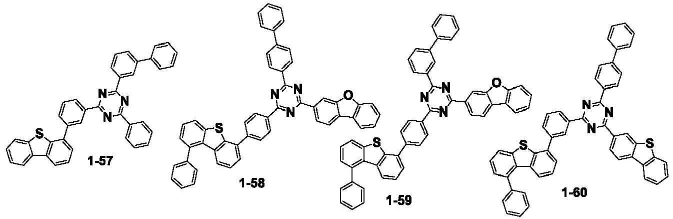

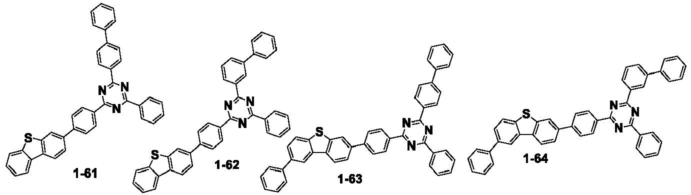

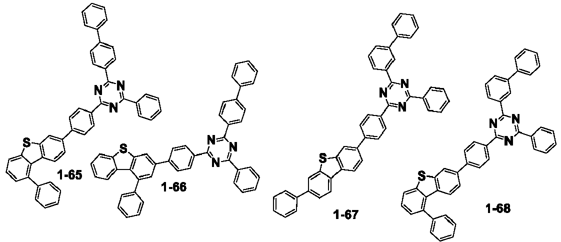

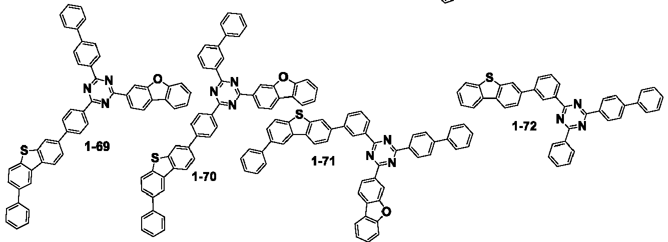

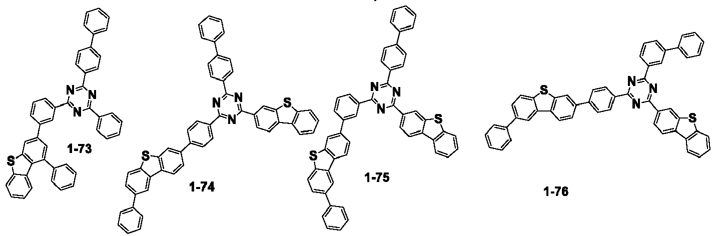

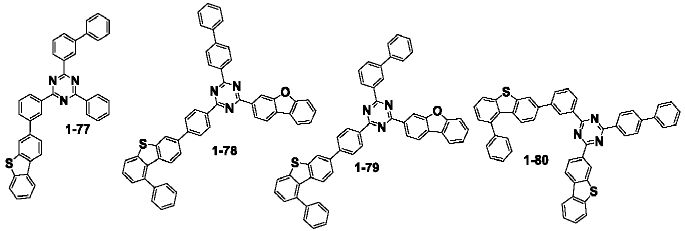

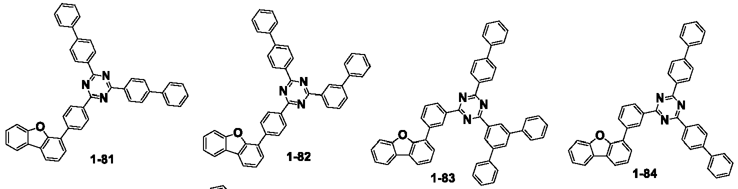

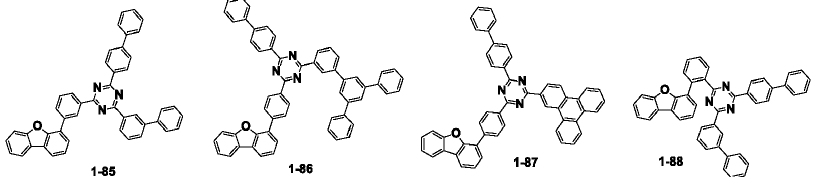

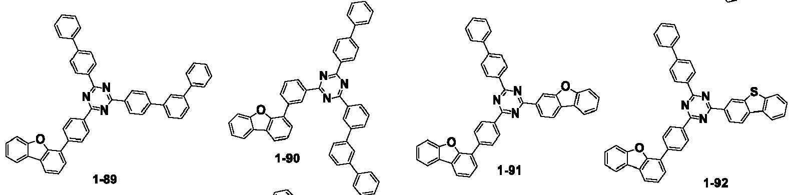

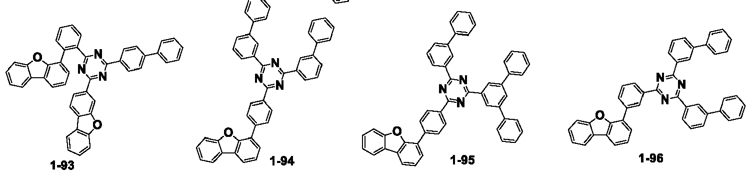

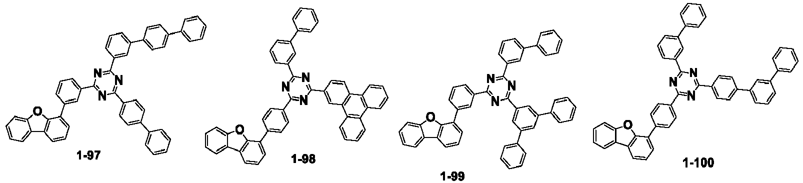

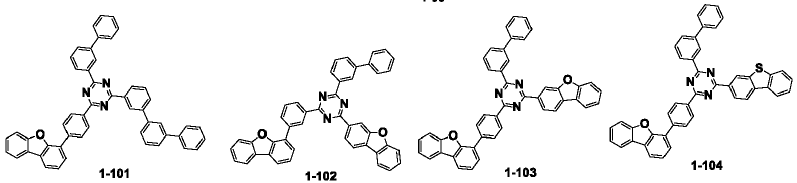

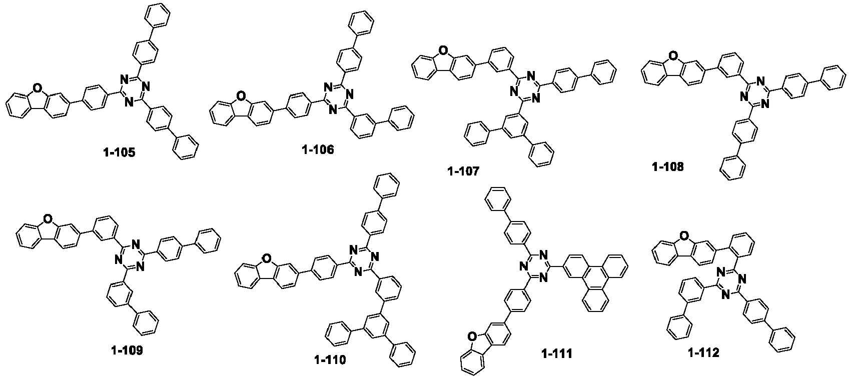

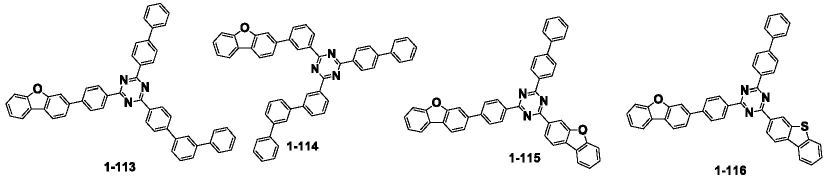

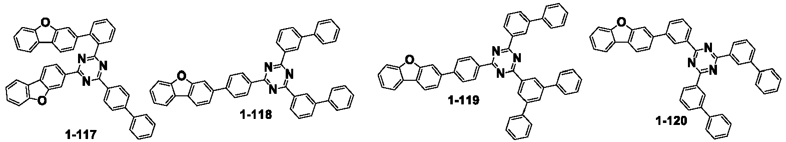

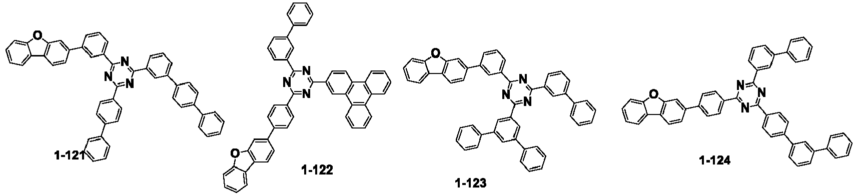

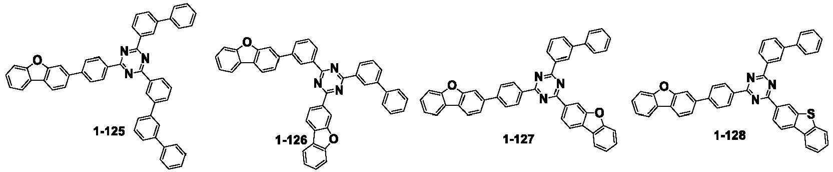

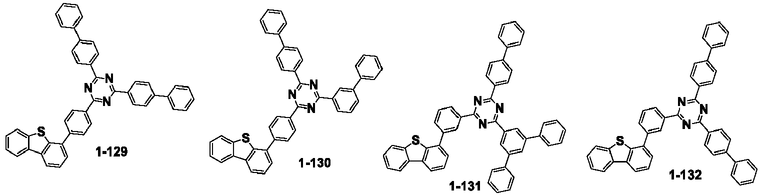

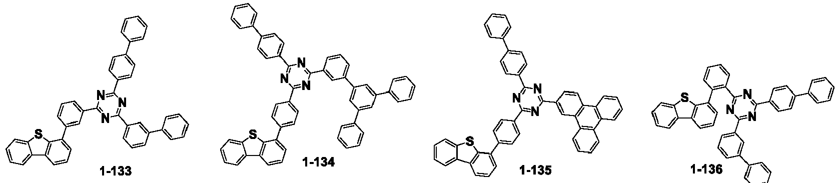

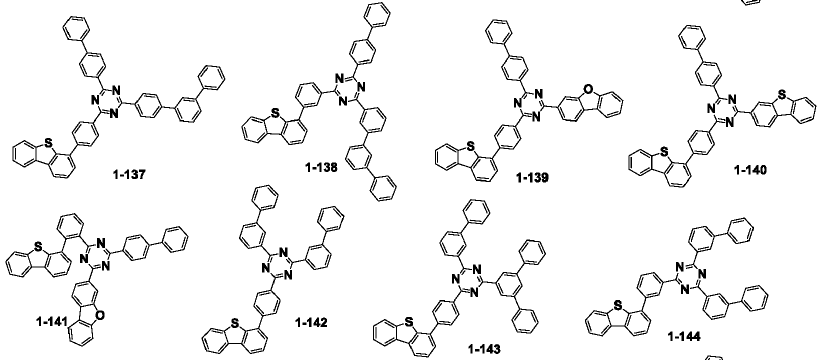

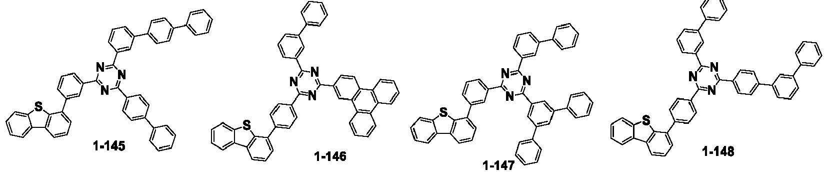

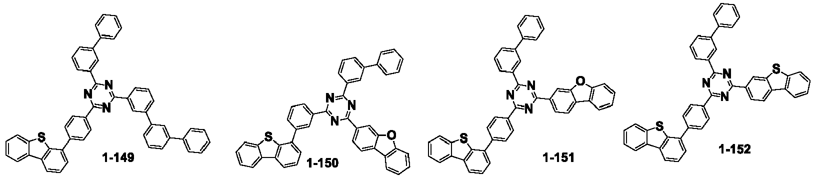

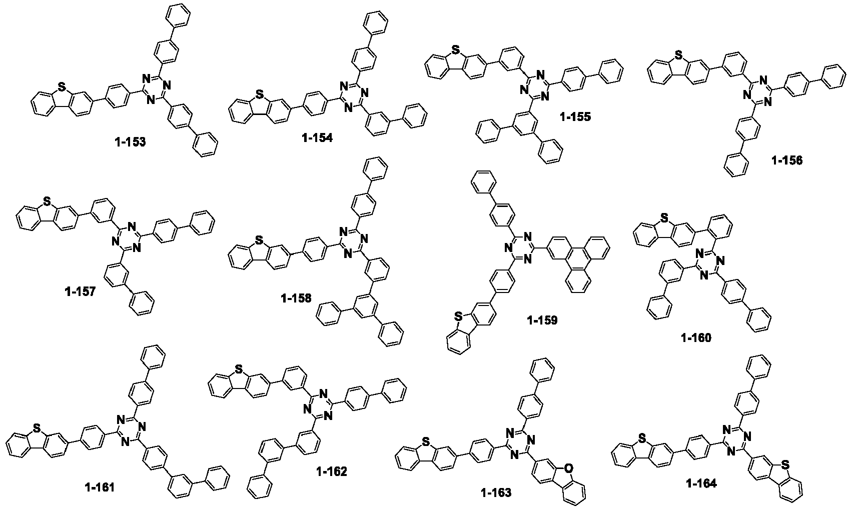

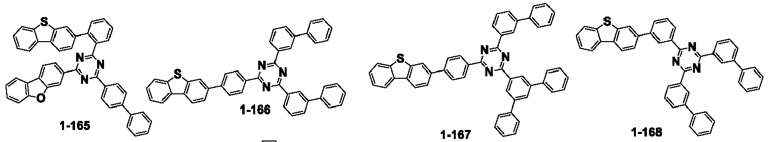

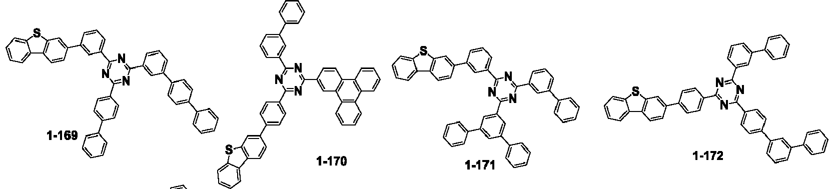

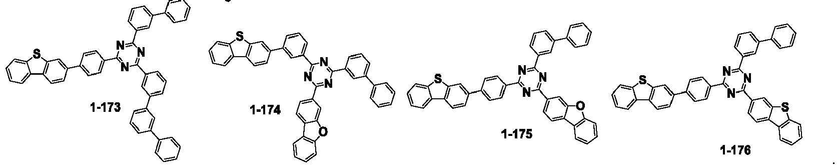

7. The organic light emitting device of claim 1, wherein Chemical Formula 2 above is represented by any one of the following compounds:

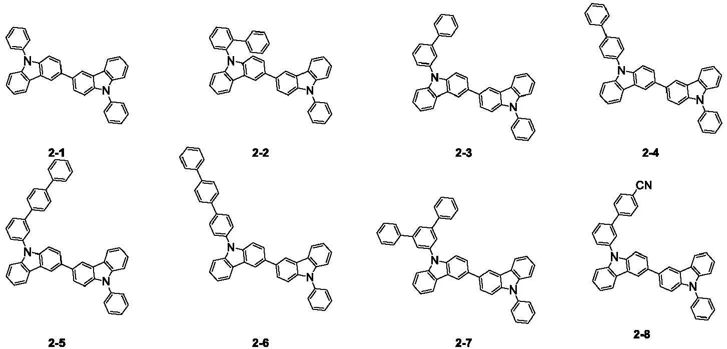

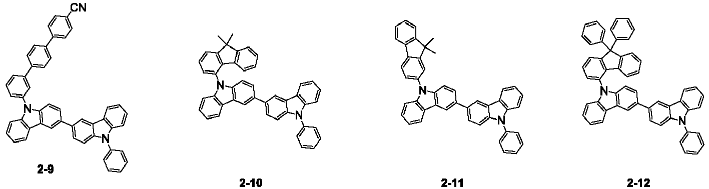

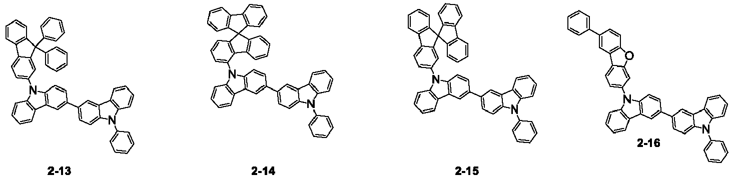

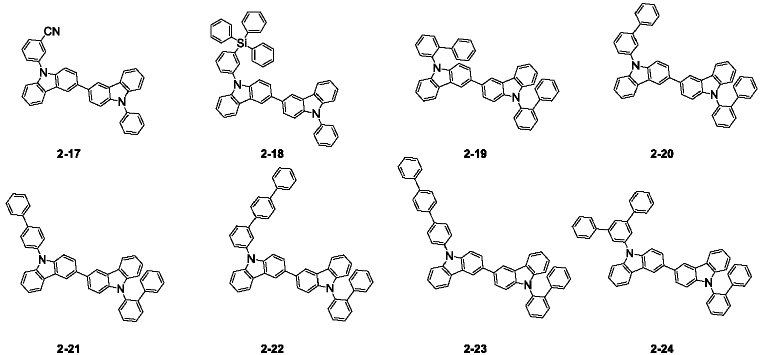

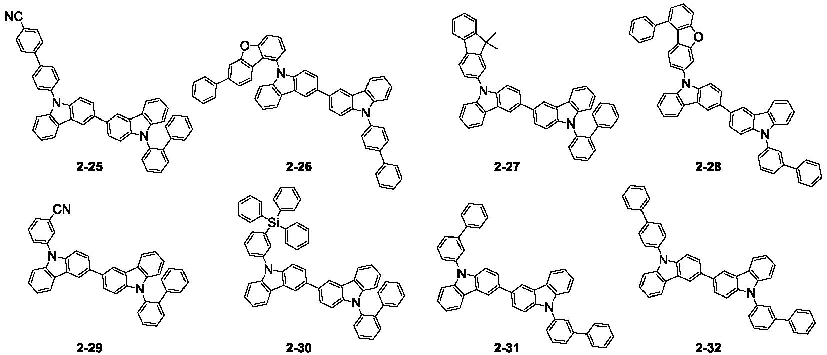

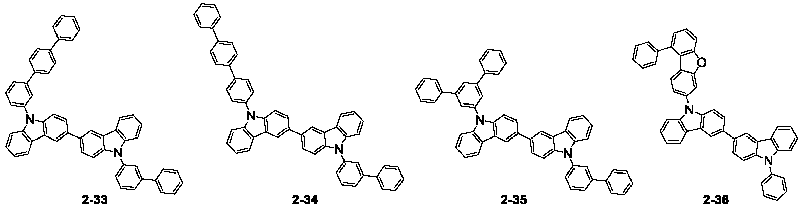

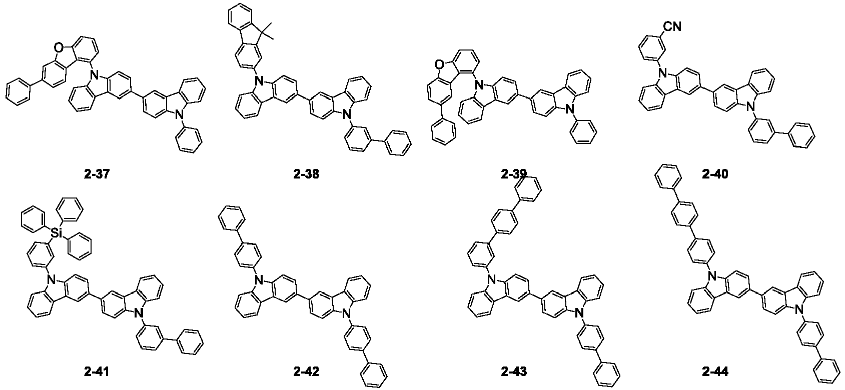

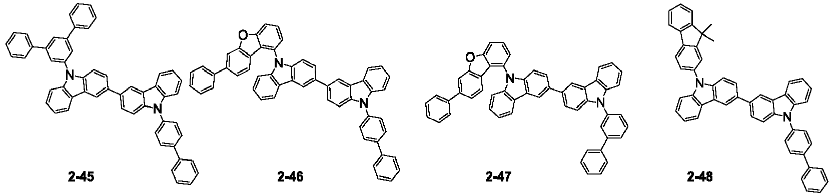

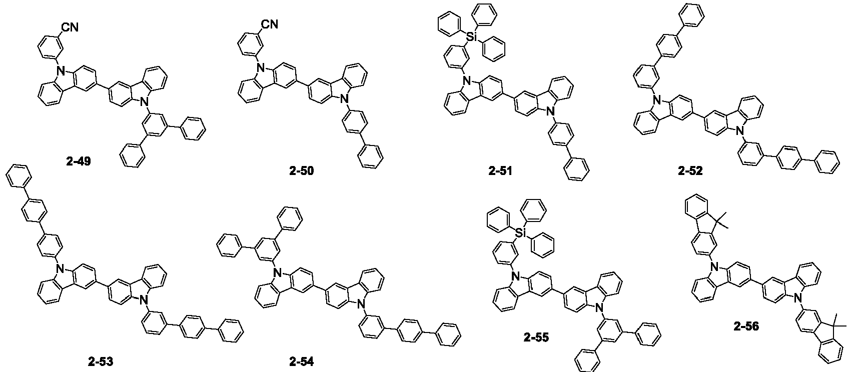

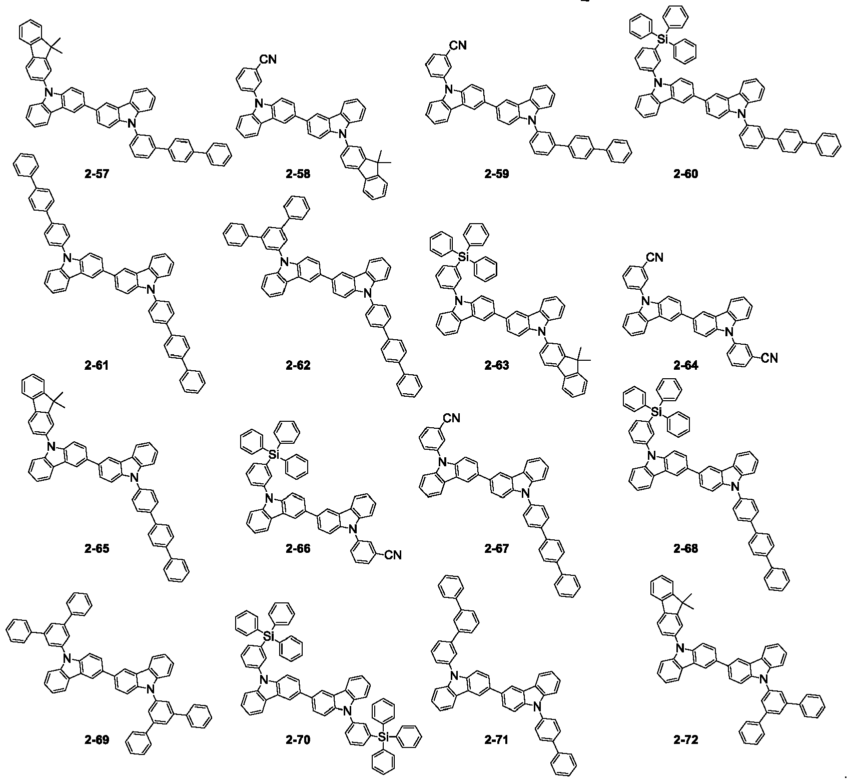

8. The organic light emitting device of claim 1, wherein the organic material layer comprises at least one layer of a hole blocking layer, an electron injection layer, and an electron transport layer, and the at least one layer of the hole blocking layer, the electron injection layer, and the electron transport layer comprises a heterocyclic compound represented by Chemical Formula 1 above and a heterocyclic compound represented by Chemical Formula 2 above.

9. The organic light emitting device of claim 1, wherein the organic material layer comprises a light emitting layer, and the light emitting layer comprises a heterocyclic compound represented by Chemical Formula 1 above and a heterocyclic compound represented by Chemical Formula 2 above.

10. The organic light emitting device of claim 1, wherein the organic material layer comprises a light emitting layer, the light emitting layer comprises a host material, and the host material comprises a heterocyclic compound represented by Chemical Formula 1 above and a heterocyclic compound represented by Chemical Formula 2 above.

11. The organic light emitting device of claim 1, further comprising one layer or two or more layers selected from the group comprising a light emitting layer, a hole injection layer, a hole transport layer, an electron injection layer, an electron transport layer, an electron blocking layer, and a hole blocking layer.

12. A composition for the organic material layer of the organic light emitting device, comprising the heterocyclic compound represented by Chemical Formula 1 above and the heterocyclic compound represented by Chemical Formula 2 above according to claim 1.

13. The composition for the organic material layer of the organic light emitting device of claim 12, wherein the weight ratio of the heterocyclic compound represented by Chemical Formula 1 above to the heterocyclic compound represented by Chemical Formula 2 above in the composition is 1:10 to 10:1

14. A method for manufacturing an organic light emitting device, comprising the steps of:
preparing a substrate;
forming a first electrode on the substrate;
forming an organic material layer comprising one or more layers on the first electrode; and
forming a second electrode on the organic material layer, wherein the step of forming the organic material layer comprises a step of forming an organic material layer comprising one or more layers using the composition for the organic material layer according to claim 12.

15. The method of claim 14, wherein the step of forming the organic material layer is forming the organic material layer using a thermal vacuum deposition method by premixing the heterocyclic compound of Chemical Formula 1 above and the heterocyclic compound of Chemical Formula 2 above.
